# EUROPEAN PATENT SPECIFICATION

(11) **EP 2 379 087 B1**
(45) Date of publication and mention of the grant of the patent: **20.08.2014**
(21) Application number: 09795654.4
(22) Date of filing: 19.12.2009
(51) Int. Cl.: A61K 35/44, C12N 5/078

(54) **UMBILICAL CORD TISSUE DERIVED CELLS FOR TREATING NEUROPATHIC PAIN AND SPASTICITY**
AUS NABELSCHNURGEWEBE GEWONNENE ZELLEN ZUR BEHANDLUNG VON NEUROPATHISCHEN SCHMERZEN UND SPASTIZITÄT
CELLULES EXTRAITES DU TISSU DU CORDON OMBILICAL POUR LE TRAITEMENT DE DOULEUR NEUROPATHIQUE ET DE LA SPASTICITÉ

(30) Priority: 19.12.2008 US 139169 P
(43) Date of publication of application: 26.10.2011
(73) Proprietor: DePuy Synthes Products, LLC, Raynham, MA 02767-0350 (US)
(72) Inventor: KRAMER, Brian C., Plainfield New Jersey 07060 (US); HERZBERG, Uri, Bridgewater New Jersey 08807 (US)
(74) Representative: Hallybone, Huw George
(86) International application number: PCT/US2009/068879
(87) International publication number: WO 2010/071862

(56) References cited:
- WO-A1-2009/085860
- WO-A2-2006/055685
- WO-A2-2006/117237
- WO-A2-2008/085221
- US-A1- 2008 305 148
- MEIER CAROLA ET AL: "Spastic paresis after perinatal brain damage in rats is reduced by human cord blood mononuclear cells." PEDIATRIC RESEARCH FEB 2006, vol. 59, no. 2, February 2006 (2006-02), pages 244-249, XP002569949 ISSN: 0031-3998

## Description

### CROSS REFERENCE TO RELATED APPLICATIONS

This application claims benefit to United States Provisional Patent Application No. 61/139,169, filed December 19, 2008.

### FIELD OF THE INVENTION

The present invention relates generally to compositions, for treating neuropathic pain by administration of cells. In particular, the invention provides administering cells systemically to a patient to normalize inflammatory or degenerative or a state of pathology of the nervous system. Such a pathology could be at the subcellular, cellular and tissue milieu, thereby modifying neuronal interactions and decreasing pain.

### BACKGROUND OF THE INVENTION

Various patents and other publications are referred to throughout the specification.

Chronic pain in general is a public health issue affecting 30-60% of all Americans. In most cases, there is little to no correlation between objective disease findings (X-ray, MRI and CT scan of the region in pain) and subjective pain reports. Chronic pain includes pain which persists beyond the normal healing time for a disease or injury, pain related to chronic degenerative disease or a persistent neurologic condition, pain that emerges or persists, even recurnng for months to years without an identifiable cause, or as pain associated with cancer.

Chronic pain is often caused by disorders of the nervous system, also known as neuropathy or neuropathic pain. Neuropathic pain is typically accompanied by tissue damage, including nerve fibers that are damaged, dysfunction or injured. Neuropathic pain may be caused by a variety of problems, including pathologic lesions, neurodegeneration processes, or prolonged dysfunction of parts of the peripheral or central nervous system. Neuropathic pain can also be present when no detectable damage can be assessed or defined.

Clinical and scientific literature points to neuropathic pain as having two components: central plasticity and changes in peripheral nerves. Central plasticity can be the result of changes in receptor population or receptor sensitivity at any level of the CNS. In addition, there is evidence pointing to changes taking place in neurons and in microglia. In fact, recent data points to microglial activity as an important mediator of central sensitization of the spinal cord. Such central sensitization is known to play a major role in mediating chronic inflammatory as well as neuropathic pain. In the periphery, changes in Schwann cell - axonal interaction are known to play a role in the induction and maintenance of neuropathic pain.

The standard course of treatment for chronic pain involves a step ladder approach which begins with non-opioid analgesics and progresses from moderate opiates to potent opiates. Opiates are often used in combination with other agents. In this way, a physician is able to monitor and adjust the dose of the agent to limit the undesired side effects of opioids, which includes sedation, cognitive impairment, myoclonus, addiction, tolerance, and respiratory depression. However, opioids can also induce nausea, constipation, confusion, respiratory depression, and dependence. In addition, opiate tolerance is a well documented side effect observed in chronic pain patients.

Other agents used to treat chronic pain include nonsteroidal anti-inflammatory drugs (NSAIDs), which are both anti-inflammatory and analgesic, antidepressants, anticonvulsants, topical agents, cannabinoids, botulinum toxin, NMDA antagonists, antepileptics, anti-depressants and dietary supplements. These compounds, however, all have side effects which can be debilitating, including CNS depression, cardiovascular effects, gastrointestinal disturbances, ulceration, renal damage, decreased libido and hypersensitivity reactions. In addition, these compounds must be taken repeatedly, typically more than once a day, and some compounds become ineffective with time, resulting in tolerance to the drug.

In addition, current treatments are unable to relieve pain in many clinically severe chronic neuropathic disorders, such as diabetic neuropathy, cervical radiculopathy, neuralgic amyotrophy, HIV neuropathy, neuralgic amyotrophy, or post herpetic neuralgia. Other chronic conditions intractable to current medical strategies are associated with both peripheral and/or central pain such as, post spinal cord injury, muscular dystrophy, trigeminal neuralgia, phantom limb pain, fibromyalgia syndrome, causalgia, and diabetic and alcoholic polyneuropathies. Spasticity of spinal cord origin, which results from multiple sclerosis or spinal cord injury, is another condition which often resists current treatments and which can result in chronic pain.

Presently, there is interest in using transplanted cells at the site of neural damage to assist in the repair or reversal of neural cell damage. For instance, some researchers have transplanted neural cells to the site of injury of a patient with a sensory neural pathway disorder or injury. *See,* U.S. Patent Application No. 09/163,684 which issued as US Patent No. 6,444,205. Other researchers focus on transplantation of stem cells to reconstitute a target tissue, thereby restoring physiologic and anatomic functionality. For instance, Klass administered marrow mononuclear cells containing stem cell populations to a neuropathic pain model to find if pain decreased. *See,* Klass et al. Anesth Analg., 2007; 104:944 - 49. A viable, reliable method of administering cells to decrease neuropathic pain does not presently exist.

Given the current limitations in treating chronic and neuropathic pain, there exists a need for alleviating chronic and neuropathic pain in individuals with treatments that do not need to be administered on a daily basis.

### SUMMARY OF THE INVENTION

The problems presented are solved by the compositions of the illustrative embodiments described herein. These embodiments provide methods for treating chronic and neuropathic pain by administering a population of cells. While not wishing to be bound by any mechanism of action, the inventors believe that the cells administered have the capacity of normalizing inflammatory or degenerative cellular and tissue milieu at the Schwann cell - axonal interaction and/or the microglia - neuronal interaction to decrease pain. Further, the cell administration may block ectopic neuronal firing, thus decreasing pain. The present invention is based, at least in part, on the discovery that cells, including cells derived from human umbilical cord tissue, can be administered systemically to a patient in need of chronic pain treatment.

Specific options provided by the invention are directed to the direct repair, regeneration, replacement of, or the support of the repair, regeneration, or replacement of neural cells for the treatment of neural damage, injury and/or pain.

The disclosure also pertains to a method of treating a subject having neural damage, injury and/or pain by administering a population of cells in an amount effective, such that the damage, injury and/or pain is treated. The cells administered are umbilical cord tissue-derived cells.

The cells are administered systemically.

The cells may be administered by any means which allows systemic distribution of the cells, including but not limited to, intramuscular, intravenous, or intra-arterial delivery via syringes with needles and/or catheters with or without pump devices. In some specific embodiments the population of cells is administered with a hydrogel. Further embodiments are to specific hydrogels, such as collagen, atelocollagen, fibrin constructs, thrombin-fibrin constructs. In addition, some embodiments include use of one or more growth factors injected in parallel, sequentially, or formulated directly into a hydrogel.

In some embodiments, the population of cells is induced *in vitro* to differentiate into a specific type of cell. In other embodiments, the cells are genetically engineered to produce a gene product that promotes treatment of chronic pain.

In some embodiments, the populations of cells are administered with at least one other agent, including but not limited to, selected extracellular matrix components, anti-apoptotic agents, anti-inflammatory compounds, immunosuppressive or immunomodulatory agents, local anesthetics, and other angiogenic factors. The other agent can be administered simultaneously with, before, or after the population of cells. In one embodiment, the composition further comprises at least one of the agents or factors selected from the group consisting of neurotrophic factors. In one embodiment, the population of cells is administered with growth factors and/or other agents which promote differentiation of the cells into a predetermined, desired neural cell. In another embodiment, the desired neural cells are capable of secreting one or more neurotransmitters.

Also disclosed herein are compositions and kits for treating a patient with chronic pain comprising at least a population of cells and a pharmaceutically acceptable carrier. Other composition and kit forms may include other agents, growth factors, and compounds to promote growth and/or healing of the tissue, such that the chronic pain decreases in severity or length. The pharmaceutical compositions and kits are designed and/or formulated for practicing the methods of the disclosure as outlined above and below.

Other objects, features, and advantages of the illustrative embodiments will become apparent with reference to the drawings and detailed description that follow.

### BRIEF DESCRIPTION OF THE DRAWINGS

Figure 1 is a color photograph showing hUTC cell viability after four days of implantation in a construct.
Figure 2 is a graph showing the effect of local injection on animals separated into the groups of construct, vehicle and injury.
Figure 3 is a graph showing the effect of systemic injection on animals separated into the groups of construct, vehicle and injury.
Figure 4 is a graph showing results with Collagen (Colbar). Administration was carried out locally to the affected side (left). The change from neuropathic pain is (score in tested day following administration - score in neuropathic pain/score in neuropathic pain x 100).
Figure 5 is a graph showing results with Collagen (Colbar). Administration was carried out locally to the contralateral side (right). The change from neuropathic pain is (score in tested day following administration - score in neuropathic pain/score in neuropathic pain x 100).
Figure 6 is a graph showing results with Evicel (Omrix). Administration was carried out locally to the affected side (left). The change from neuropathic pain is (score in tested day following administration - score in neuropathic pain/score in neuropathic pain x 100).
Figure 7 is a graph showing results with Evicel (Omrix). Administration was carried out locally to the contralateral side (right). The change from neuropathic pain is (score in tested day following administration - score in neuropathic pain/score in neuropathic pain x 100).
Figure 8 is a graph showing results of hUTC. Administration was systemic and the change from neuropathic pain is (score in tested day following administration - score in neuropathic pain/score in neuropathic pain x 100).
Figure 9 is a graph showing results of hUTC. Administration was systemic and the change from neuropathic pain is (score in tested day following administration - score in neuropathic pain/score in neuropathic pain x 100).
Figures 10A-C contain statistical results for Figure 4.
Figures 11A-C contain statistical results for Figure 5.
Figures 12A-C contain statistical results for Figure 6.

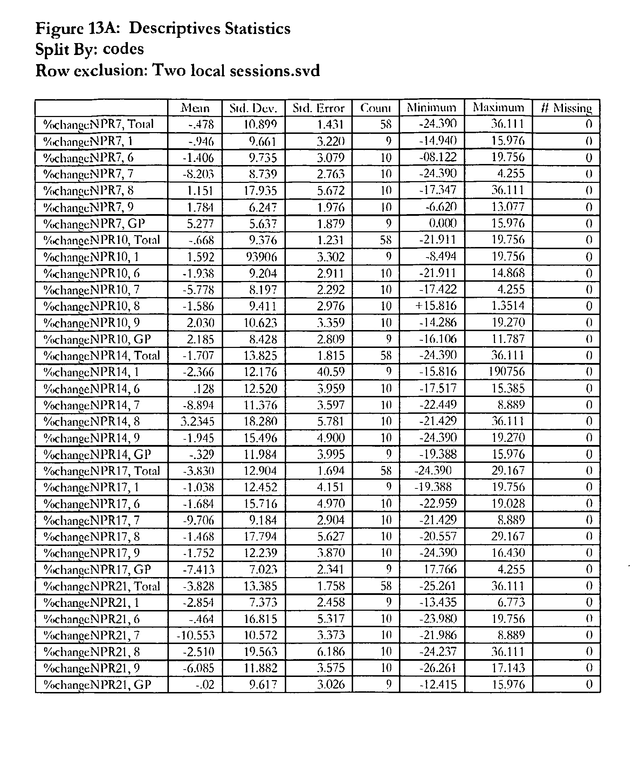

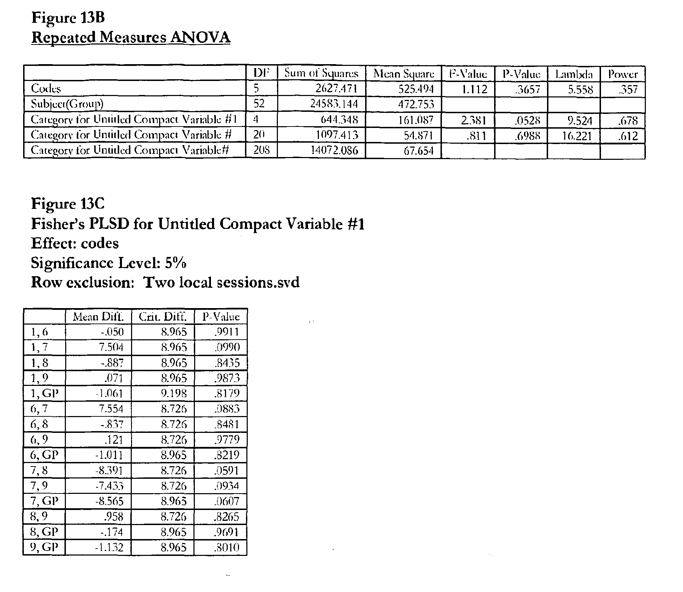

Figures 13A-C contain statistical results for Figure 7.
Figures 14A-C contain statistical results for Figure 8.
Figures 15A-C contain statistical results for Figure 9.

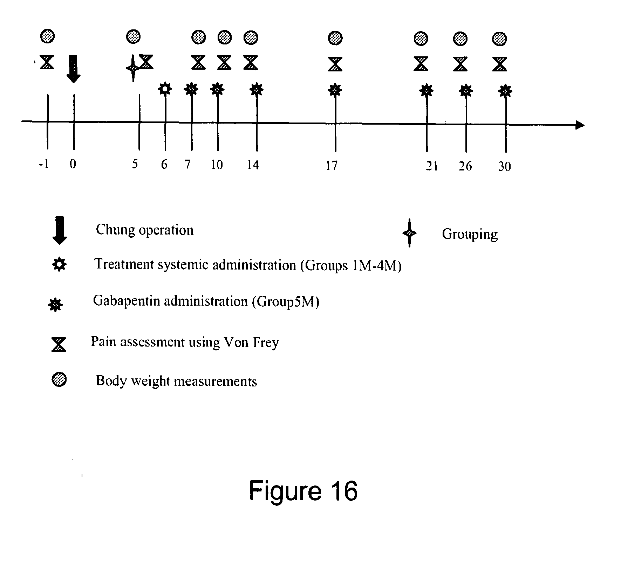

Figure 16 is a schematic depiction of operation and treatment for the test procedures in Example 4.
Figure 17 provides mean body weight for groups.
Figure 18 is body weight gain after Chung surgery and hUTC treatment by systemic administration.
Figure 19 is the mean delta of the Von Frey response. The calculation used was: mean of the right leg minus the mean of the left leg.
Figure 20 is the mean delta of the Von Frey response of leg withdrawal after Chung surgery and after systemic administration of hUTC in study day 6.
Figure 21 is the mean delta of the Von Frey response of leg withdrawal after Chung surgery and systemic administration of hUTC on day 6.

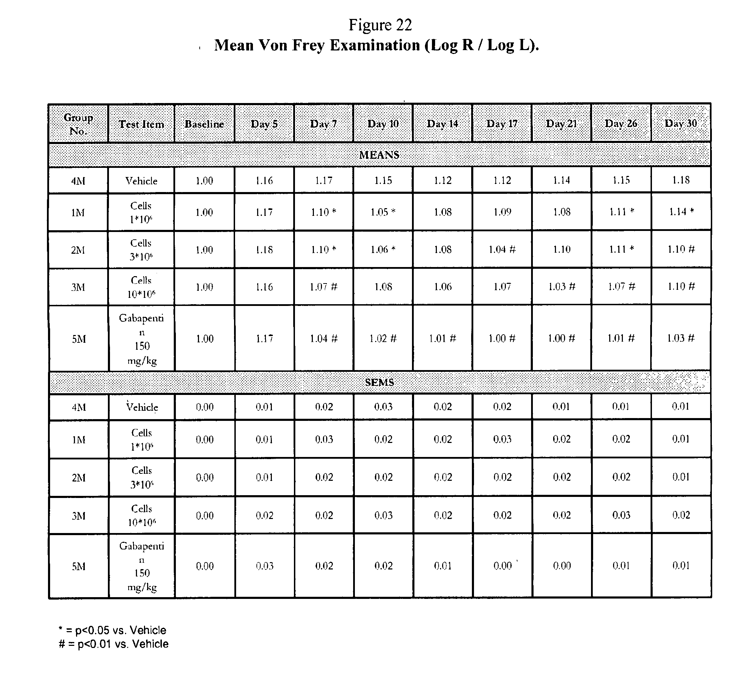

Figure 22 is the mean Von Frey examination.

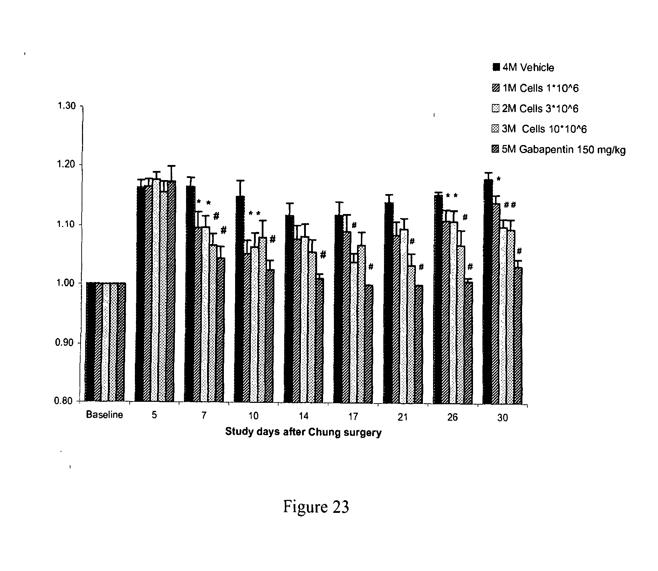

Figure 23 is the difference between paws (represented by the minimal withdrawal Log force of the right leg divided by Log force of the left leg) after systemic administration of hUTC on study day 6 following Chung surgery.

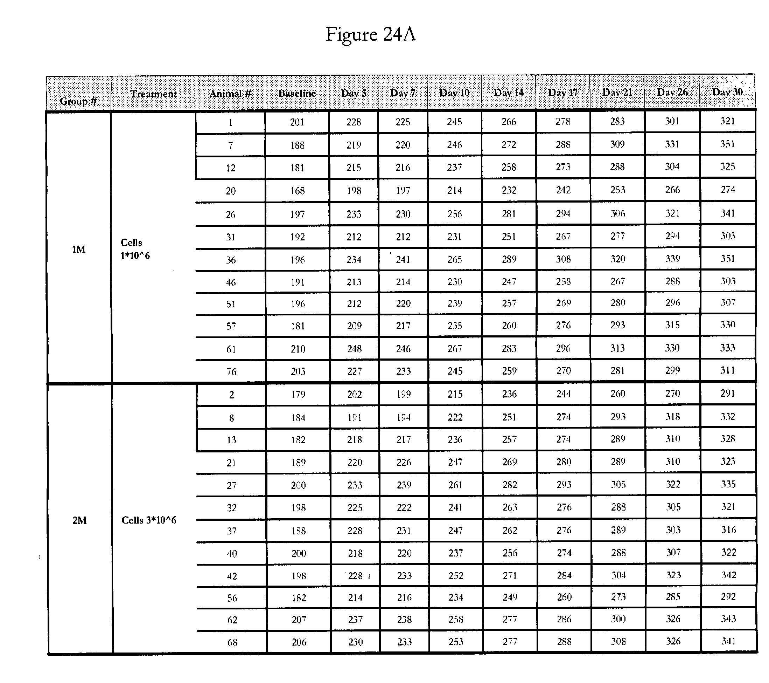

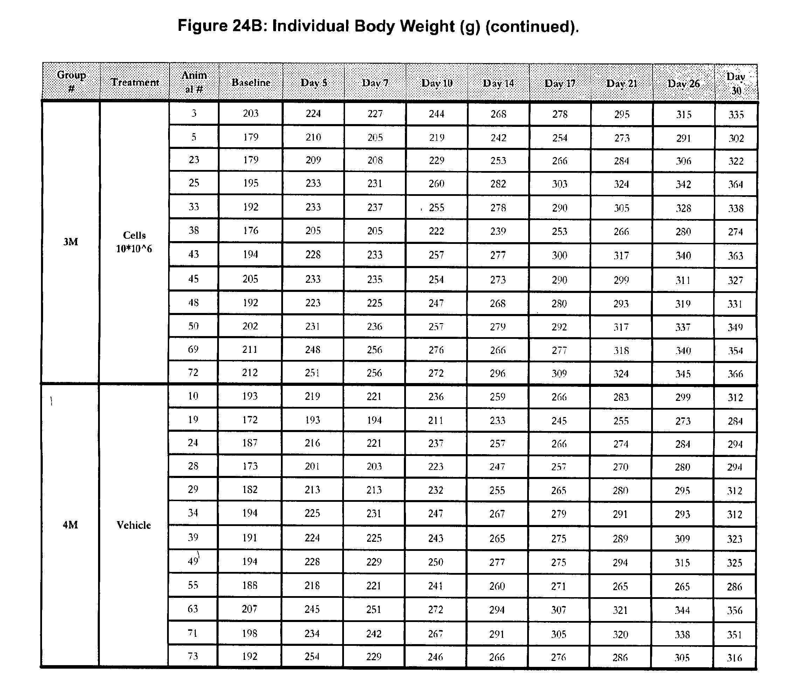

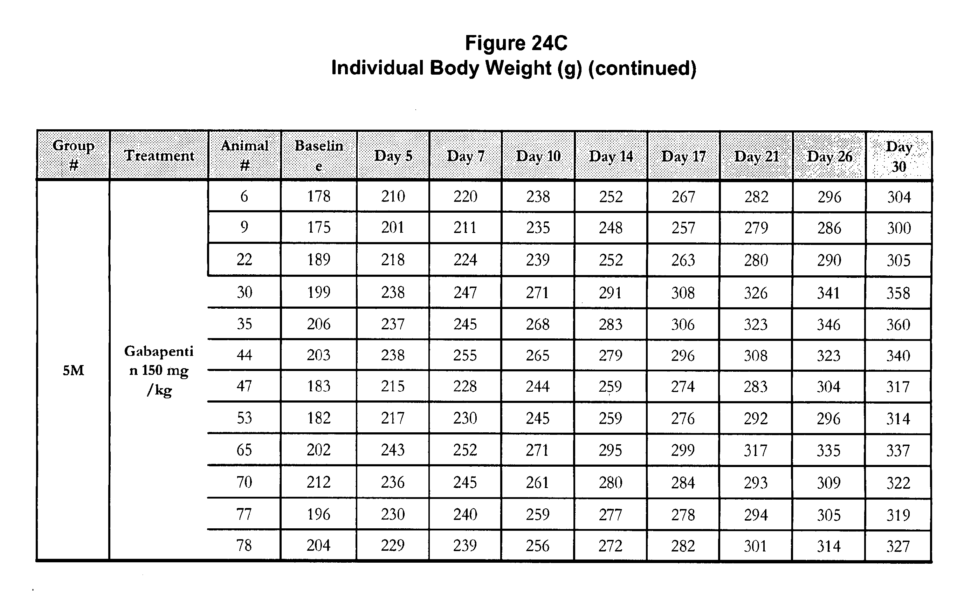

Figures 24A-C are individual data tables of individual body weight.

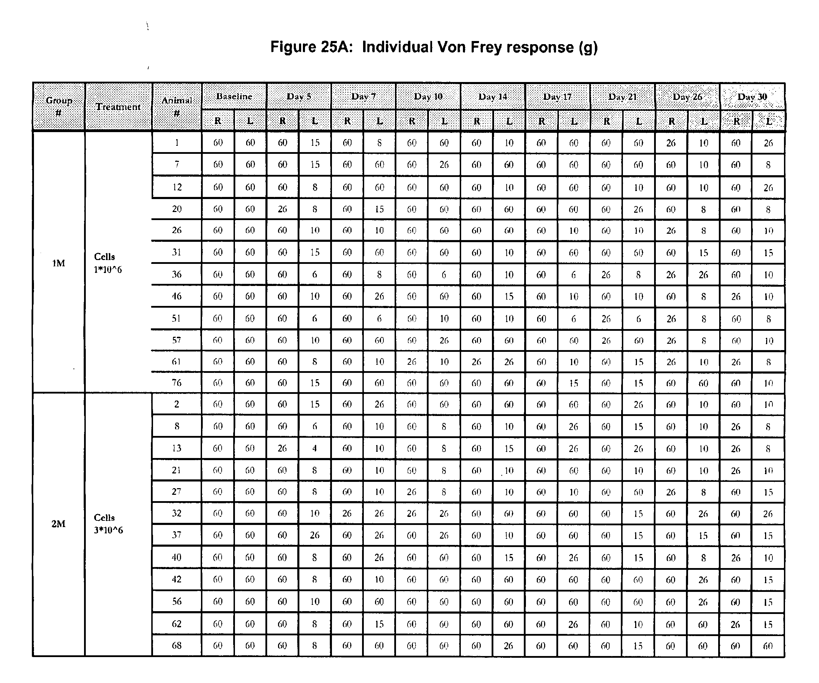

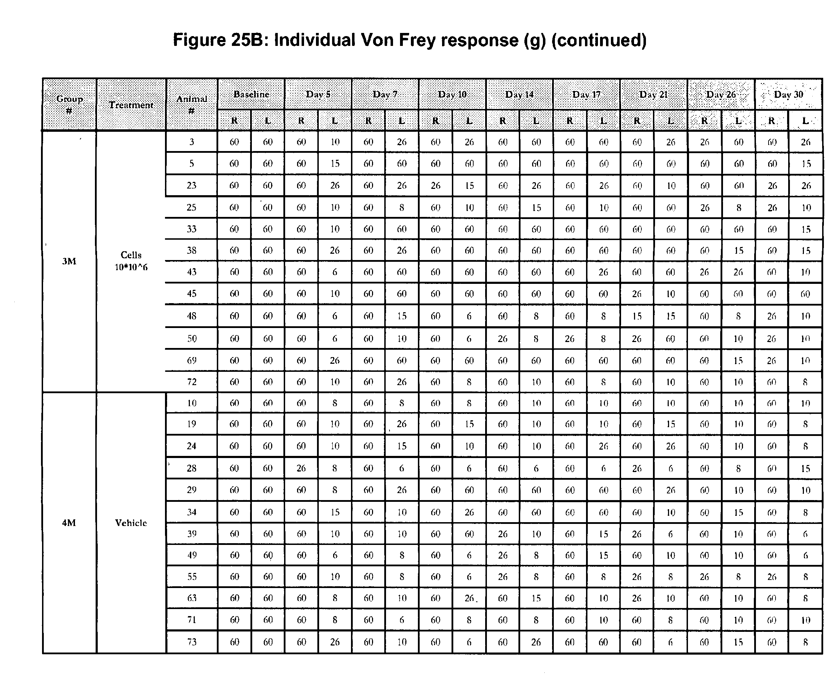

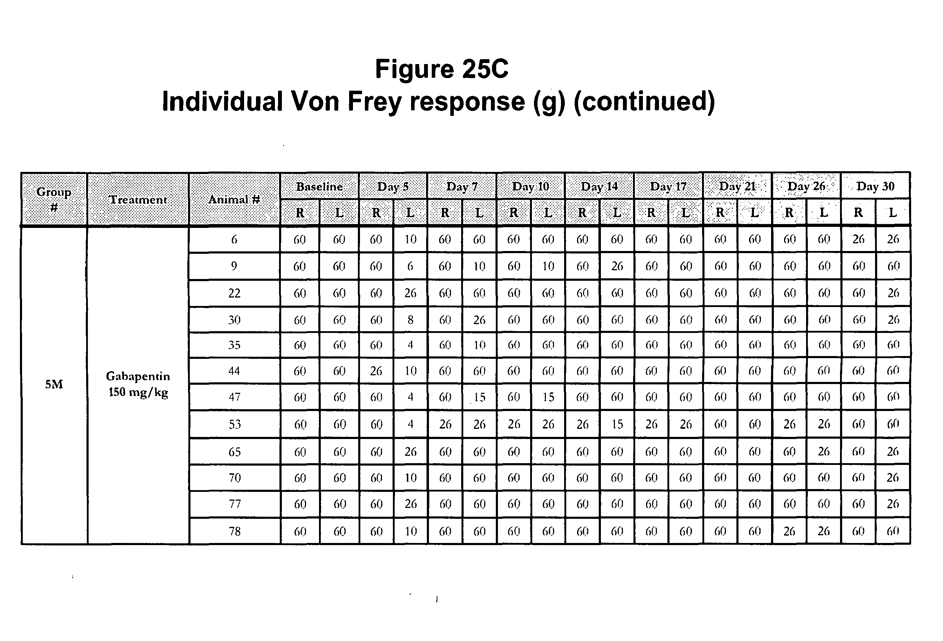

Figures 25A-C are individual data tables of Von Frey response.

### DETAILED DESCRIPTION OF THE INVENTION

In the following detailed description of the illustrative embodiments, reference is made to the accompanying drawings that form a part hereof. These embodiments are described in sufficient detail to enable those skilled in the art to practice the invention, and it is understood that other embodiments may be utilized and that logical structural, mechanical, electrical, and chemical changes may be made without departing from the scope of the invention. To avoid detail not necessary to enable those skilled in the art to practice the embodiments described herein, the description may omit certain information known to those skilled in the art. The following detailed description is, therefore, not to be taken in a limiting sense.

To better clarify the invention, the below definitions are provided.

The terms "chronic pain" and "neuropathic pain" as used interchangeably herein generally refer to conditions in which pain persists and fails to respond to conventional treatment. These terms include pain of long duration and pain that can be medically refractory. These terms also include pain characterized by a persistent increase in the level of neuron excitability or the presence of abnormal sensations in the affected area. Exemplary chronic pain conditions include diabetic neuropathy, cervical radiculopathy, neuralgic amyotrophy, HIV neuropathy, neuralgic amyotrophy, post herpetic neuralgia, post spinal cord injury, muscular dystrophy, trigeminal neuralgia, phantom limb pain, causalgia, spasticity of spinal cord origin, and diabetic and alcoholic polyneuropathies.

The terms "tissue site," "neural damage," "neural injury" and "site of neural damage" as used herein are interchangeable and generally refer to a wound or defect located on, within, or adjacent to neural tissue of an individual; and may include but are not limited to, dermal tissue, vascular tissue, connective tissue, cartilage, adipose tissue, muscle tissue, tendons or ligaments. The terms may further refer to areas of any tissue that are not necessarily wounded or defective, but are instead areas in which it is desired to add or promote growth of additional tissue. The term may also refer to nervous tissue that while having no apparent damage, clinical observations, tests and patients' report indicate the presence of an abnormality.

The terms "individual," "patient" or "subject" as used herein generally refer to any form of animal, including mammals, such as humans and monkeys, who are treated with the pharmaceutical or therapeutic compositions or in accordance with the methods described. The term "xenogeneic" as used herein refers to transplantation of cells from a donor of one species into a subject of a different species.

The terms "treat," "treating" or "treatment" as used herein generally refer to amelioration or reduction in pain or injury for a period of time following administration of a population of cells into a subject suffering from chronic pain. The amelioration or reduction also includes any objective or subjective parameter such as abatement, remission, diminishing of symptoms or making the injury, pathology, or condition more tolerable to the patient, slowing in the rate of degeneration or decline, making the final point of degeneration less debilitating, improving a subject's physical or mental well-being, or prolonging the length of survival. The treatment or amelioration of symptoms can be based on objective or subjective parameters; including the results of a physical examination or neurological examination.

The terms "effective period," "effective period of time" or "effective conditions" refer generally to a period of time or other controllable conditions (*e.g.,* temperature, humidity for *in vitro* methods), necessary or preferred for an agent or pharmaceutical composition to achieve its intended result.

The term "effective amount" as used herein generally refers to a concentration or amount of a compound, material, or composition, as described herein, that is effective to achieve a particular biological result. Such results include, but are not limited to, the regeneration, repair, or improvement of neural tissue, the improvement of blood flow, and/or the decrease of inflammation in patients with chronic pain. Such effective activity may be achieved, for example, by administering the cells and/or compositions of the present invention to patients with chronic pain. With respect to the population of cells as administered to a patient, an effective amount is generally more about 10⁴ cells/kg body weight, and may range from about 10⁴cells/kg body weight to about 10⁶ cells/kg body weight when delivered locally, or from about 10⁵ to about 10⁷ cells/kg body weight when delivered systemically. In specific embodiments, an effective amount may range from about 10⁵ to about 10⁸ cells/kg body weight. It will be appreciated that the number of cells to be administered will vary depending on the specifics of the disorder to be treated, including but not limited to size or total volume/surface area to be treated, and proximity of the site of administration to the location of the region to be treated, among other factors familiar to the medicinal biologist.

"Stem cells" are undifferentiated cells defined by the ability of a single cell both to self-renew, and to differentiate to produce progeny cells, including self-renewing progenitors, non-renewing progenitors, and terminally differentiated cells. Stem cells are also characterized by their ability to differentiate *in vitro* into functional cells of various cell lineages from multiple germ layers (endoderm, mesoderm and ectoderm), as well as to give rise to tissues of multiple germ layers following transplantation, and to contribute substantially to most, if not all, tissues following injection into blastocysts.

Stem cells are classified according to their developmental potential as: (1) *totipotent;* (2) *pluripotent;* (3) *multipotent;* (4) *oligopotent;* and (5) *unipotent. Totipotent* cells are able to give rise to all embryonic and extraembryonic cell types. *Pluripotent* cells are able to give rise to all embryonic cell types. *Multipotent* cells include those able to give rise to a subset of cell lineages, but all within a particular tissue, organ, or physiological system. For example, hematopoietic stem cells (HSC) can produce progeny that include HSC (self-renewal), blood cell-restricted oligopotent progenitors, and all cell types and elements (e.g., platelets) that are normal components of the blood. Cells that are *oligopotent* can give rise to a more restricted subset of cell lineages than multipotent stem cells. Cells that are *unipotent* are able to give rise to a single cell lineage (e.g., spermatogenic stem cells).

Stem cells are also categorized on the basis of the source from which they are obtained. An *"adult stem cell"* is generally a multipotent undifferentiated cell found in tissue comprising multiple differentiated cell types. The adult stem cell can renew itself. Under normal circumstances, it can also differentiate to yield the specialized cell types of the tissue from which it originated, and possibly other tissue types. An *"embryonic stem cell"* is a pluripotent cell from the inner cell mass of a blastocyst-stage embryo. A *fetal* stem cell is one that originates from fetal tissues or membranes. A *"postpartum stem cell"* is a multipotent or pluripotent cell that originates substantially from extraembryonic tissue available after birth, namely, the placenta and the umbilical cord. These cells have been found to possess features characteristic of pluripotent stem cells, including rapid proliferation and the potential for differentiation into many cell lineages. Postpartum stem cells may be blood-derived (*e.g.,* as are those obtained from umbilical cord blood) or non-blood-derived (e.g., as obtained from the non-blood tissues of the umbilical cord and placenta).

Various terms are used to describe cells in culture. "Cell culture" refers generally to cells taken from a living organism and grown under controlled condition ("in culture" or "cultured"). A "primary cell culture" is a culture of cells, tissues, or organs taken directly from an organism(s) before the first subculture. Cells are "expanded" in culture when they are placed in a growth medium under conditions that facilitate cell growth and/or division, resulting in a larger population of the cells. When cells are expanded in culture, the rate of cell proliferation is sometimes measured by the amount of time needed for the cells to double in number. This is referred to as "doubling time".

The term "standard growth conditions," as used herein refers to culturing of cells at 37°C, in a standard atmosphere comprising 5% CO₂ and relative humidity maintained at about 100%. While the foregoing conditions are useful for culturing, it is to be understood that such conditions are capable of being varied by the skilled artisan who will appreciate the options available in the art for culturing cells.

A "conditioned medium" is a medium in which a specific cell or population of cells has been cultured, and then removed. When cells are cultured in a medium, they may secrete cellular factors that can provide trophic support to other cells. Such trophic factors include, but are not limited to hormones, cytokines, extracellular matrix (ECM), proteins, vesicles, antibodies, and granules. The medium containing the cellular factors is the conditioned medium.

"Differentiation" is the process by which an unspecialized ("uncommitted") or less specialized cell acquires the features of a specialized cell, such as a nerve cell or a muscle cell, for example. A "differentiated" cell is one that has taken on a more specialized ("committed") position within the lineage of a cell. The term "committed," when applied to the process of differentiation, refers to a cell that has proceeded in the differentiation pathway to a point where, under normal circumstances, it will continue to differentiate into a specific cell type or subset of cell types, and cannot, under normal circumstances, differentiate into a different cell type or revert to a less differentiated cell type. "De-differentiation" refers to the process by which a cell reverts to a less specialized (or committed) position within the lineage of a cell. As used herein, the "lineage" of a cell defines the heredity of the cell, i.e. which cells it came from and what cells it can give rise to. The lineage of a cell places the cell within a hereditary scheme of development and differentiation.

In a broad sense, a "progenitor cell" is a cell that has the capacity to create progeny that are more differentiated than itself, and yet retains the capacity to replenish the pool of progenitors. By that definition, stem cells themselves are also progenitor cells, as arc the more immediate precursors to terminally differentiated cells. When referring to the cells of the present invention, as described in greater detail below, this broad definition of progenitor cell may be used. In a narrower sense, a progenitor cell is often defined as a cell that is intermediate in the differentiation pathway, i.e., it arises from a stem cell and is intermediate in the production of a mature cell type or subset of cell types. This type of progenitor cell is generally not able to self-renew. Accordingly, if this type of cell is referred to herein, it will be referred to as a "non-renewing progenitor cell" or as an "intermediate progenitor or precursor cell".

Several terms are used herein with respect to cell or tissue transplantation or cell replacement therapy. The terms "autologous transfer," "autologous transplantation," "autograft" and the like refer to treatments wherein the cell or transplant donor is also the cell or transplant recipient. The terms "allogeneic transfer," "allogeneic transplantation," "allograft" and the like refer to treatments wherein the cell or transplant donor is of the same species as the recipient, but is not the same individual. A cell transfer in which the donor's cells have been histocompatibly matched with a recipient is sometimes referred to as a "syngeneic transfer". The terms "xenogeneic transfer," "xenogeneic transplantation," "xenograft" and the like refer to treatments wherein the cell or transplant donor is of a different species than the recipient.

As used herein the phrase "neural cell" includes both nerve cells (i.e., neurons, e.g., uni-, bi-, or multipolar neurons) and their precursors and glial cells (e.g., macroglia such as oligodendrocytes, Schwann cells, and astrocytes, or microglia) and their precursors.

The cells used in the present invention are generally referred to as "postpartum cells" or "postpartum-derived cells" (PPDC(s))." The cells are more specifically "umbilicus-derived cells" or "umbilical cord-derived cells" (UDC(s)), or "umbilical cord tissue-derived cells" (UTC(s)). In addition, the cells may be described as being stem or progenitor cells, the latter term being used in the broad sense. The term "derived" is used to indicate that the cells have been obtained from their biological source and grown or otherwise manipulated *in vitro* (e.g., cultured in a growth medium to expand the population and/or to produce a cell line). The *in vitro* manipulations of umbilical stem cells and the unique features of the umbilicus-derived cells of the present invention are described in detail below.

The term "isolate" as used herein generally refers to a cell which has been separated from its natural environment. This term includes gross physical separation from its natural environment, *e.g*., removal from the donor animal. In preferred embodiments, an isolated cell is not present in a tissue, *i.e.,* the cell is separated or dissociated from the neighboring cells with which it is normally in contact. Preferably, cells are administered as a cell suspension. As used herein, the phrase "cell suspension" includes cells which are in contact with a medium and which have been dissociated, *e.g*., by subjecting a piece of tissue to gentle trituration.

As used herein, the term "growth medium" generally refers to a medium sufficient for the culturing of umbilical cord tissue-derived cells. In particular, one medium for the culturing of the cells of the invention comprises Dulbecco's Modified Essential Media (DMEM). Particularly preferred is DMEM-Low glucose (DMEM-LG) (Invitrogen, Carlsbad, Ca.). The DMEM-LG is preferably supplemented with serum, most preferably fetal bovine serum or human serum. Typically, 15% (v/v) fetal bovine serum (*e.g*. defined fetal bovine serum, Hyclone, Logan Ut.) is added, along with antibiotics/antimycotics (preferably 100 Unit/milliliter penicillin, 100 milligrams/milliliter streptomycin, and 0.25 microgram/milliliter amphotericin B; Invitrogen, Carlsbad, Ca.), and 0.001% (v/v) 2-mercaptoethanol (Sigma, St. Louis Mo.). In some cases different growth media are used or different supplementations are provided, and these are normally indicated in the text as supplementations to growth medium. In certain chemically-defined media the cells may be grown without serum present at all. In such cases, the cells may require certain growth factors, which can be added to the medium to support and sustain the cells. Presently preferred factors to be added for growth in serum-free media include one or more of bFGF, EGF, IGF-I, and PDGF. In more preferred embodiments, two, three or all four of the factors are added to serum free or chemically defined media. In other embodiments, LIF is added to serum-free medium to support or improve growth of the cells.

The term "cell line" generally refers to a population of cells formed by one or more subcultivations of a primary cell culture. Each round of subculturing is referred to as a passage. When cells are subcultured, they are referred to as having been "passaged." A specific population of cells, or a cell line, is sometimes referred to or characterized by the number of times it has been passaged. For example, a cultured cell population that has been passaged ten times may be referred to as a P10 culture. The primary culture, *i.e.,* the first culture following the isolation of cells from tissue, is designated P0. Following the first subculture, the cells are described as a secondary culture (P1 or passage 1). After the second subculture, the cells become a tertiary culture (P2 or passage 2), and so on. It will be understood by those of skill in the art that there may be many population doublings during the period of passaging; therefore, the number of population doublings of a culture is greater than the passage number. The expansion of cells (*i.e.,* the number of population doublings) during the period between passaging depends on many factors, including, but not limited to, the seeding density, substrate, medium, growth conditions, and time between passaging.

The terms "pharmaceutically acceptable carrier" or "pharmaceutically acceptable medium," which may be used interachangeably with the terms "biologically compatible carrier" or "biologically compatible medium," generally refer to reagents, cells, compounds, materials, compositions, and/or dosage forms that are not only compatible with the cells and other agents to be administered therapeutically, but also are suitable for use in contact with the tissues of human beings and animals without excessive toxicity, irritation, allergic response, or other complication commensurate with a reasonable benefit/risk ratio. As described in greater detail herein, pharmaceutically acceptable carriers suitable for use in the present invention include liquids, semi-solid (*e.g.,* gels) and solid materials (*e.g.,* cell scaffolds and matrices, tubes, sheets and other such materials known in the art and described in greater detail herein). These semi-solid and solid materials may be designed to resist degradation within the body (non-biodegradable) or they may be designed to degrade within the body (biodegradable, bioerodable). A biodegradable material may further be bioresorbable or bioabsorbable, *i.e.,* it may be dissolved and absorbed into bodily fluids (water-soluble implants are one example), or degraded and ultimately eliminated from the body, either by conversion into other materials or breakdown and elimination through natural pathways. The biodegradation rate can vary according to the desired release rate once implanted in the body.

The term "matrix" as used herein generally refers to biodegradable and/or bioresorbable materials that are administrated with the cells to a patient. The matrix may act as a temporary scaffold until replaced by newly grown cells. In some embodiments, the matrix may provide for sustained the release of neurotrophic factors or other agents used in conjunction with the cells and may provide a structure for developing tissue growth in the patient. In other embodiments, the matrix simply provides a temporary scaffold for the developing tissue. The matrix can be in particulate form (macroparticles greater than 10 microns in diameter or microparticles less than 10 microns in diameter), or it can be in the form of a structurally stable, three-dimensional implant (e.g., a scaffold). The matrix can be a slurry, hydrogel or alternatively a three dimensional structure such as a cube, cylinder, tube, block, film, sheet or an appropriate anatomical form.

The term "scaffold" as used herein generally refers to a three dimensional porous structure that provides a template for cell growth. A scaffold is made of biodegradable and/or bioresorbable materials that degrade over time within the body. The length of time taken for the scaffold to degrade may depend upon the molecular weight of the materials. Thus, higher molecular weight material may result in polymer scaffolds which retain their structural integrity for longer periods of time; while lower molecular weights result in both slower release and shorter scaffold lives. The scaffold may be made by any means known in the art. Examples of polymers which can be used to form the scaffold include natural and synthetic polymers.

In some embodiments of the invention, the scaffold may be infused with, coated with, or comprised of a population of cells, growth factors, or other nutrients to promote cell growth. In some preferred embodiments, the scaffold contains growth inducing agents including neurotrophins. Further, the growth inducing agents may be synthetic or naturally produced, and may be a fragment, derivative or analog of a growth inducing agent.

"Neurotrophic factor" or "trophic factor" is defined as a substance that promotes survival, growth, proliferation and/or maturation of a cell, or stimulates increased activity of a cell.

### SPECIFIC EMBODIMENTS

In its various embodiments described herein, the present invention features pharmaceutical compositions for treatment of chronic and neuropathic pain. These pharmaceutical compositions are designed to stimulate and support neural tissue growth or healing, and to improve the regeneration and repair of tissues surrounding the neural tissue, including but not limited to, dermal tissue, vascular tissue, connective tissue, cartilage, adipose tissue, muscle tissue, tendons or ligaments.

The cells of the invention include, progenitor cells and cell populations derived from, umbilicus tissue. A more detailed explanation of preferred cells may be found below.

### Cells

The description of the isolation and characterization of the preferred cells of the invention are described in U.S. Patent Publication Nos. 2005/0032209, 2005/0058631 and 2005/0054098.

In some embodiments, the cells are stem cells. Stem cells are undifferentiated cells defined by the ability of a single cell both to self-renew and to differentiate to produce progeny cells, including self-renewing progenitors, non-renewing progenitors and terminally differentiated cells.

The stem cells are umbilical cord tissue-derived cells. To isolate umbilical cord tissue-derived cells, an umbilical cord is recovered upon or shortly after termination of either a full-term or pre-term pregnancy, for example, after expulsion of after birth. The umbilical cord tissue may be transported from the birth site to a laboratory in a sterile container such as a flask, beaker, culture dish, or bag. The container may have a solution or medium, including but not limited to a salt solution, such as Dulbecco's Modified Eagle's Medium (DMEM) (also known as Dulbecco's Minimal Essential Medium) or phosphate buffered saline (PBS), or any solution used for the transportation of organs used for transplantation, such as University of Wisconsin solution or perfluorochemical solution. One or more antibiotic and/or antimycotic agents, such as, but not limited to, penicillin, streptomycin, amphotericin B, gentamicin, and nystatin, may be added to the medium or buffer. The umbilical cord tissue may be rinsed with an anticoagulant solution such as heparin-containing solution. It is preferable to keep the tissue at about 4 to about 10°C prior to extraction of the cells. It is even more preferable that the tissue not be frozen prior to extraction of the cells.

The umbilical cord tissue-derived cells are preferably isolated in an aseptic environment. The umbilical cord may be separated from the placenta by means known in the art. Blood and debris are preferably removed from the postpartum tissue prior to isolation of umbilical cord tissue-derived cells. For example, the postpartum tissue may be washed with buffer solution, including but not limited to phosphate buffered saline. The wash buffer also may comprise one or more antimycotic and/or antibiotic agents, including but not limited to penicillin, streptomycin, amphotericin B, gentamicin, and nystatin.

Postpartum tissue comprising a whole umbilicus or a fragment or section thereof is preferably disaggregated by mechanical force (mincing or shear forces). In a presently preferred embodiment, the isolation procedure may also utilize an enzymatic digestion process. Many enzymes are known in the art to be useful for the isolation of individual cells from complex tissue matrices to facilitate growth in culture. Digestion enzymes range from weakly digestive *(e.g.* deoxyribonucleases and the neutral protease, dispase) to strongly digestive *(e.g.* papain and trypsin), and are available commercially. A nonexhaustive list of such enzymes includes mucolytic enzyme activities, metalloproteases, neutral proteases, serine proteases (such as trypsin, chymotrypsin, or elastase), and deoxyribonucleases. Presently preferred are enzyme activities selected from metalloproteases, neutral proteases and mucolytic activities. For example, collagenases are known to be useful for isolating various cells from tissues. Deoxyribonucleases can digest single-stranded DNA and can minimize cell-clumping during isolation. Preferred methods involve enzymatic treatment with collagenase and dispase, or collagenase, dispase, and hyaluronidase. The skilled artisan will appreciate that many such enzyme treatments are known in the art for isolating cells from various tissue sources, and is well-equipped to assess new or additional enzymes or enzyme combinations for their utility in isolating the cells of the invention. Preferred enzyme treatments can be from about 0.5 to 2 hours long or longer. In other preferred embodiments, the tissue is incubated at 37°C during the enzyme treatment of the dissociation step.

Methods for the selection of the most appropriate culture medium, medium preparation, and cell culture techniques are well known in the art and are described in a variety of sources, including Doyle et al., (eds.), 1995, Cell & Tissue Culture Laboratory Procecdures, John Wiley & Sons, Chichester; and Ho and Wang (eds.), 1991, Animal Cell Bioreactors, Butterworth-Heinemann, Boston.

In some forms of the invention, the cells are passaged or removed to a separate culture vessel containing fresh medium of the same or a different type as that used initially, where the population of cells can be mitotically expanded. The cells of the invention may be used at any point between passage 0 and senescence. The cells preferably are passaged between about 3 and about 25 times, more preferably are passaged about 4 to about 12 times and preferably are passaged 10 or 11 times. Cloning and/or subcloning may be performed to confirm that a clonal population of cells has been isolated.

In some aspects of the invention, the different cell types present in postpartum tissue are fractionated into subpopulations from which the umbilical cord tissue-derived cells can be isolated. Fractionation or selection may be accomplished using standard techniques for cell separation. Such techniques include, but are not limited to, enzymatic treatment to dissociate postpartum tissue into its component cells, followed by cloning and selection of specific cell types, including, but not limited to, selection based on morphological and/or biochemical markers; selective growth of desired cells (positive selection), selective destruction of unwanted cells (negative selection); separation based upon differential cell agglutinability in the mixed population as, for example, with soybean agglutinin; freeze-thaw procedures; differential adherence properties of the cells in the mixed population; filtration; conventional and zonal centrifugation; centrifugal elutriation (counter-streaming centrifugation); unit gravity separation; countercurrent distribution; electrophoresis; and fluorescence activated cell sorting (FACS).

The culture medium is changed as necessary. For example, by carefully aspirating the medium from the dish with a pipette and replenishing with fresh medium. Incubation is continued until a sufficient number or density of cells accumulates in the dish. Thereafter, any original explanted tissue sections that exist may be removed and the remaining cells separated from the dish by trypsinization using standard techniques or by using a cell scraper. After trypsinization the cells are collected, removed to fresh medium and incubated as above. In some embodiments, the medium is changed at least once at approximately 24 hours post-trypsinization to remove any floating cells. The cells remaining in culture are considered to be umbilical cord tissue-derived cells.

Umbilical cord tissue-derived cells may be cryopreserved. Accordingly, in a preferred embodiment described in greater detail below, umbilical cord tissue-derived cells for autologous transfer (for either the mother or child) may be derived from appropriate postpartum tissues following the birth of a child, then cryopreserved so as to be available in the event they are later needed for transplantation.

Umbilical cord tissue-derived cells may be characterized, for example, by growth characteristics (*e.g*., population doubling capability, doubling time, passages to senescence), karyotype analysis (*e.g*., normal karyotype; maternal or neonatal lineage), flow cytometry *(e.g.,* FACS analysis), immunohistochemistry and/or immunocytochemistry *(e.g.,* for detection of epitopes), gene expression profiling (*e.g*., gene chip arrays; polymerase chain reaction (for example, reverse transcriptase PCR, real time PCR, and conventional PCR)), protein arrays, protein secretion (*e.g*., by plasma clotting assay or analysis of PDC-conditioned medium, for example, by enzyme linked immunosorbent assay (ELISA), mixed lymphocyte reaction (*e.g*., as measure of stimulation of PBMCs), and/or other methods known in the art.

Examples of umbilical cord tissue-derived cells were deposited with the American Type Culture Collection on June 10, 2004, and assigned ATCC Accession Numbers as follows: (1) strain designation UMB 022803 (P7) was assigned Accession No. PTA-6067; and (2) strain designation UMB 022803 (P17) was assigned Accession No. PTA-6068.

In various embodiments, the umbilical cord tissue-derived cells possess one or more of the following growth features: (1) they require L-valine for growth in culture; (2) they are capable of growth in atmospheres containing oxygen from about 5% to about 20%; (3) they have the potential for at least about 40 doublings in culture before reaching senescence; and (4) they attach and expand tissue culture vessels that are uncoated or that are coated with gelatin, laminin, collagen, polyornithine, vitronectin or fibronectin.

In certain embodiments the umbilical cord tissue-derived cells possess a normal karyotype, which is maintained as the cells are passaged. Methods for karyotyping are available and known to those of skill in the art.

In other embodiments, the umbilical cord tissue-derived cells may be characterized by production of certain proteins, including: (1) production of at least one of tissue factor, vimentin, and alpha-smooth muscle actin; and (2) production of at least one of: CD 10, CD13, CD44, CD73, CD90, PDGFr-alpha, PD-L2 and HLA-A,B,C cell surface markers, as detected by flow cytometry. In other embodiments, the umbilical cord tissue-derived cells are/may be characterized by lack of production of at least one of: CD31, CD34, CD45, CD80, CD86, CD117, CD141, CD178, B7-H2, HLA-G, and HLA-DR,DP,DQ cell surface markers, as detected by flow cytometry. Particularly preferred in some applications are cells that produce at least two of: tissue factor; vimentin; and alpha-smooth muscle actin. More preferred are those cells producing all three of the proteins: tissue factor; vimentin; and alpha-smooth muscle actin.

In other embodiments, the umbilical cord tissue-derived cells may be characterized by gene expression relative to a human cell that is a fibroblast, a mesenchymal stem cell, or an iliac crest bone marrow cell, which is increased for a gene encoding at least one of: interleukin 8; reticulon 1; chemokine (C-X-C motif) ligand 1 (melonoma growth stimulating activity, alpha); chemokine (C-X-C motif) ligand 6 (granulocyte chemotactic protein 2); chemokine (C-X-C motif) ligand 3; and tumor necrosis factor, alpha-induced protein 3.

In yet other embodiments, the umbilical cord tissue-derived cells may be characterized by gene expression relative to a human cell that is a fibroblast, a mesenchymal stem cell, or an iliac crest bone marrow cell, which is reduced for a gene encoding at least one of: short stature homeobox 2; heat shock 27 kDa protein 2; chemokine (C-X-C motif) ligand 12 (stromal cell-derived factor 1); elastin (supravalvular aortic stenosis, Williams-Beuren syndrome); *Homo sapiens* mRNA; cDNA DKFZp586M2022 (from clone DKFZp586M2022); mesenchyme homeo box 2 (growth arrest-specific homeo box); sine oculis homeobox homolog 1 (*Drosophila*); crystallin, alpha B; disheveled associated activator of morphogenesis 2; DKFZP586B2420 protein; similar to neuralin 1; tetranectin (plasminogen binding protein); src homology three (SH3) and cysteine rich domain; cholesterol 25-hydroxylase; runt-related transcription factor 3; interleukin 11 receptor, alpha; procollagen C-endopeptidase enhancer; frizzled homolog 7 (*Drosophila*)*;* hypothetical gene BC008967; collagen, type VIII, alpha 1; tenascin C (hexabrachion); iroquois homeobox protein 5; hephaestin; integrin, beta 8; synaptic vesicle glycoprotein 2; neuroblastoma, suppression of tumorigenicity 1; insulin-like growth factor binding protein 2, 36kDa; *Homo sapiens* cDNA FLJ12280 fis, clone MAMMA1001744; cytokine receptor-like factor 1; potassium intermediate/small conductance calcium-activated channel, subfamily N, member 4; integrin, beta 7; transcriptional co-activator with PDZ-binding motif (TAZ); sine oculis homeobox homolog 2 (Drosophila); KIAA1034 protein; vesicle-associated membrane protein 5 (myobrevin); EGF-containing fibulin-like extracellular matrix protein 1; early growth response 3; distal-less homeo box 5; hypothetical protein FLJ20373; aldo-keto reductase family 1, member C3 (3-alpha hydroxysteroid dehydrogenase, type II); biglycan; transcriptional co-activator with PDZ-binding motif (TAZ); fibronectin 1; proenkephalin; integrin, beta-like 1 (with EGF-like repeat domains); *Homo sapiens* mRNA full length insert cDNA clone EUROIMAGE 1968422; EphA3; KIAA0367 protein; natriuretic peptide receptor C/guanylate cyclase C (atrionatriuretic peptide receptor C); hypothetical protein FLJ14054; Homo sapiens mRNA; cDNA DKFZp564B222 (from clone DKFZp564B222); BCL2/adenovirus E1B 19kDa interacting protein 3-like; AE binding protein 1; and/or cytochrome c oxidase subunit VIIa polypeptide 1 (muscle).

In other embodiments, the umbilical cord tissue-derived cells may be characterized by secretion of at least one of: MCP-1; IL-6; IL-8; GCP-2; HGF; KGF; FGF; HB-EGF; BDNF; TPO; RANTES; MIP1b; 1309; MDC; and TIMP1. In some embodiments, the umbilical cord tissue-derived cells may be characterized by a lack of secretion of at least one of: TGF-beta2; ANG2; MIP1a; PDGFbb; and VEGF, as detected by ELISA.

The umbilical cord tissue-derived cells are derived from umbilical cord tissue substantially free of blood and are capable of self-renewal and expansion in culture. They may require L-valine for growth, can grow in at least about 5% oxygen, and comprise at least one of the following characteristics: (1) the potential for at least about 40 doublings in culture; (2) the ability to attach and expand on an uncoated tissue culture vessel or one coated with gelatin, laminin, collagen, polyornithine, vitronectin, or fibronectin; (3) production ofvimentin and alpha-smooth muscle actin;(4) production of CD10, CD13, CD44, CD73, and CD90; and (5) expression of a gene, which relative to a human cell that is a fibroblast, a mesenchymal stem cell, or an iliac crest bone marrow cell, is increased for a gene encoding interleukin 8 and reticulon 1. Such umbilical cord tissue-derived cells may optionally not produce CD45 and are negative for CD117.

In preferred embodiments, the cell comprises two or more of the above-listed growth, protein/surface marker production, gene expression or substance-secretion characteristics. More preferred are those cells comprising three, four, five or more of the characteristics. Still more preferred are umbilical cord tissue-derived cells comprising six, seven, eight or more of the characteristics. Still more preferred presently are those cells comprising all of above characteristics.

Among cells that are presently preferred for use with the invention in several of its aspects are umbilical cord tissue-derived cells having the characteristics described above and more particularly those wherein the cells have normal karyotypes and maintain normal karyotypes with passaging, and further wherein the cells express each of the markers CD 10, CD 13, CD44, CD73, CD90, PDGFr-alpha, and HLA-A,B,C, and wherein the cells produce the immunologically-detectable proteins which correspond to the listed markers. Still more preferred are those cells which, in addition to the foregoing, do not produce proteins corresponding to any of the markers CD31, CD34, CD45, CD141, or HLA-DR,DP,DQ, as detected by flow cytometry.

Certain cells having the potential to differentiate along lines leading to various phenotypes are unstable and thus can spontaneously differentiate. Presently preferred for use with the invention are cells that do not spontaneously differentiate, for example, along myoblast, skeletal muscle, vascular smooth muscle, pericyte, hemangiogenic, angiogenic, vasculogenic, or vascular endothelial lines. Preferred cells, when grown in growth medium, are substantially stable with respect to the cell markers produced on their surface, and with respect to the expression pattern of various genes, for example, as determined using a medical diagnostic test sold under the trade name GENECHIP (Affymetrix, Inc., Santa Clara, CA). The cells remain substantially constant, for example, in their surface marker characteristics over passaging and through multiple population doublings.

Another aspect provided by the invention features the use of populations of the umbilical cord tissue-derived cells described above. In some embodiments, the cell population is heterogeneous. A heterogeneous cell population of the invention may comprise at least about 5%, 10%, 20%, 30%, 40%, 50%, 60%, 70%, 80%, 90%, or 95% umbilical cord tissue-derived cells of the invention. The heterogeneous cell populations of the invention may further comprise stem cells or other progenitor cells, such as myoblasts or other muscle progenitor cells, hemangioblasts, or blood vessel precursor cells; or it may further comprise fully differentiated skeletal muscle cells, smooth muscle cells, pericytes, or blood vessel endothelial cells. In some embodiments, the population is substantially homogeneous, *i.e.,* comprises substantially only umbilical cord tissue-derived cells (preferably at least about 96%, 97%, 98%, 99% or more umbilical cord tissue-derived cells). The homogeneous cell population of the invention may comprise umbilicus-derived cells. Homogeneous populations of umbilicus-derived cells are preferably free of cells of maternal lineage. Homogeneity of a cell population may be achieved by any method known in the art, for example, by cell sorting (e.g., flow cytometry) or by clonal expansion in accordance with known methods. Thus, preferred homogeneous umbilical cord tissue-derived cell populations may comprise a clonal cell line of umbilical cord tissue-derived cells. Such populations are particularly useful when a cell clone with highly desirable functionality has been isolated.

In one embodiment, the cells are umbilical cord tissue-derived cells that are administered as undifferentiated cells, *i.e.,* as cultured in growth medium. Alternatively, the umbilical cord tissue-derived cells may be administered following exposure in culture to conditions that stimulate differentiation toward a desired neural tissue. In one preferred embodiment, the cells are UTC. In another embodiment, the cells are hUTC.

Further, the population of cells may include more than one type of cell. Indeed, some embodiments include administration of cells which surround and support the neural cell. Such cells may include, but are not limited to, dermal tissue, vascular tissue, connective tissue, cartilage, adipose tissue, muscle tissue, tendons or ligaments.

The general protocol, isolation and characterization of an umbilical cord tissue-derived cell may be found at Examples 5-15.

### Genetically Modified Cells

Cells used in the invention may also be genetically modified to produce therapeutically useful gene products, to produce agents to facilitate or support neural tissue formation, healing and/or growth, or to produce factors to recruit progenitor cells to the area of neural damage.

Genetic modification may be accomplished using any of a variety of vectors including, but not limited to, integrating viral vectors, e.g., retrovirus vector or adeno-associated viral vectors; non-integrating replicating vectors, *e.g.*, papilloma virus vectors, SV40 vectors, adenoviral vectors; or replication-defective viral vectors. Other methods of introducing DNA into cells include the use of liposomes, electroporation, a particle gun, or by direct DNA injection.

For instance, the cells may be genetically engineered to express and/or secrete a foreign molecule (*e.g*., a heterologous molecule not normally made by the cell) or to modify the production of a molecule to treat chronic pain. Such molecules can be produced by the cells upon introduction of heterologous nucleic acid molecules using techniques which are well known in the art.

In one embodiment, the cells of the invention can be modified to express a receptor to a neurotransmitter. In another embodiment, the cells of the invention are modified to produce a neurotransmitter, including but not limited to, serotonin, histamine, gamma aminobutyric acid, glutamate, aspartate, glycine, neuropeptide Y, ATP, GRP, adenosine, epinephrine, neuroepinephrine, dopamine, acetylcholine, melatonin, n-acetylaspartylglutamate, octopamine, tyramine, gastrin, cholecytokinin, vasopressin, oxytocin, neurphysin I and II, pancreatic polypeptide, peptide YY, corticotrophin, dynorphin, endorphin, enkephaline, secretin, motilin, glucagons, vasoactive intestinal peptide, growth hormone-releasing factor, somatostatin, neurokinin A and B, substance P, bombesin, gastric releasing peptide, nitric oxide, carbon monoxide, and anandamide. In yet another embodiment, the cells of the invention are modified to produce a fragment of a neurotransmitter, including but not limited to a fragment from the C' terminus or N' terminus.

In another embodiment a foreign molecule enhances the neuroregenerative capacity of the transplanted cells, aids in reestablishing sensorineural communication of GABA interneurons, and/or aids in reestablishment of the excitatory/inhibitory neurotransmitter balance in the subject

In yet another embodiment, the cells are genetically engineered to express and/or secret foreign molecules that directly reduce pain in the subject, promote success of transplantation (*e.g.*, by downmodulation of an immune response in the subject), and/or promote survival or function of the transplanted cells. Exemplary molecules include, *e.g.,* a neurotrophic factor, or a neuroprotective agent.

In yet another embodiment, unmodified or modified cells can be introduced together with other types of cells genetically modified to perform a useful function. For example, in order to promote growth of neurons the cells can be administered together with other cells which secrete or have been modified to secrete, for example, a neurotrophic factor. Examples of cells that act as carriers of transgenes to a subject include fibroblasts, adrenal chromaffin cells, astrocytes, and myoblasts. Such cells, for example fibroblasts and glial cells, can also be used to deliver retroviruses containing genes such as the herpes simplex thymidine kinase gene, the gene products of which are targets for other therapeutic drugs or agents such as ganciclovir to target cells.

Hosts cells may be transformed or transfected with DNA controlled by, or in operative association with, one or more appropriate expression control elements such as promoter or enhancer sequences, transcription terminators, polyadenylation sites, among others, and a selectable marker. Any promoter may be used to drive the expression of the inserted gene. For example, viral promoters include, but are not limited to, the CMV promoter/enhancer, SV 40, papillomavirus, Epstein-Barr virus or elastin gene promoter. In some embodiments, the control elements used to control expression of the gene of interest can allow for the regulated expression of the gene so that the product is synthesized only when needed *in vivo.* If transient expression is desired, constitutive promoters are preferably used in a non-integrating and/or replication-defective vector. Alternatively, inducible promoters could be used to drive the expression of the inserted gene when necessary. Inducible promoters include, but are not limited to, those associated with metallothionein and heat shock proteins.

Following the introduction of the foreign DNA, engineered cells may be allowed to grow in enriched media and then switched to selective media. The selectable marker in the foreign DNA confers resistance to the selection and allows cells to stably integrate the foreign DNA as, for example, on a plasmid, into their chromosomes and grow to form foci which, in turn, can be cloned and expanded into cell lines. This method can be advantageously used to engineer cell lines that express the gene product.

The cells of the invention may be genetically engineered to "knock out" or "knock down" expression of factors that promote inflammation or rejection at the implant site. Negative modulatory techniques for the reduction of target gene expression levels or target gene product activity levels are discussed below. "Negative modulation," as used herein, refers to a reduction in the level and/or activity of target gene product relative to the level and/or activity of the target gene product in the absence of the modulatory treatment. The expression of a gene native to a skeletal muscle cell, vascular smooth muscle cell, pericyte, vascular endothelial cell, neural cell, or progenitor cells thereof can be reduced or knocked out using a number of techniques including, for example, inhibition of expression by inactivating the gene using the homologous recombination technique. Typically, an exon encoding an important region of the protein (or an exon 5' to that region) is interrupted by a positive selectable marker, *e.g.,* neo, preventing the production of normal mRNA from the target gene and resulting in inactivation of the gene. A gene may also be inactivated by creating a deletion in part of a gene, or by deleting the entire gene. By using a construct with two regions of homology to the target gene that are far apart in the genome, the sequences intervening the two regions can be deleted (Mombaerts et al., Proc. Nat. Acad. Sci. U.S.A., 1991; 88:3084). Antisense, DNAzymes, ribozymes, small interfering RNA (siRNA) and other such molecules that inhibit expression of the target gene can also be used to reduce the level of target gene activity. For example, antisense RNA molecules that inhibit the expression of major histocompatibility gene complexes (HLA) have been shown to be most versatile with respect to immune responses. Still further, triple helix molecules can be utilized in reducing the level of target gene activity. These techniques are described in detail by Davis, L.G. et al. (eds), Basic Methods in Molecular Biology, 2nd ed., 1994, Appleton & Lange, Norwalk, Ct.

In other aspects, the disclosure involves the use of cell lysates and cell soluble fractions prepared from a umbilical cord tissue-derived cells (not claimed). Such lysates and fractions thereof have many utilities. Use of such lysate soluble fractions (*i.e.,* substantially free of membranes) *in vivo,* for example, allows the beneficial intracellular milieu to be used allogeneically in a patient without introducing an appreciable amount of the cell surface proteins most likely to trigger rejection, or other adverse immunological responses. Methods of lysing cells are well-known in the art and include various means of mechanical disruption, enzymatic disruption, or chemical disruption, or combinations thereof. Such cell lysates may be prepared from cells directly in their growth medium, and thus contain secreted growth factors and the like, or they may be prepared from cells washed free of medium in, for example, PBS or other solution. Washed cells may be resuspended at concentrations greater than the original population density if preferred.

In one option whole cell lysates are prepared, *e.g.,* by disrupting cells without subsequent separation of cell fractions. In another option a cell membrane fraction is separated from a soluble fraction of the cells by routine methods known in the art, *e.g.,* centrifugation, filtration, or similar methods.

Cell lysates or cell soluble fractions prepared from populations of postpartum-derived cells may be used as is, further concentrated by, for example, ultrafiltration or lyophilization, or even dried, partially purified, combined with pharmaceutically-acceptable carriers or diluents as are known in the art, or combined with other compounds such as biologicals, for example, pharmaceutically useful protein compositions. Cell lysates or fractions thereof may be used *in vitro* or *in vivo,* alone or, for example, with autologous or syngeneic live cells. The lysates, if introduced *in vivo,* may be introduced locally at a site of treatment, or remotely to provide, for example, needed cellular growth factors to a patient.

In a further option the UTC can be cultured *in vitro* to produce biological products in high yield. An umbilical cord tissue-derived cell that either naturally produces a particular biological product of interest (*e.g*., a trophic factor), or that has been genetically engineered to produce such a biological product, can be clonally expanded using the culture techniques described herein. Alternatively, cells may be expanded in a medium that induces differentiation to a neural cell. In each case, biological products produced by the cell and secreted into the medium can be readily isolated from the conditioned medium using standard separation techniques, *e.g*., such as differential protein precipitation, ion-exchange chromatography, gel filtration chromatography, electrophoresis, and HPLC, to name a few. A "bioreactor" may be used to take advantage of the flow method for feeding, for example, a three-dimensional culture *in vitro*. Essentially, as fresh media is passed through the three-dimensional culture, the biological product is washed out of the culture and may then be isolated from the outflow, as above.

Alternatively, a biological product of interest may remain within the cell and, thus, its collection may require that the cells be lysed, as described above. The biological product may then be purified using any one or more of the above-listed techniques.

In other options, the disclosure involves the use of conditioned medium from cultured umbilical cord tissue-derived cells for use *in vitro* and *in vivo* as described below. Use of the umbilical cord tissue-derived cells conditioned medium allows the beneficial trophic factors secreted by the umbilical cord tissue-derived cells to be used allogeneically in a patient without introducing intact cells that could trigger rejection, or other adverse immunological responses. Conditioned medium is prepared by culturing cells in a culture medium, then removing the cells from the medium.

Conditioned medium prepared from populations of umbilical cord-derived cells may be used as is, further concentrated, for example, by ultrafiltration or lyophilization, or even dried, partially purified, combined with pharmaceutically acceptable carriers or diluents as are known in the art, or combined with other compounds such as biologicals, for example, pharmaceutically useful protein compositions. Conditioned medium may be used *in vitro* or *in vivo,* alone or combined with autologous or syngeneic live cells, for example. The conditioned medium, if introduced *in vivo,* may be introduced locally at a site of treatment, or remotely to provide needed cellular growth or trophic factors to a patient.

In another option an extracellular matrix (ECM) produced by culturing the umbilical cord tissue-derived cells on liquid, solid or semi-solid substrates is prepared, collected and utilized as an alternative to implanting live cells into a subject in need the repair , replacement, or regeneration of neural cells. The umbilical cord tissue-derived cells are cultured *in vitro,* on a three dimensional framework as described elsewhere herein, under conditions such that a desired amount of ECM is secreted onto the framework. The cells comprising the new tissue are removed, and the ECM processed for further use, for example, as an injectable preparation. To accomplish this, cells on the framework are killed and any cellular debris is removed from the framework. This process may be carried out in a number of different ways. For example, the living tissue can be flash-frozen in liquid nitrogen without a cryopreservative, or the tissue can be immersed in sterile distilled water so that the cells burst in response to osmotic pressure.

Once the cells have been killed, the cellular membranes may be disrupted and cellular debris removed by treatment with a mild detergent rinse, such as EDTA, CHAPS or a zwitterionic detergent. Alternatively, the tissue can be enzymatically digested and/or extracted with reagents that break down cellular membranes and allow removal of cell contents. Examples of such enzymes include, but are not limited to, hyaluronidase, dispase, proteases, and nucleases. Examples of detergents include non-ionic detergents such as, for example, alkylaryl polyether alcohol (TRITON X-100), octylphenoxy polyethoxy-ethanol (Rohm and Haas, Philadelphia, Pa.), BRIJ-35, a polyethoxyethanol lauryl ether (Atlas Chemical Co., San Diego, Ca.), polysorbate 20 (TWEEN 20), a polyethoxyethanol sorbitan monolaureate (Rohm and Haas, Philadelphia, Pa.), polyethylene lauryl ether (Rohm and Haas, Philadelphia, Pa.); and ionic detergents such as sodium dodecyl sulfate, sulfated higher aliphatic alcohols, sulfonated alkanes and sulfonated alkylarenes containing 7 to 22 carbon atoms in a branched or unbranched chain.

The collection of the ECM can be accomplished in a variety of ways, depending at least in part on whether the new tissue has been formed on a three-dimensional framework that is biodegradable or non-biodegradable, as in the case of metals. For example, if the framework is non-biodegradable, the ECM can be removed by subjecting the framework to sonication, high pressure water jets, mechanical scraping, or mild treatment with detergents or enzymes, or any combination of the above.

If the framework is biodegradable, the ECM can be collected, for example, by allowing the framework to degrade or dissolve in solution. Alternatively, if the biodegradable framework is composed of a material that can itself be injected along with the ECM, the framework and the ECM can be processed in toto for subsequent injection. Alternatively, the ECM can be removed from the biodegradable framework by any of the methods described above for collection of ECM from a non-biodegradable framework. All collection processes are preferably designed so as not to denature the ECM.

After it has been collected, the ECM may be processed further. For example, the ECM can be homogenized to fine particles using techniques well known in the art such as by sonication, so that it can pass through a surgical needle. The components of the ECM can also be crosslinked, if desired, by gamma irradiation. Preferably, the ECM can be irradiated between 0.25 to 2 mega rads to sterilize and crosslink the ECM. Chemical crosslinking using agents that are toxic, such as glutaraldehyde, is possible but not generally preferred.

The amounts and/or ratios of proteins, such as the various types of collagen present in the ECM, may be adjusted by mixing the ECM produced by the cells of the invention with ECM of one or more other cell types. In addition, biologically active substances such as proteins, growth factors and/or drugs, can be incorporated into the ECM. Exemplary biologically active substances include tissue growth factors, such as TGF-beta, and the like, which promote healing and tissue repair at the site of the injection. Such additional agents may be utilized in any of the embodiments described herein above, e.g., with whole cell lysates, soluble cell fractions, or further purified components and products produced by the umbilical cord tissue-derived cells.

### Cell Culture

The isolated cells may be used to initiate, or seed, cell cultures. Isolated cells are transferred to sterile tissue culture vessels either uncoated or coated with extracellular matrix or ligands such as laminin, collagen (native, denatured or crosslinked), gelatin, fibronectin, and other extracellular matrix proteins. The cells are cultured in any culture medium capable of sustaining growth of the cells such as, but not limited to, DMEM (high or low glucose), advanced DMEM, DMEM/MCDB 201, Eagle's basal medium, Ham's F10 medium (F10), Ham's F-12 medium (F12), Iscove's modified Dulbecco's medium, mesenchymal stem cell growth medium (MSCGM), DMEM/F12, RPMI 1640, and serum/media free medium sold under the trade name CELL-GRO-FREE (Mediatech, Inc., Herndon, Va.). The culture medium may be supplemented with one or more components including, for example, fetal bovine serum (FBS), preferably about 2-15% (v/v); equine serum (ES); human serum (HS); beta-mercaptoethanol (BME or 2-ME), preferably about 0.001% (v/v); one or more growth factors, for example, platelet-derived growth factor (PDGF), epidermal growth factor (EGF), fibroblast growth factor (FGF), vascular endothelial growth factor (VEGF), insulin-like growth factor-1 (IGF-1), leukocyte inhibitory factor (LIF) and erythropoietin (EPO); amino acids, including L-valine; and one or more antibiotic and/or antimycotic agents to control microbial contamination, such as penicillin G, streptomycin sulfate, amphotericin B, gentamicin, and nystatin, either alone or in combination. The culture medium preferably comprises growth medium. (e.g. DMEM-Low glucose, serum, BME and an antibotic agent.)

The cells are seeded in culture vessels at a density to allow cell growth. In a preferred embodiment, the cells are cultured at about 0 to about 5 percent by volume CO₂ in air. In some preferred embodiments, the cells are cultured at about 2 to about 25 percent O₂ in air, preferably about 5 to about 20 percent O₂ in air. The cells preferably are cultured at a temperature of about 25 to about 40°C and more preferably are cultured at 37°C. The cells are preferably cultured in an incubator. The medium in the culture vessel can be static or agitated, for example, using a bioreactor. In some embodiments, the cells are grown under low oxidative stress (e.g., with addition of glutathione, vitamin C, catalase, vitamin E, N-acetylcysteine). "low oxidative stress," as used herein, refers to conditions of no or minimal free radical damage to the cultured cells.

### Systemic Administration

The population of cells is administered systemically to treat pain and/or site of neural damage. The administration site may be any determined by the medical professional to be best, and thus may be intravenous, intramusculature, intraperitoneal, and the like. The cells may be administered by any means including, but not limited to, injection and infusion.

Specific embodiments of the invention are directed to systemic administration of a population of cells for the direct repair, regeneration, replacement of, or the support of the repair, regeneration, or replacement of neural cells for the treatment of neural damage, injury and/or pain.

Routes of systemic administration of the cells of the invention or compositions thereof include, but are not limited to, intravenous, interperitoneally, intraarterial, or via syringes with needles or catheters with or without pump devices. The migration of the population of cells can be guided by movement of fluids within the individual's body, such as blood or lymph movement, as well as chemical signals, growth factors, and the like.

In one specific embodiment, a delivery catheter may be used to deliver the population of cells into a delivery device which facilitates introduction by e.g., injection, of the cells into the subjects. Such delivery devices include tubes, e.g., catheters, for injecting cells and fluids into the body of a recipient subject. Further, the population of cells can be administered in any physiologically compatible carrier, such as a buffered saline solution. Pharmaceutically acceptable carriers and diluents include saline, aqueous buffer solutions, solvents and/or dispersion media. Preferably, the solution is stable under the conditions of manufacture and storage and preserved against the contaminating action of microorganisms such as bacteria and fungi through the use of antimicrobials and antifungals including, for example, parabens, chlorobutanol, phenol, ascorbic acid, thimerosol, and the like. Solutions of the invention can be prepared by using a pharmaceutically acceptable carrier or diluent and, as required, other ingredients enumerated above, followed by filtered sterilization, and then incorporating the population of cells as described herein.

For both locally and systemically administered cells, the dosage forms and regimes for administering the population of cells or any of the other therapeutic or pharmaceutical compositions described herein are developed in accordance with good medical practice, taking into account the condition of the individual patient, e.g., nature and extent of the neural injury or damage, age, sex, body weight and general medical condition, and other factors known to medical practitioners. Thus, the effective amount of a pharmaceutical composition to be administered to a patient is determined by these considerations as known in the art.

If the population of cells used by the medical practitioner is umbilical cord tissue-derived cells, then transplantation with allogeneic, or even xenogeneic, cells may be tolerated in some instances as these cells have been shown not to stimulate allogeneic PBMCs in a mixed lymphocyte reaction. Accordingly, it is recognized that the cells themselves provide an immunosuppressant effect, thereby preventing host rejection of the transplanted population of cells. In such instances, pharmacological immunosuppression during cell therapy may not be necessary.

However, in other instances it may be desirable or appropriate to pharmacologically immunosuppress a patient prior to initiating cell therapy. This may be accomplished through the use of systemic or local immunosuppressive agents, or it may be accomplished by delivering the cells in an encapsulated device, as described above. These and other means for reducing or eliminating an immune response to the transplanted cells are known in the art. As an alternative, the population of cells may be genetically modified to reduce their immunogenicity, as mentioned above.

In addition, survival of a transplanted population of cells in a living patient can be determined through the use of a variety of scanning techniques, e.g., computerized axial tomography (CAT or CT) scan, magnetic resonance imaging (MRI) or positron emission tomography (PET) scans. Determination of transplant survival can also be done *post mortem* by removing the neural tissue and surrounding tissues, and examining it visually or through a microscope. Alternatively, cells can be treated with stains that are specific for neural tissue, or its surrounding tissues. Transplanted cells can also be identified by prior incorporation of tracer dyes such as rhodamine- or fluorescein-labeled microspheres, fast blue, ferric microparticles, bisbenzamide or genetically introduced reporter gene products, such as beta-galactosidase or beta-glucuronidase.

### Agents or Compounds Administered with the Population of Cells

The cells of the present invention can be incubated and/or treated at any stage in their preparation for transplantation, e.g., during dissection, limited digestion, dissociation, plating, and/or production of cell suspensions for transplantation, with a number of agents or factors which promote the survival, growth, differentiation, and/or integration of the cells *in vitro* and/or in the recipient subject, or which further aid in the treatment of chronic pain. The administration of additional agents can begin prior to transplantation of cells, can begin at the time of transplantation, or can begin after transplantation. The administration of additional agents can be limited in duration (e.g., can consist of a single administration of the agent) or can be of prolonged duration (e.g., can be given to the subject repeatedly over a long period of time).

In some embodiments, one or more compounds or components are administered in parallel, sequentially or formulated directly with the population of cells. Examples of other components that may be added to the administered cells include, but are not limited to: (1) other neurotrophic factors such as brain derived neurotrophic factor, ciliary neurotrophic factor, neurotrophin-3, neurotrophin 4/5, nerve growth factor, acidic fibroblast growth factor, basic fibroblast growth factor, platelet-derived growth factor, thyrotropin releasing hormone, epidermal growth factor, amphiregulin, transforming growth factor, transforming growth factor, insulin-like growth factor; (2) selected extracellular matrix components, such as one or more types of collagen known in the art, and/or growth factors, platelet-rich plasma, and drugs (alternatively, umbilical cord tissue-derived cells may be genetically engineered to express and produce growth factors); (3) anti-apoptotic agents (*e.g.,* erythropoietin (EPO), EPO mimetibody, thrombopoietin, insulin-like growth factor (IGF)-I, IGF-II, hepatocyte growth factor, caspase inhibitors); (4) anti-inflammatory compounds (e.g., p38 MAP kinase inhibitors, TGF-beta inhibitors, statins, IL-6 and IL-1 inhibitors, Pemirolast, Tranilast, Remicade (Centocor, Inc., Malvern, Pa.), Sirolimus, and non-steroidal anti-inflammatory drugs (NSAIDS) (such as Tepoxalin, Tolmetin, and Suprafen); (5) immunosuppressive or immunomodulatory agents, such as calcineurin inhibitors, mTOR inhibitors, antiproliferatives, corticosteroids and various antibodies; (6) local anesthetics; and (7) other angiogenic factors, angiogenic drugs, or myoregenerative or myooprotective factors or drugs.

In one embodiment, such agents or factors can be added at the site of transplantation in the recipient subject after the cells of the invention have been transplanted therein. In some instances, for example, these agents can minimize or counteract detrimental effects on the cells resulting from the procedures used to prepare the cells for transplantation. For example, cells prepared for transplantation, may experience cellular trauma and/or hypoxia which leads to the production of reactive oxygen species (ROS) such as superoxide radical anion, hydrogen peroxide, and the hydroxyl free radical. ROS are known to adversely affect cell function, most likely by affecting a variety of membrane and intracellular components including ion channels, membrane lipids, transport mechanisms such as the Na/K ATPase and Na/glutamate exchange transport and cytosolic enzymes such as glutamine synthase. In addition, reactive oxygen species provoke membrane lipid peroxidation, and consequently may reduce the survival of the cells in the transplants.

To minimize and/or counteract the adverse effects of these types of oxidative stress during preparation of the cells for transplantation, the cells of the present invention can be incubated and/or treated with antioxidants at any stage during the preparation. Examples of such antioxidants include the enzyme antioxidants superoxide dismutase (SOD) and glutathione peroxidase and agents which promote glutathione formation, e.g. N-acetyl cysteine (NAC). Other antioxidants includes lazaroids, e.g., U-74389G and U-83836E, which are aminosteroids that are designed to localize in the cell membrane and inhibit lipid peroxidation while scavenging free radicals. Other examples of antioxidants which can be added to the cell cultures and cell suspensions include TGF, vitamin E, vitamin C, beta carotene, and other compounds which scavenge ROS, inhibit the production of ROS, and/or inhibit lipid peroxidation.

Antioxidant enzymes, such as SOD, scavenge ROS and prevent the reaction of superoxide with nitric oxide to form peroxynitrite anion, which has been shown to be toxic to cultured cells. These enzymes can be incubated with the cells of the invention as described above. Another method, of introducing these enzymes into the cellular preparations of the present invention, is to genetically modify the cells to contain the nucleic acid encoding such enzymes. The genetically modified cells can then produce agents which enhance the survival, growth, and differentiation of the grafted cells in the recipient subject. For example, cells of the invention can be transfected with the human gene for Cu/Zn superoxide dismutase, a pivotal enzyme in the detoxification of oxygen free radicals, which results in the transfected cells expressing SOD and, consequently, efficiently detoxifying ROS generated during tissue preparation and implantation to thereby increase transplanted cell survival.

In addition, the oxidative environment of the cells *in vitro* can be modified to inhibit cellular oxidative stress. For example, before transplantation, the partial pressure of oxygen in the cells environment can be decreased from the normal oxygen partial pressure, *i.e.,* approximately 150 torr 02, to a decreased oxygen partial pressure, *i.e.,* 38 torr 02 (about 5% 02). This method of decreasing oxidative stress can be combined with treatment of the cells with one or more of the above-described antioxidants.

Inhibitors of NOS, such as gangliosides, FK506, and cyclosporine A, can be added to the cell preparations to inhibit the production of NO, thereby decreasing the production of peroxynitrite and its derivatives. Superoxide dismutase is another agent which can decrease the adverse effects of overproduction of NO and the toxic effects it mediates.

To prevent trauma and its associated adverse effects, e.g., membrane peroxidation, free radical induced cell damage induced by preparation of the cells of the invention for implantation, the cells of the invention can be transfected with nucleic acids encoding antiapoptotic gene products such as the bcl-2 and/or the crmA gene product. Further, the transfected cells of the invention can be treated with agents which unregulate the expression or function of these gene products, e.g., TGF1 and TGF3 which upregulate the expression of bcl-2, nerve growth factor (NGF) and platelet-derived growth factor (PDGF). Further, the cells of the invention can also be transfected with nucleic acid encoding these factors.

To further promote the survival of the cells of the invention in the recipient subject, the cells can be transplanted in conjunction with an angiogenic agent or transfected with nucleic acid encoding an angiogenic agent. Upon transplantation, the angiogenic agent promotes the ingrowth of blood vessels into the population of cells. As a result of this vessel ingrowth, the transplanted cells obtain sufficient nutrients to proliferate and survive within the recipient subject. Many growth factors exhibit angiogenic activity. For example, vascular endothelial growth factor (VEGF), PDGF, acidic and basic fibroblast growth factor (FGF), epidermal growth factor (EGF), and K-FGF possess angiogenic activity and can be used in the methods of the invention to encourage blood vessel ingrowth into the transplanted cells of the invention.

Other factors, such as neurotrophic factors, which contribute to neural development, nerve fiber formation, and maintenance of neurons can be added to the cells of the invention *in vitro* during preparation for transplantation and/or to the cell suspension itself for introduction into the individual subject along with the cells of the invention. The cells of the invention can also be genetically modified to produce such neurotrophic factors as described herein. The neurotrophic factor which is added to the cells of the present invention can be selected based on the presence of its receptors on the cells which are to be transplanted. For example, mesencephalic cells possess receptors for the following neurotrophic factors: glial cell line-derived neurotrophic factor (GDNF), which promotes the survival of, morphological differentiation of, and high affinity dopamine uptake in mesencephalic cells; brain-derived neurotrophic factor (BDNF); ciliary neurotrophic factor (CNTF), which prevents axotomy induced degeneration of mesencephalic cells; midkine, which promotes the survival and differentiation of mesencephalic cells; EGF, which increases survival and maturation of mesencephalic cells; insulin-like growth factor I and II and insulin; acidic FGF; basic FGF, which induce a significant increase in the number of neurite-bearing cells as well as in the degree of their fiber network; neurotrophin-3 (NT-3) and neurotrophin 4/5 (NT-4/5); and transforming growth factor-2 (TGF2) and transforming growth factor-3 (TGF3).

Neurotrophic factors which promote the survival of neural cells can be selected based on the presence of receptors on the cells. Receptors for basic FGF, BDNF, NT-3 and NT-4/5 can be found on certain neural cells. Thus, in one embodiment, the cells of the invention can be transfected with the nucleic acids encoding one or more of these factors. In another embodiment, one or more of these factors can be added to the preparation of neural cells prior to transplantation. These neurotrophic factors enhance the survival of the cells of the invention in the recipient subject. Similarly, neurotrophic factors which exhibit specificity for cortical cells, and consequently, which can be used to promote the survival of such cells upon engraftment into a recipient subject, include nerve growth factor (NGF), which prevents, for example, atrophy of axotomized forebrain cholinergic neurons; BDNF, and NT-3 and NT-4/5.

In another embodiment, the neurotrophic factors described herein can be used together or in combination with other compounds, such as neurotransmitters, to augment their neurotrophic effects. In addition, it is contemplated that various combinations of neurotrophic factors described herein can act synergistically and, therefore, can be used together to promote survival of the transplanted cells of the invention.

Certain drugs also possess neurotrophic activity. Examples of such drugs include FK506 and cyclosporin A which block the neurotoxicity elicited by glutamate acting at N-methyl-D-aspartate (NMDA) receptors by, for example, augmenting phosphorylated levels of NOS. As phosphorylated NOS inhibits its catalytic activity, these drugs effectively reduce NO formation and prevent the neurotoxic effects of NMDA on these cells. Other drugs which possess neurotrophic activity and can be used in the present invention are those small molecules which bind to the same binding proteins as FK506 and/or cyclosporin A and, therefore, mediate similar neuroprotective effects. In one embodiment, these drugs are administered to the subject in addition to the population of cells to treat chronic pain and/or spasticity.

In one embodiment, combinations of one or more of the above-described agents and factors can be used to promote survival of the cells of the invention prior to or after the cells are transplanted into recipient subjects. For example, cells of the present invention can be contacted with one or more of the agents or factors described herein to promote survival of the cells *in vitro* and/or *in vivo.* In another embodiment, the cells of the invention can be transfected with the nucleic acid of one or more of the agents or factors described herein and also contacted with one or more of the agents or factors described herein. Moreover, although many of the neurotrophic factors described herein are specific for a particular cell type, the association of these factors with such a cell type does not exclude the use of that factor with a different cell type. Treatment of the cells of the invention with the agents or factors described herein can occur simultaneously or sequentially.

In another embodiment, the administration of the population of cells to treat chronic pain can be coupled with administration of traditional therapies for these conditions (*e.g*., with opiods or baclofen). In certain subjects, such combination therapies may result in optimal amelioration of symptoms.

In another embodiment, agents which inhibit T cell activity in the subject can be administered in addition to the subject cells. As used herein, an agent which inhibits T cell activity is defined as an agent which results in removal or destruction of T cells within a subject or inhibits T cell functions within the subject, thus the T cells may still be present in the subject but are in a non-functional state, such that they are unable to proliferate or elicit or perform effector functions, such as cytokine production, cytotoxicity etc. The term "T cell" encompasses mature peripheral blood T lymphocytes. The agent which inhibits T cell activity may also inhibit the activity or maturation of immature T cells.

The following examples describe the invention in greater detail. These examples are intended to further illustrate, not to limit, aspects of the invention described herein.

### EXAMPLE 1

### Seeding Cells In Fibrinogen-Thrombin Constructs

Human UTCs were removed from cryogenic storage, removed from cryoprotectant and washed with PBS containing Ca/Mg. Cells were resuspended in a volume of 200-300µl Fibrinogen and thrombin were diluted in 50µm aliquots such that addition of 50µl thrombin and 50µl fibrinogen to cells resulted in a final dilution of 1:133 and 1:8 respectively. Immediately upon addition of the thrombin component, material was dispensed into a low cluster cell culture dish and placed in the incubator with culture media as previously described. Cells and construct were placed in a 37°C incubator with 5% CO2 for 4 days. To assess viability, cells were incubated with Live/Dead stain (Invitrogen, Carlsbad CA) using manufacturers instructions and viewed under a fluorescent microscope.

Human UTCs were plated with thrombin and fibrinogen. After four days, the hUTCs were checked for viability by fluorescent microscopy following application of a viable stain. The constructs were feasible for delivery of hUTCs because the hUTCs remain viable in fibrinogen-thrombin constructs *in vitro* at four days

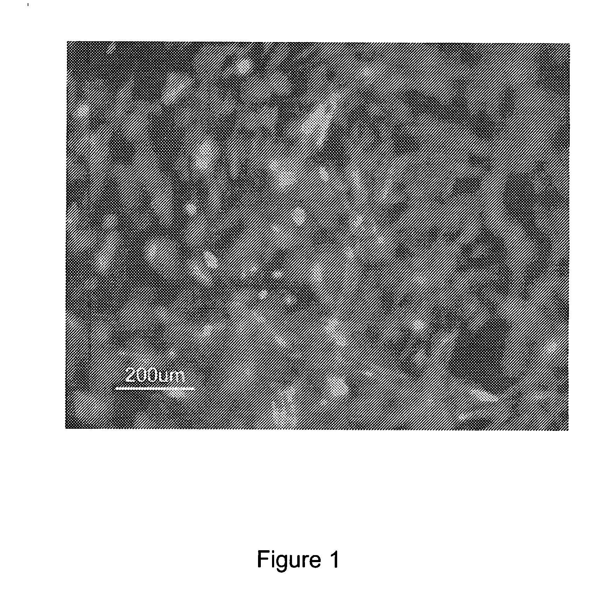

A photograph showing cell viability is illustrated in Figure 1.

### EXAMPLE 2

### Local and Systemic Administration of Cells

To demonstrate that local and systemic administration of hUTCs to animals reduced pain behavior, the inventors subjected animals to choronic constriction injury (CCI). CCI is a common model for testing agents and therapies for neuropathic pain (see Bennett and Xie, Pain, 1988; 33:87 - 107). Sprague-Dawley rats weighing 200-225g were first anesthetized with xylazine and ketamine. The animals' sciatic nerve was isolated. Four loose ligatures using 4-0 chromic catgut suture were placed on the sciatic nerve as it exits the sciatic notch. Baseline behavior (mechanical sensitivity to Semmes Weinstein filaments) was obtained for all animals prior to the surgery. Five or six days following surgery animals were re-tested and groups were stratified to assure that each group demonstrates similar pain behavior and that the distribution of pain severity in each group is similar.

At 5-6 days following surgery, immediately following testing, animals were treated with one of the following:
1. Eight animals were treated with a modified thrombin-fibrinogen construct. The construct (a modified hemostat) was applied to the injured nerve: while under anesthesia 300µl - 400µl of gel material was injected to the injured nerve vicinity. The gel was prepared as follows: 300µl of PBS containing calcium and magnesium was mixed with thrombin 50µl (1:133, final) and 50µl fibrinogen (1:8, final).
2. Eight animals were treated with a local formulation of cells. The formulation was comprised of a dose of 3e⁵ cells in 300µl of PBS containing calcium and magnesium, 50µl thrombin (1:133, final) and 50µl fibrinogen (1:8, final). While under anesthesia 300µl - 400µl of gel material was injected to the injured nerve vicinity.
3. Eight animals were treated with an IV dose of 3e⁶ cells in 2ml of PBS containing calcium and magnesium.
4. Eight animals were treated with an IV dose of 2ml of PBS containing calcium and magnesium.
5. Sixteen control animals that underwent CCI remained with no treatment.

### Local Administration

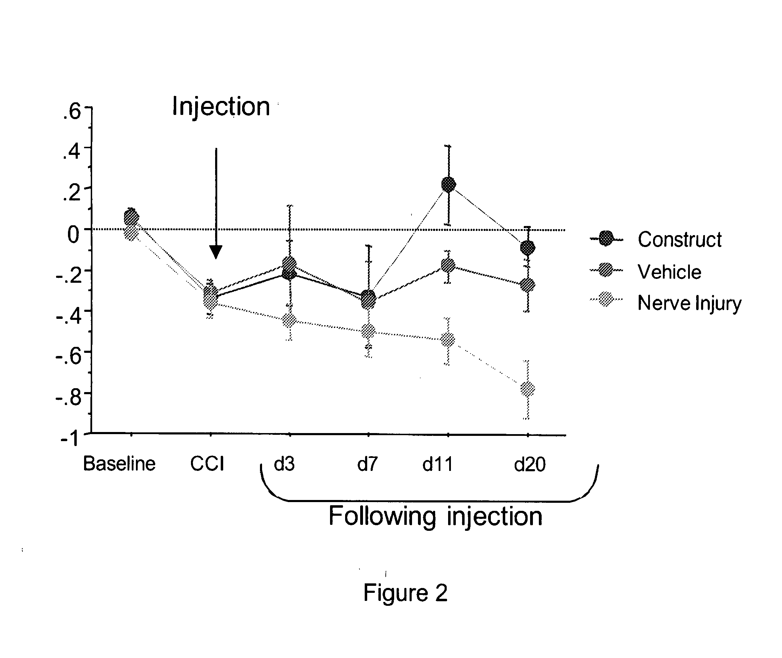

The results of the local administration are illustrated in Figure 2.

The pre-treatment baseline scores were not significantly different from the predicted normal values (difference scores of 'zero'). Moreover, there were no significant between-group differences for the baseline scores.

Five days following the surgery all three groups developed significant mechanical allodynia (CCI group: -0.36±0.08 log10gm, vehicle group: -0.31±0.07 log10gm, construct group: -0.34±0.08 log10gm) compared to the baseline levels (CCI group: -0.02±0.01 log10gm, vehicle group: -0.05±0.02 log10gm, construct group: -0.06±0.04 log10gm). Eleven and 20 days following the administration, the CCI group (no treatment) remained significantly hypersensitive compared to the baseline levels (-0.54±0.12 log10gm and -0.78±0.14 log10gm respectively). The vehicle group remained significantly hypersensitive compared to the baseline on the 11^{th} and 20^{th} days following administration, however significantly less sensitive than the CCI group (-0.18±0.01 log10gm and -0.27±0.12 log10gm respectively). The construct group developed significant hyposensitivity on day 11 following the administration (0.21±0.19 log10gm) and significantly reduced hypersensitivity on day 20 (-0.08±0.03 log10gm).

### Systemic (I.V.) Administration

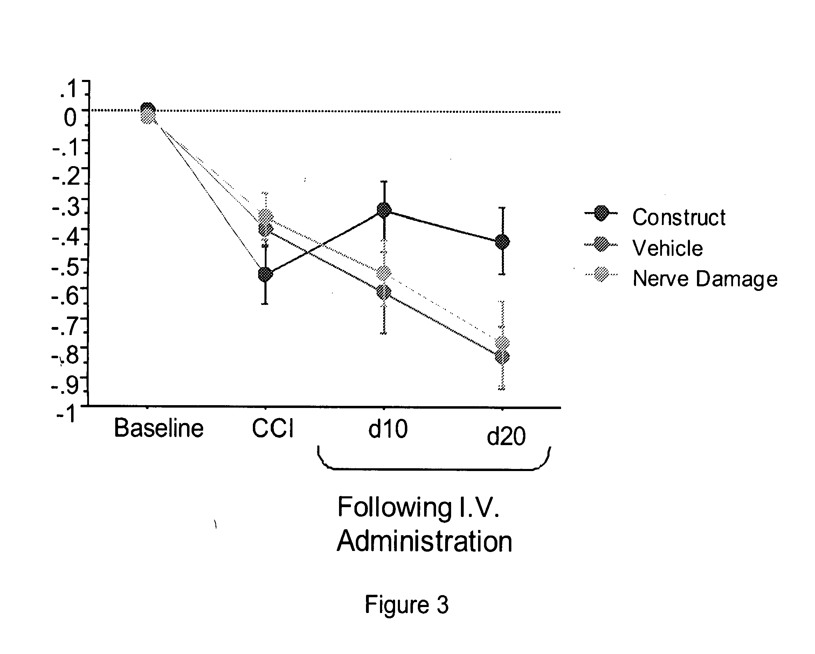

The results of systemic (i.v.) administration are illustrated in Figure 3.

The pre-treatment baseline scores were not significantly different from the predicted normal values (difference scores of 'zero'). Moreover, there were no significant between-group differences for the baseline scores.

Five days following the surgery all three groups developed significant mechanical allodynia (CCI group: -0.35±0.08 log10gm, vehicle group: -0.40±0.05 log10gm, construct group: -0.55±0.09 log10gm) compared to the baseline levels (CCI group: -0.02±0.01 log10gm, vehicle group: -0.01±0.01 log10gm, construct group: -0.00±0.01 log10gm). Ten and 20 days following the administration, the CCI (no treatment) and the vehicle groups remained significantly hypersensitive compared to the baseline levels (CCI: -0.53±0.11 log10gm and - 0.76±0.10 log10gm respectively, vehicle: -0.61±0.14 log10gm and -0.83±0.11 log10gm respectively). The hypersensitivity of the construct group was significantly reduced on day 10 and day 20 compared to the two other groups (-0.33±0.10 log10gm and -0.41±0.11 log10gm).

### EXAMPLE 3

### Efficacy of the Biological Construct in Alleviating Neuropathic Pain

Pain behavior was examined following local injection and following administration to the tail vein.

### Material and Methods

All rats were subjected to CCI as previously described. Briefly, under xylazine/ketamine anesthesia, the animals' sciatic nerve was isolated. Four loose ligatures using 4-0 chromic catgut suture were placed on the sciatic nerve as it exits the sciatic notch. Baseline behavior (mechanical sensitivity to Semmes Weinstein filaments) was obtained for all animals prior to the surgery. Six days following surgery the animals were re-tested and groups were stratified to assure that each group demonstrates similar pain behavior and that the distribution of pain severity in each group is similar.

At 6 days following surgery (as the neuropathic pain was verified by the presence of tactile allodynia) animals were treated with one of the following treatments:
1. Systemic administration of about 2 ml of fluid containing cells or carrier to the tail vein (Table 3-1).
2. Local administration of cells or carrier (Table 3-2)
3. Control animals that were treated with Gabapentin priorto testing.

Animals were then tested for mechanical sensitivity (tactile allodynia) at day 7, 10, 14, 17 and 21 following the treatment. The examiners were blind to the treatment; the code was opened only in the end of the study and following submission of the results to the doctor in charge of the study.

The study was performed in three separate sessions.

**Table 3-1: Systemic Administration**

| Group | Treatment | Regime | Pain Assessment |
|---|---|---|---|
| A | 1e6 hUTC Cells | Once on Study Day 6 | Prior to Injury. 5-6 days post injury. 7, 10, 14, 17, 21 days post treatment |
| B | 1e7 hUTC Cells | | |
| C | pbs | | |
| D | 3e6 hUTC Cells | | |
| GP | Gabapentin | Prior tests | |

**Table 3-2: Local Administration**

| Group | Treatment | Regime | Pain Assessment |
|---|---|---|---|
| 1 | Cells 1.00E+06 | Once on Study Day 6 | Prior to Injury. 5-6 days post injury. 7, 10, 14, 17,21 days post treatment |
| 2 | Collagen (Colbar) | | |
| 3 | 1.00E+06 | | |
| 4 | 3.00E+05 | | |
| 5 | 1.00+05 | | |
| | 0.00+00 | | |
| 6 | Evicel (Omrix) 1.00E+06 | | |
| 7 | 3.00E+05 | | |
| 8 | 1.00+05 | | |
| 9 | 0.00+00 | | |
| GP | Gabapentin | Prior tests | |

Following euthanasia the relevant nerves were harvested and stored in formalin. The construct administration and the behavior test were performed by a person that was blind to the treatment group.

*Statistical analysis:* Each animal was evaluated against its own baseline (for evaluating baseline pain behavior) and against its own pain behavior and contrasted with its own pre-surgical mechanical sensitivity to assess recovery. Data is presented as percentage of change from the levels in the neuropathic pain stage (5-6 days following the surgery). For treatment repeated measures ANOVA was performed followed by Fisher's PLSD test. Separate data for affected and contralateral paws is provided.

### Results:

### Local Administration

The pre-treatment baseline scores were not significantly different from the predicted normal values (difference scores of'zero' between two paws). Moreover, there were no significant between-group differences for the baseline scores. Five days following the surgery all three groups developed significant mechanical allodynia compared to the baseline levels (see for row data).

### Affected Side:

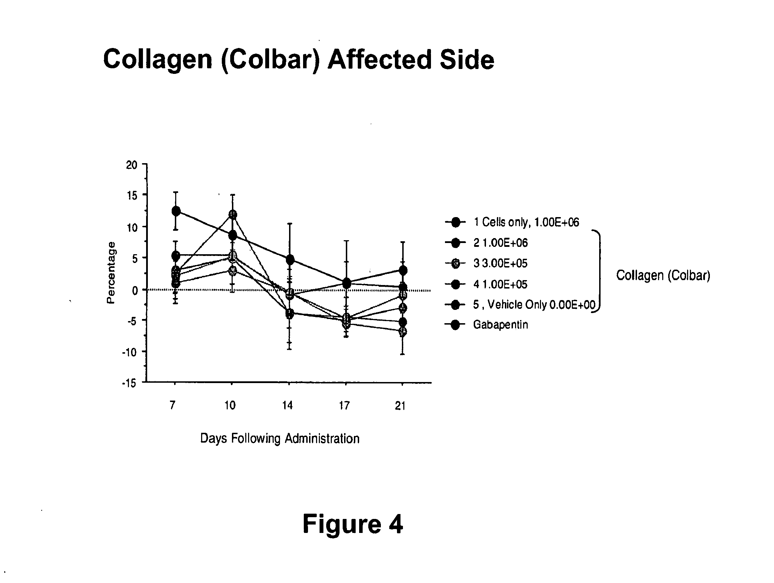

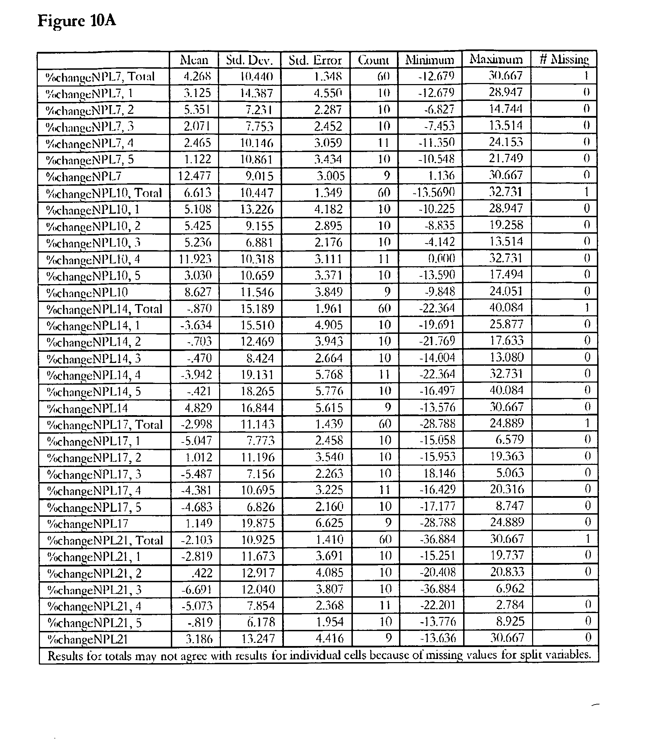

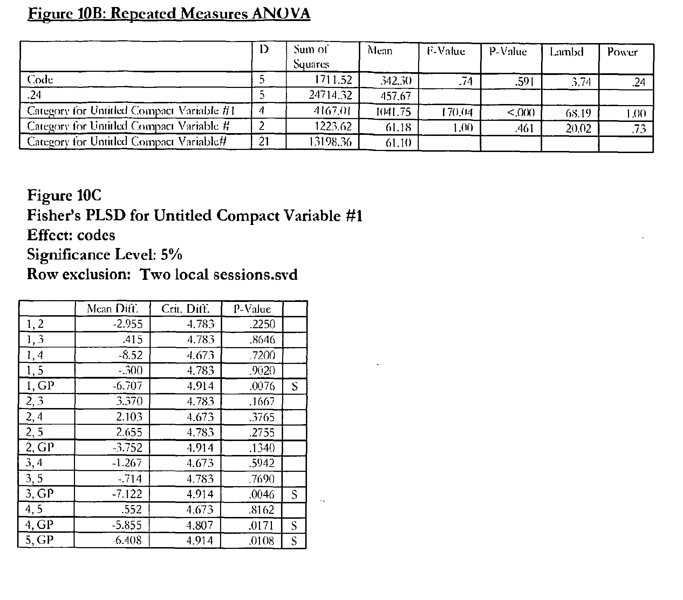

As shown in Figure 4 for collagen (Colbar) expected, gabapentin reduced the pain. The vehicle and the cells alone had no effect on pain behavior. The low dose (group 4) had a short effect, up to 10 days following the administration. The high dose (group 2) had the most significant effect that for overall analysis (Fisher's PLSD test) was not significantly different from the successful treatment with gabapentin. However it is important to note that this treatment was not significantly different from the other groups effect. (Statistical analysis is provided in Figure 10).

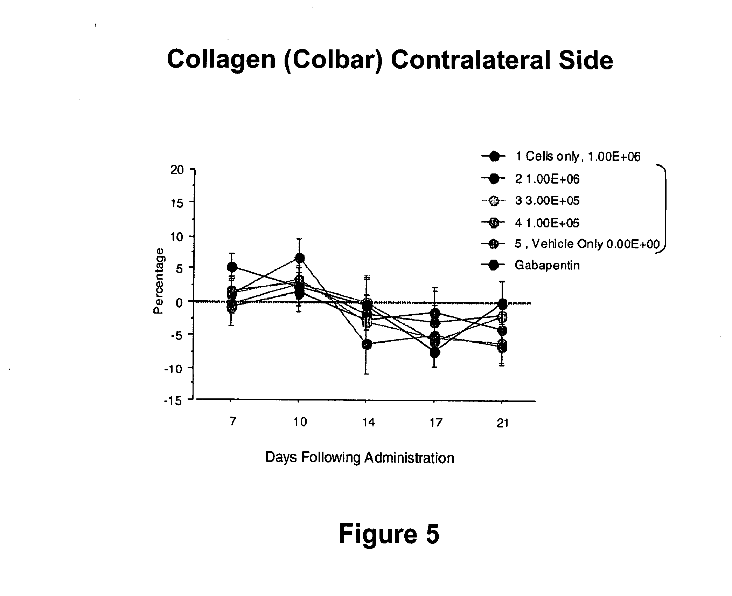

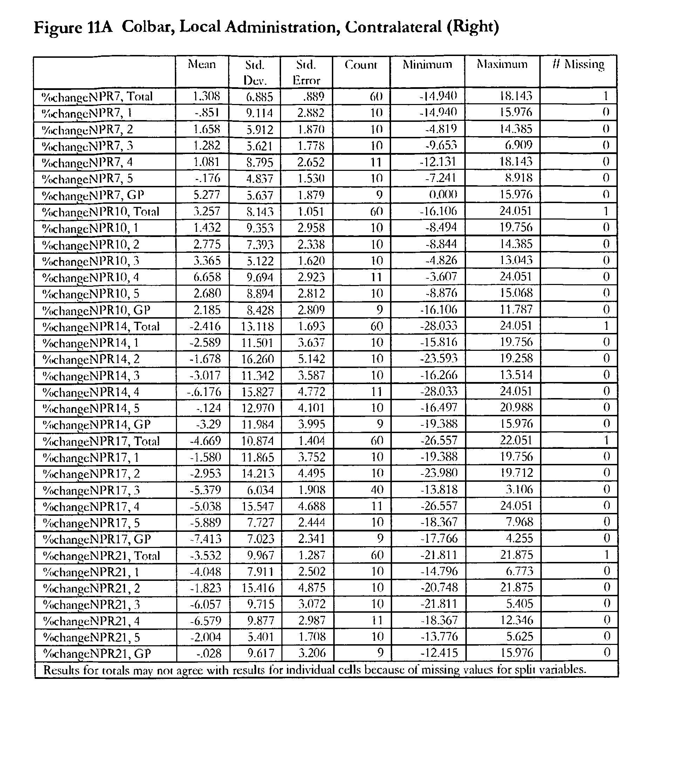

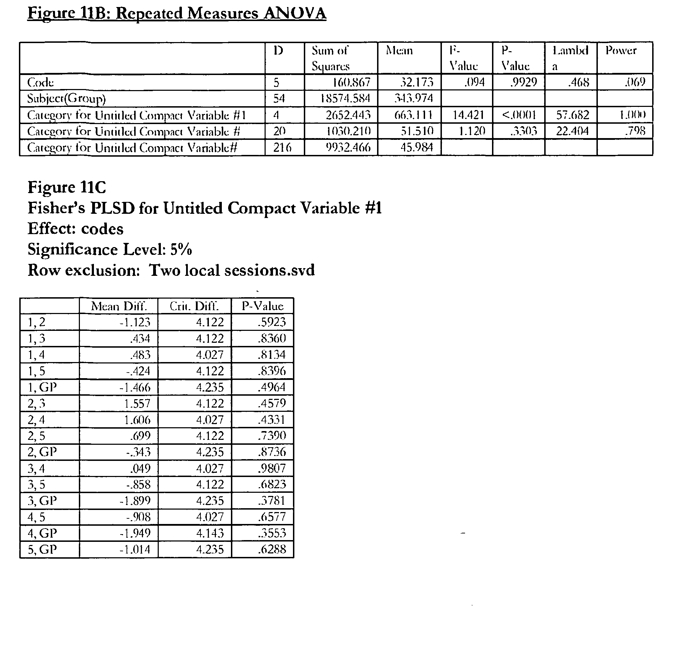

The contralateral paws did not demonstrate significant tactile allodynia, none of the treatment groups had a significant effect on this side tests. (Results in Figure 5 and statistical analysis are provided in Figure 11).

### Evicel (Omrix)

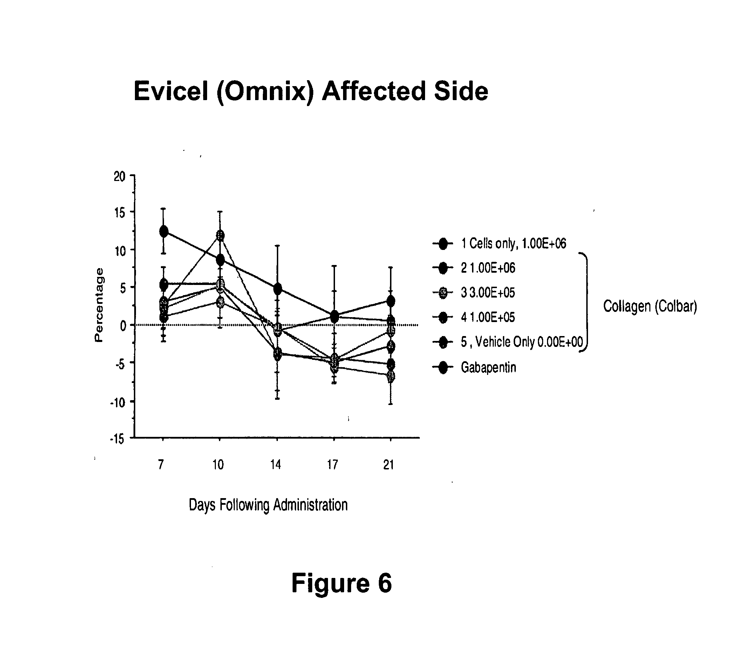

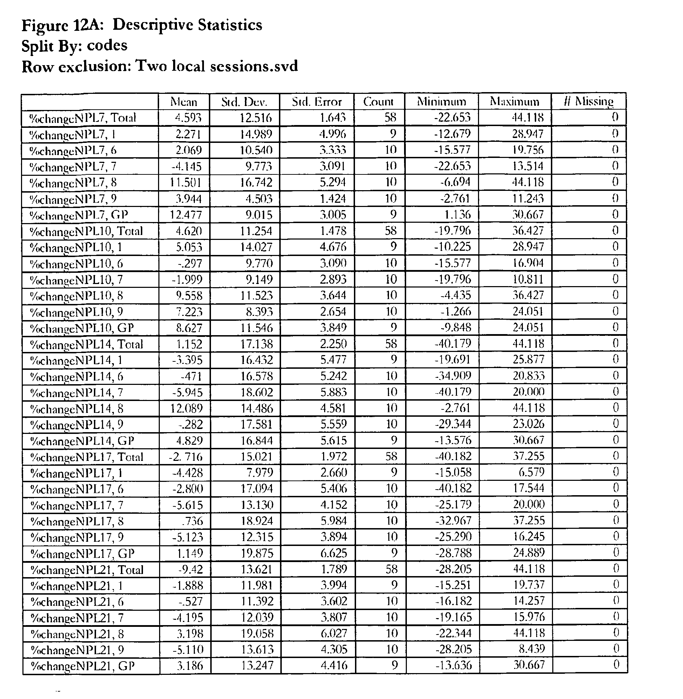

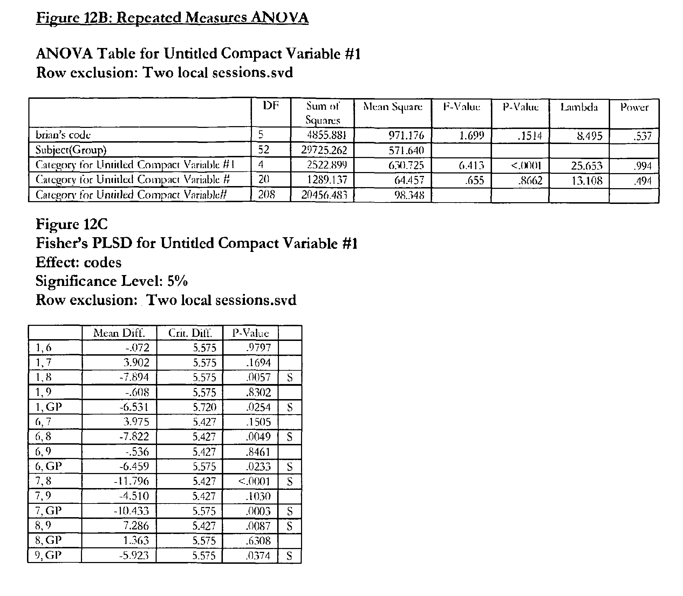

As shown in Figure 6 for Evicel (Omrix), the gabapentin treatment reduced pain. The low dose (group 8) was as effective as gabapentin, and even slightly superior on day 14. The vehicle (group 9) was more effective than the two higher doses (groups 6 and 7), that did not have any palliative effect at all. (Results shown in Figure 6 and statistical analysis is provided in Figure 12).

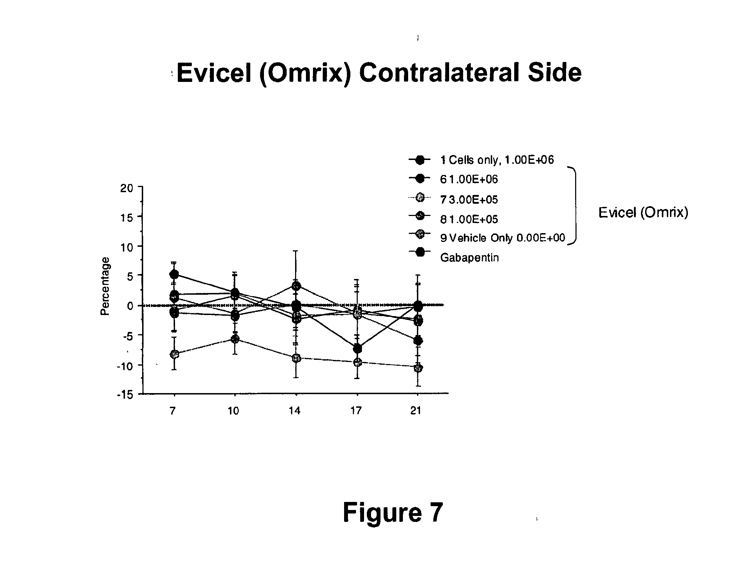

The contralateral paws did not demonstrate tactile allodynia, none of the treatment groups had a significant effect on this side tests. (Statistical analysis provided in Figure 7).

### Systemic Administration

Affected Side: Systemic Administration Affected Side (Left), Change from Neuropathic Pain

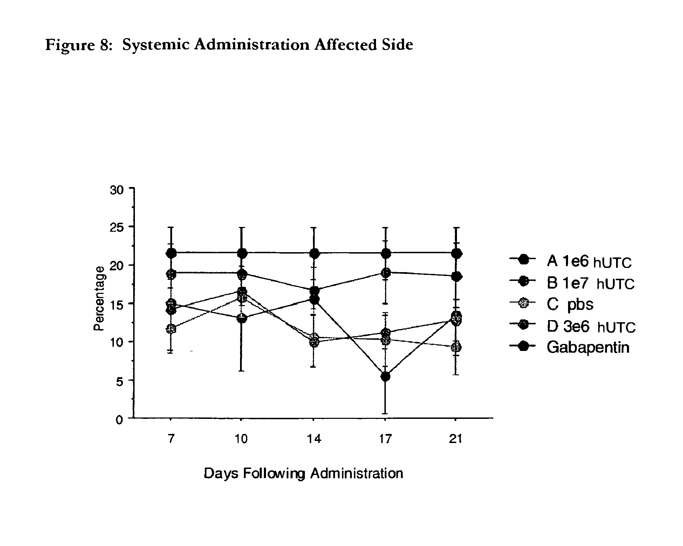

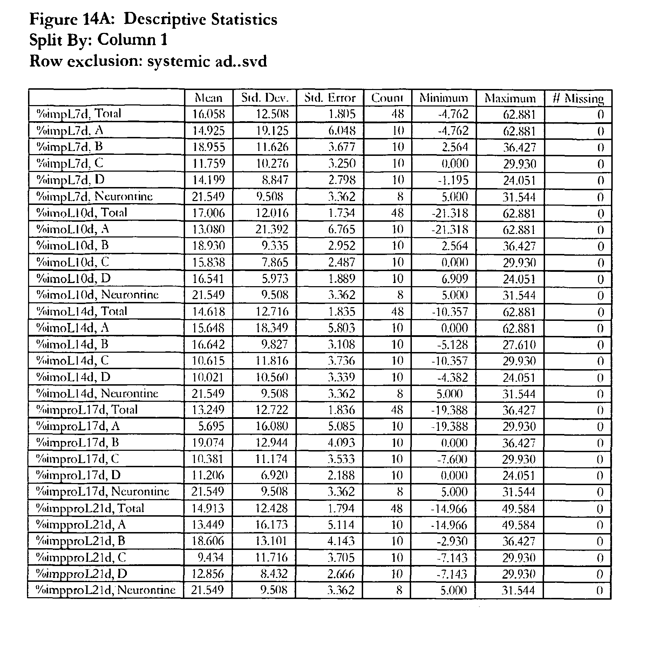

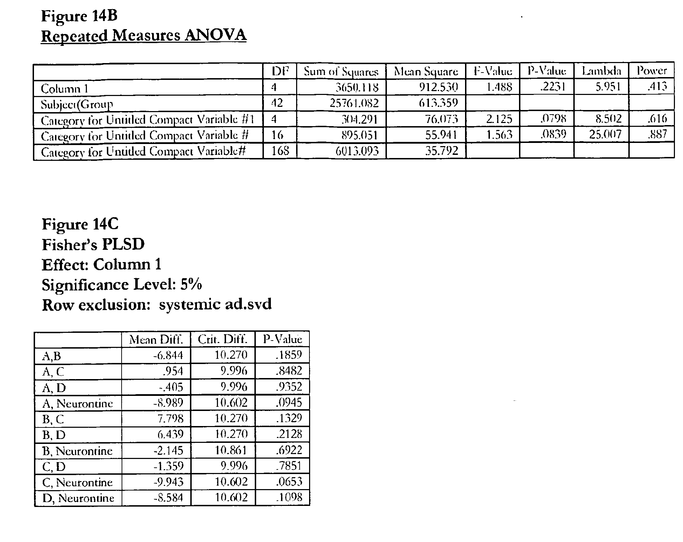

As shown Figure 8 gabapentin reduced the pain. The 1e7 (group B) effect was close to the gabapentin effect. The other two groups (A and D) effect were not different than the pbs effect. (Statistical analysis is provided in Figure 14)

Contralateral Side: Systemic Administration Contralateral Side (Right), Change From Neuropathic Pain

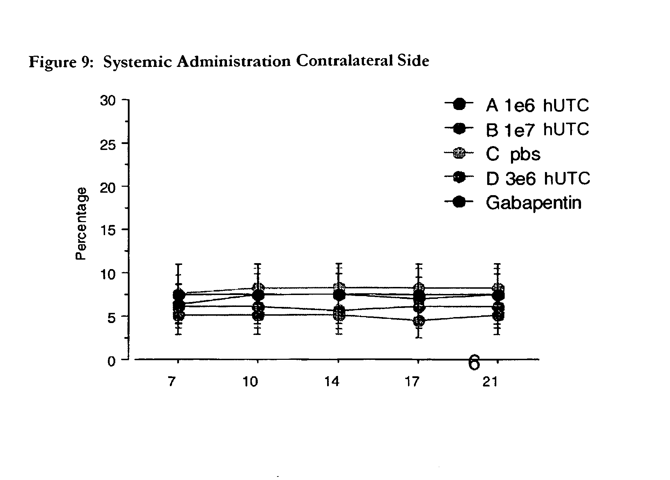

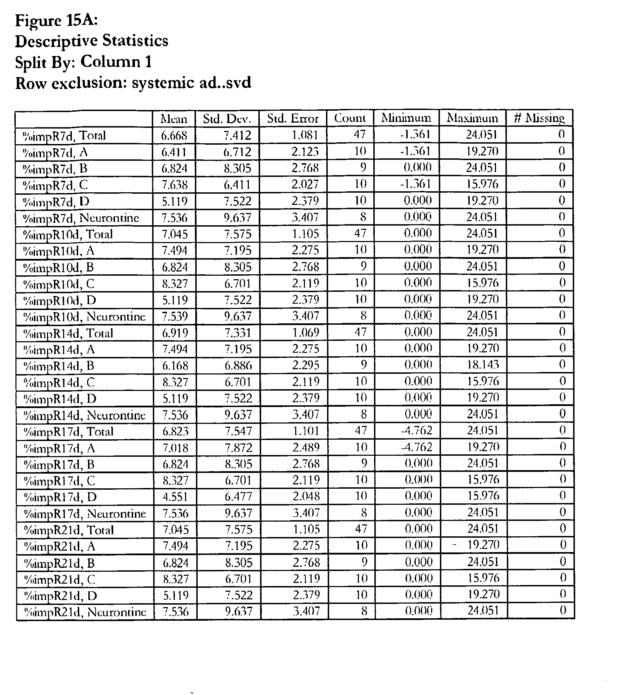

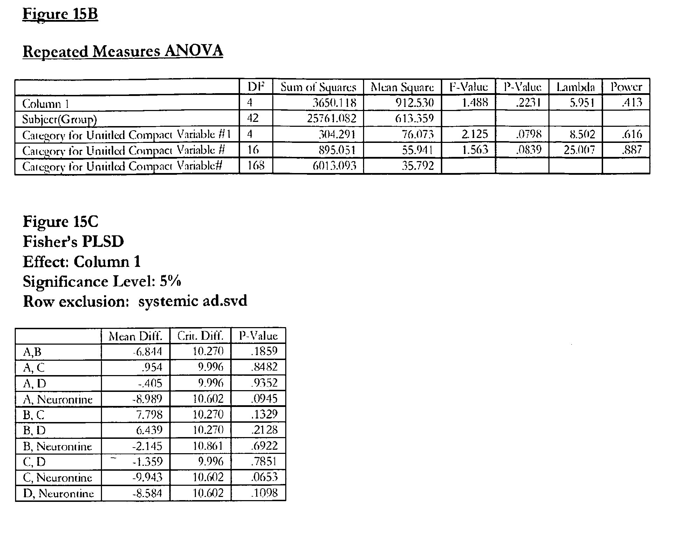

The contralateral paws did not demonstrate tactile allodynia, none of the treatment groups had a significant effect on the mechanical sensitivity. (Results shown in Figure 9 and statistical analysis provided in Figure 15).

### Conclusions

Pain was significantly reduced by systemic administration of 1e7 hUTC cells and by local administration of high dose (1.00E+065) cells in Colbar vehicle. However the most significant effect was induced by local administration of low dose (1.00E+05) of cells with Evicel vehicle. This effect was not inferior to the effect induced by the common neuropathic pain medications Gabapentin.

### EXAMPLE 4

### The Evaluation Of Treatment And Analgesic Effect Of Cells Injected Systemically In The Chung Model Of Pain In Rats

This study was to examine the antinociceptive effect of uHTC cells in the Chung model of neuropathic pain. The cell treatments were given by systemic administration on study day 6 following the surgery and their effect on pain response was measured. This study did not follow any specific regulatory guidelines. The results demonstrate that there was a significant pain relief effect in all the given doses of hUTC cells. Gabapentin, which was administered at a dose of 150 mg/kg and served as the positive control, was significantly active in reducing pain as an analgesic compound on all testing days compared to the Vehicle.

At the beginning of the study, the total mean body weight of all the animals in the study was 192.62 ± 1.42 g. All the animals gained weight throughout the study and no significant differences in weight gain were found.

Von Frey was used to assess the animals' response to pain. The results were calculated using the following three different methods.

Von Frey results calculated by log division. Response to pain was assessed using the Von Frey apparatus (Touch Test^{®}). The grams of force needed to withdrawal the leg were converted to log force according to the values given by Touch Test^{®}. The log force needed to withdraw the healthy leg (right) was divided by the log force needed to withdraw the operated leg (left). The comparison of each treatment group to the Vehicle control group at each measurement time point showed a significant pain relief in all treated groups versus the Vehicle group at several time points (p<0.01 and p<0.05). Treatment with the positive control item, Gabapentin (Group 5M), was active as a pain relief compound during all testing days compared to the Vehicle treated group (Group 4M; p<0.01.

Von Frey results calculated by simple subtractions. Another way of calculating the Von Frey values was by simple subtraction of the force needed to withdraw the right healthy leg minus the force needed to withdraw the left painful leg. When comparing the Von Frey values of each treatment group to the Vehicle control group at each measurement time point, results showed a significant pain relief in all treated groups versus the Vehicle group at several time points. The positive control, Gabapentin (Group 5M), had significant pain relief points during all testing days compared to the Vehicle treated group (Group 4M; p<0.01).

Von Frey results calculated by simple subtractions versus pretreatment. Comparison of Von Frey values of each treatment group at each measurement time point to the pretreatment Von Frey values measured on study day 5 showed a significant pain relief in all treated groups versus the Vehicle group at several time points. The positive control, Gabapentin (Group 5M), had a significant pain relief effect on all tested days compared to the same group on study day 5 before treatment (p<0.01).

The objective of the present study was to evaluate the therapeutic activity of hUTC cells given by systemic administration via a tail vein on day 6 after surgery in the Chung neuropathic pain model in rats.

### Experimental Test Items:

| Materials | Name | Cat. No. | Lot No. | MDB Int. # | Supplier | Storage Conditions | Expiry Date |
|---|---|---|---|---|---|---|---|
| Test Items | hUTC cells | N/A | Q112108 | N/A | Sponsor | -80°C | N/A |
| | Ethanol | N/A | 0307AL20.4 | N/A | Floris | Room temperature | Mar. 2011 |
| Vehicle | PBS | 020201 A | 743185 | 0906-0110 | Biological Industries | 2-8°C | Oct. 2009 |
| Positive Control | Gabapentin | 1287303 | GOE005 | USP | Sponsor | Room temperature | N/A |
| Anesthesia Items | Ketamine | N/A | 440785 | 0906-0122 | Supply | Room temperature | Oct. 2011 |
| | Xylazine | N/A | B9367 | 0607-118 | Veterinarian Supply | Room temperature | Jul. 2009 |
| | CO2 | N/A | N/A | N/A | N/A | N/A | N/A |

### Preparation of hUTC for Systemic Injection:

All steps were performed with open vials of hUTC using aseptic technique in a certified BSL II biosafety cabinet.

### Thawing the hUTC (Cells were stored at -80°C):

The cryovial of hUTC was thawed by gently swirling the vial in a 37°C water bath for approximately 1-2 minutes until just thawed. The vial was sprayed down with ethanol, dried off with a Kimwipe and placed in the biosafety cabinet. The cryovial cap was removed and the contents were gently mixed by pipetting up and down two times using a 1 ml pipette. 1 ml of thawed hUTC suspension was then transferred using the pipette to a 15 ml tube. A fresh 1 ml pipette was used to add 1 ml of sterile PBS to the cryovial. In order to suspend residual thawed hUTC cells, the PBS was pipetted up and down two times. One ml of the residual hUTC suspension was pipetted into the 15 ml tube containing the thawed hUTC suspension.

Using a fresh 10 ml pipette, 11 ml of PBS was added to the hUTC suspension to reach a total of 13 ml. For each vial of cells, 1 ml wash + 12 ml wash was used.

The hUTC suspension was centrifuged at 250 g (or 1200 rpm) for 5 minutes at room temperature. The tube was removed from the centrifuge and while taking care not to disturb the pelleted hUTC, 12.7 ml of supernatant was pipetted up and discarded using one 10 ml pipette.

One ml of sterile PBS was added per vial of defrosted hUTC pellet to equal a total volume of approximately 1 ml/vial using a sterile 5 ml pipette. The pellet was re-suspended by gently pipetting up and down ensuring not to create bubbles.

Fifty µl of trypan blue solution was added to the hUTC microfuge count tube. Using a P200 Pipetman, cells were diluted 1: 10 (50 µl in 500 µl) in PBS. Fifty µl of diluted hUTC suspension was added to the trypan blue solution in the microfuge tube using a P200 Pipetman. The contents of the tube were gently, but thoroughly, mixed by pipette and 10 µl of trypan blue stained hUTC were loaded into a hemacytometer. The hUTC were counted using the outermost four large hemacytometer girds. The number and the sum of both colorless (viable) and blue-stained (non-viable) hUTC were recorded. Viable hUTC concentration, total viable hUTC content of the cell suspension, and % viable hUTC, were calculated as follows:
1. Total colorless hUTC. ÷ 4 * 20 * 10,000 = Viable hUTC concentrate in hUTC /ml.
2. Final volume * Viable hUTC concentrate in hUTC /ml = Total Viable hUTC.
3. Total colorless hUTC ÷ Total colorless and blue-stained hUTC * 100 = % Viable hUTC.

The volume to re-suspend hUTC so as to achieve the appropriate concentration of hUTC /µl was calculated.

For 2 ml/animal:
- For 1 * 106 : 5*105 / ml
- For 3*106: 1.5*106 / ml
- For 10*106 5*106 / ml

The cells were pulled up by syringe in advance, stored horizontally on ice and mixed by gentle rolling immediately before injection.

### TEST SYSTEM

| | |
|---|---|
| **Species/Strain:** | Rat Sprague Dawley. |
| **Source:** | Harlan Laboratories Israel, Ltd. (ISO 9001 :2000 Certificate No.:US2002/3081). |
| **Gender:** | Male |
| **Total no. of Animals:** | n= 60 |

| | |
|---|---|
| **Age:** | Young adults; weighing 160-189 g at study initiation. |
| **Body Weight:** | Weight variation of animals at the time of treatment initiation did not exceed ± 20% of the mean weight. |
| **Animal Health:** | The health status of the animals used in this study was examined upon their arrival. Only animals in good health were acclimatized to laboratory conditions and were used in the study. |
| **Acclimation:** | 5 days. |
| **Housing:** | During acclimation and throughout the entire study duration, animals were housed within a limited access rodent facility and kept in groups with a maximum of 5 rats per polypropylene cages. The cages were fitted with solid bottoms and filled with sterile wood shavings as bedding material. |
| **Food and Water:** | Animals were provided *ad libitum* with a commercial, sterile rodent diet and had free access to drinking water that was supplied to each cage via polyethylene bottles with stainless steel sipper tubes. A feed lot analysis of the diet batch used in the study was included in the archives with the study data. Water was monitored periodically. |
| **Environment:** | Automatically controlled environmental conditions were set to maintain temperature at 20-24°C with a relative humidity (RH) of 30-70%, a 12: 12 hour light dark cycle and 15-30 air changes/h in the study room. Temperature and RH were monitored daily. The control computer monitored the light cycle. |
| **Identification:** | Animals were given a unique animal identification ear mark. This number also appeared on a cage card, visible on the front of each cage. The cage card also contained the study number all other relevant details as to treatment group. |
| **Randomization:** | Animals were randomly assigned to experimental groups. |
| **Termination:** | At the end of the study, surviving animals were euthanized by CO2 asphyxiation. |
| **Justification:** | The rat was selected as it represented the species of choice for this experimental animal model. |

### CONSTITUTION OF TEST GROUPS AND DOSE LEVELS:

The following table lists the 5 experimental groups comprising the study.

**Table 4-1**

| Group # | Group Size | Test Item | Route | Dose mg/kg) | Volume (ml/animal) | Regime |
|---|---|---|---|---|---|---|
| 1M | N=12 | Cells (low concentration) | systemic | 1*10⁶ | 2 ml | Once, on study day 6 |
| 2M | N=12 | Cells (medium concentration) | systemic | 3*10⁶ | 2ml | Once, on study day 6 |
| 3M | N=12 | Cells (high concentration) | systemic | 10*10⁶ | 2ml | Once, on study day 6 |
| 4M | N=12 | Vehicle control (PBS) | systemic | 0 | 2ml | Once, on study day 6 |
| 5M | N=12 | Positive control (Gabapentin) | IP | 150 | 5 | 120 minutes before each behavioral assessment starting on study day 7 |

### TEST PROCEDURES:

**Study Schedule:**

| Study Day | Task |
|---|---|
| -1 | Body weight measurements (baseline); Pain assessment (baseline) |
| 0 | Chung operation |
| 5 | Body weight measurements; Pain assessment; Grouping |
| 6 | Systemic treatment with cells |
| 7,10,14,17,21,26 | Body weight measurements; Pain assessment |
| 30 | Body weight measurements; Pain assessment; Study termination |

**Neuropathic Pain Induction:** Under anesthesia, the rat was placed in a prone position and the left paraspinal muscles were separated from the spinous process at the L4-S2 levels. The L6 transverse process was carefully removed with a small rongeur to visually identify the L4- L6 spinal nerves. The left L5 and L6 spinal nerves were isolated and tightly ligated with 3-0 silk thread, and then cut with a blade. Following the surgery, the rats were returned to the cages and remained under a heating lamp until they awoke.

In order to form homogenous treatment groups and to adhere to randomization, all the operated rats were grouped according to inclusion/exclusion criteria. Only 60 operated animals were selected on study day 5 after the Chung procedure according to inclusion / exclusion criteria.

### Inclusion criteria:

- Licking of the operated paw, accompanied by gentle biting or pulling on the nails with the mouth;
- Holding the leg in the air;
- Bearing weight on the contralateral side of the nerve injury;
- Deformities of the hind paw and abnormal posture and walking;
- Weakness of the left hind paw.

### Exclusion criteria:

Animals that could not move their paws demonstrated signs the L4 was disrupted. These animals were excluded from the study.

### Treatment:

On study day 6 after the Chung surgery, the animals (Groups 1M, 2M, 3M and 4M) were given their respective treatments via systemic injection to the tail vein. Animals in Group 5M received treatment with gabapentin, at a dose of 150 mg/kg via IP, 120 minutes before each behavioral assessment starting on study day 7. In all instances, all dosing was applied as a single administration. At the end of the study, the animals were euthanized with exposure to CO2.

### OBSERVATIONS AND EXAMINATIONS:

### Von Frey Examinations:

Response to pain was assessed using a Von Frey apparatus (Touch Test®) for mechanical allodynia. The rat was placed in an enclosure positioned with a metal mesh surface, but allowed to move freely. The rats' cabins were covered with red cellophane to diminish environmental distributions. The tests began after cessation of exploratory behavior. The set of monofilaments provided an approximately logarithmic scale of actual force and a linear scale of perceived intensity. The logarithmic scale used for calculations is below:

| | | | | | | | | | | | | | | | | | | | |
|---|---|---|---|---|---|---|---|---|---|---|---|---|---|---|---|---|---|---|---|
| | 1.65 | 2.36 | 2.44 | 2.83 | 3.22 | 3.61 | 3.84 | 4.08 | 4.17 | 4.31 | 4.56 | 4.74 | 4.93 | 5.07 | 5.18 | 5.46 | 5.88 | 6.10 | 6.45 |
| ) | 0.008 | 0.02 | 0.04 | 0.07 | 0.16 | 0.40 | 0.60 | 1.00 | 1.40 | 2.00 | 4.00 | 6.00 | 8.00 | 10 | 15 | 26 | 60 | 100 | 180 |

The operating principle: When the tip of a fiber of given length and diameter is pressed against the skin at right angles, the force of application increases as long as the researcher continues to advance the probe until the fiber bends. After the fiber bends, the probe can continue to advance which causes the fiber to bend more, but without applying additional force. This principle makes it possible for the researcher to use a hand held probe to apply a reproducible force within a wide tolerance to the paw.

Rodents exhibit a paw withdrawal reflex when the paw is unexpectedly touched. The Touch Test™ Sensory Evaluator can be used on the plantar surfaces of the rat's foot and the animal will indicate sensation by pulling back its paw. The minimal force needed to elevate the withdrawal reflex is considered as the value of reference.

Method of calculation: The raw data of Von Frey force in grams was converted to the log force according to the above table. In addition, the maximum (60 g) was applied to diminish the arbitrary high gap between the two legs. The log force necessary to withdraw the healthy leg (right) was divided by the log force needed to withdraw the operated leg (left).

The calculation presents the values of legs in the healthy state near 1 when the ratio between the force of each leg is similar. The increase in values presents a more painful state. Results of the baseline values indicated a value of 1.

Body Weights: Determination of the individual body weights of the animals was made on each testing day.

Statistic Analysis: All parameters are represented as means and standard error of the mean (SEM) and analyzed using a T -test, paired and unpaired, two tailed (Microsoft Excel). Probability (p) values smaller than 0.05 or smaller than 0.01 were considered significant.

Humane Endpoints: At the end of the study, the animals were euthanized with exposure to CO2•

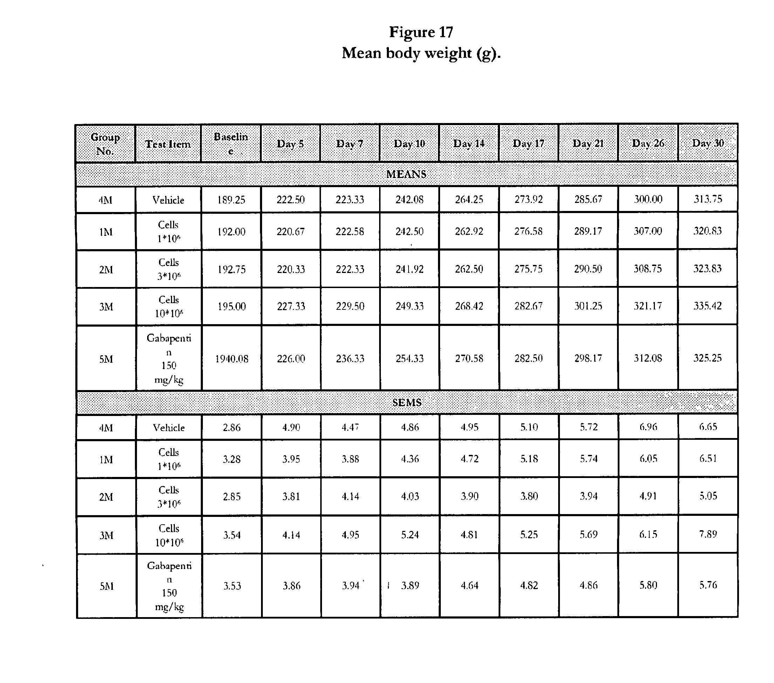

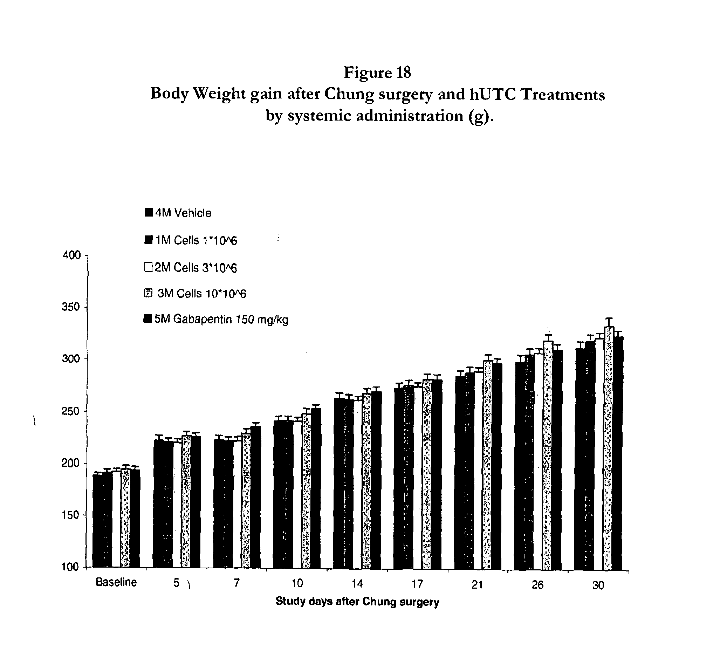

Results: At the beginning of the study, the total mean body weight for all the animal was 192.62± 1.42 g. Although all the animals gained weight during the study period, there were no significant differences observed between the groups. (Figures 17 and 18.)

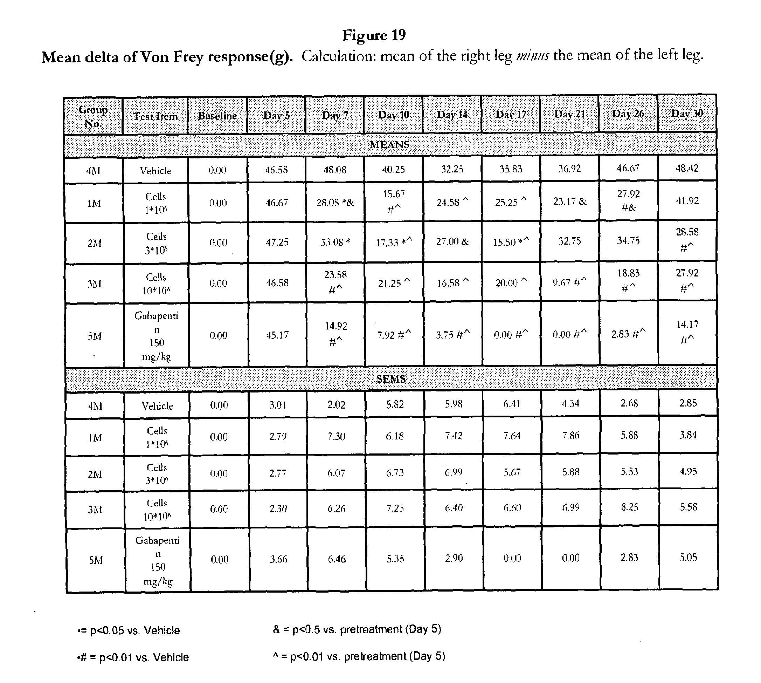

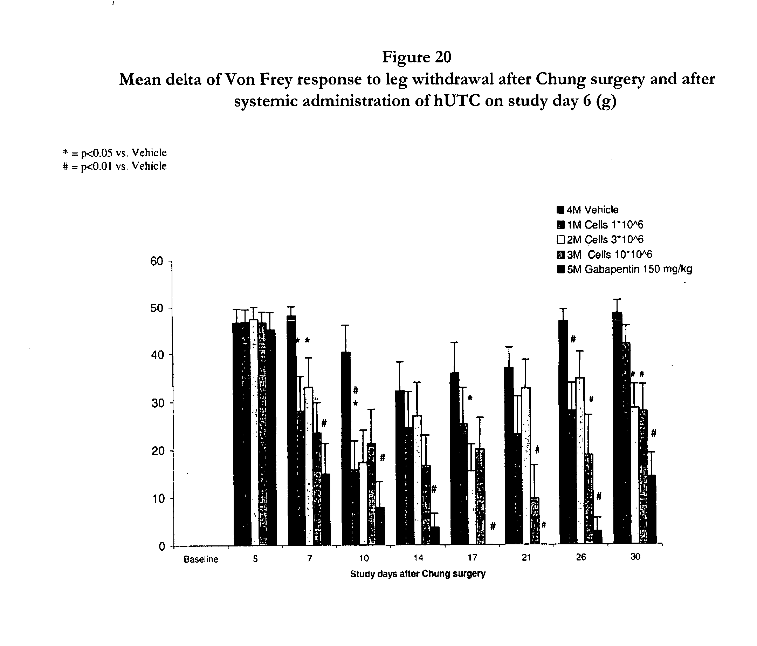

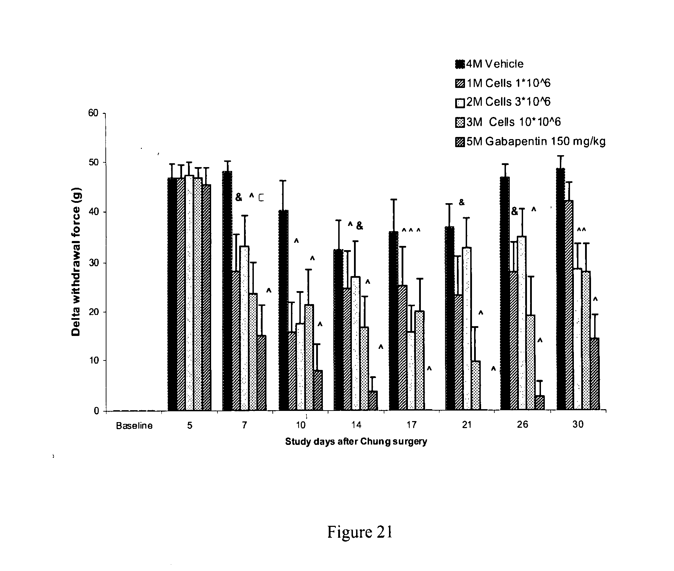

Von Frey Examination: Response to pain was assessed using Von Frey apparatus (Touch Test®) for mechanical allodynia. The withdrawal force in grams was converted to log force according to the values given by Touch Test® (Reference Section 7.1). The log force needed to withdraw the healthy leg (right) was divided by the log force needed to withdraw the operated leg (left). The calculation presents the values of legs in the healthy state near 1 when the ratio between the force of each leg is similar. The increase in values presents a more painful state. (Figures 19, 20 and 21.)

Von Frey results calculated by log division: When comparing each treatment group to the Vehicle control group at each time point, the treatment groups showed significant pain relief as follows:

Animals treated with cells at a low concentration (Group 1M) showed significant pain relief on study days 7, 10, 26 and 30 compared to the Vehicle treated group (Group 4M): 1.1 0±0.03 vs. 1.17±0.02 in Group 4M on study day 7 (p<0.05).

Animals treated with cells at a medium concentration (Group 2M) showed significant pain relief on study days 7, 10 and 26 with p<0.05 and on study days 17 and 30 with p<0.01 compared to the Vehicle treated group (Group 4M): 1.1 0±0.02 vs. 1.17±0.02 in Group 4M on study day 7 (p<0.05).

Animals treated with cells at a high concentration (Group 3M) showed significant pain relief on study days 7, 21, 26 and 30 compared to the Vehicle treated group (Group 4M): 1.07±0.02 vs. 1.17±0.02 in Group 4M on study day 7 (p<0.01)

Treatment with the positive control item, gabapentin (Group 5M), was active as pain relief compound during all testing days compared to the Vehicle treated group: 1.04±0.02 vs. 1.17±0.02 in Group 4M on study day 7 (p<0.01).

Von Frey results calculated by simple subtractions: Von Frey results were also calculated using simple subtraction: The force needed to withdraw the right healthy leg minus the force needed to withdraw the left painful leg.

Comparison of Von Frey values for each treatment group to the Vehicle control group at each time point showed significant pain relief as follows.

Animals treated with cells at a low concentration (Group 1M) showed significant pain relief on study day 7 (p<0.05) and on study days 10 and 26 (p<0.01) compared to the Vehicle treated group (Group 4M): 15.67±6.18 g vs. 40.25±5.82 g in Group 4M on study day 10 (p<0.01). Animals treated with cells at a medium concentration (Group 2M) showed significant pain relief on study days 7, 10 and 17 (p<0.05) and on study day 30 (p<0.01) compared to the Vehicle treated group (Group 4M): 17.33±6.73 g vs. 40.25±5.82 g in Group 4M on study day 10 (p<0.05). Animals treated with cells at a high concentration (Group 3M) showed significant pain relief on study days 7, 21, 26 and 30 (p<0.01) compared to the Vehicle treated group (Group 4M): 18.83±8.25 g vs. 46.67±2.68 g in Group 4M on study day 26 (p<0.01). Treatment with the positive control item, gabapentin (Group 5M), was active as a pain relief compound during all testing days compared to the Vehicle treated group (Group 4M): 7.92±5.35 g vs. 40.25±5.82 g in Group 4M on study day 10 (p<0.01).

Von Frey results calculated by simple subtractions versus pretreatment: Comparison of Von Frey values for each treatment group at each time point to the pretreatment Von Frey values measured on study day 5 showed significant pain relief as follows. Animals treated with cells at a low concentration (Group 1 M) showed significant pain relief on study days 7, 21 and 26 (p<0.05) and on study days 10, 14 and 17 (p<0.01) compared to the same group on study day 5 before treatment: 28.08±7.30 g on study day 7 vs. 46.67±2.79 g on study day 5 (p<0.05). Animals treated with cells at a medium concentration (Group 2M) had significant pain relief on study days 14 (p<0.05) and on study days 10, 17 and 30 (p<0.011) compared the same group on study day 5 before treatment: 17.33±6.73 g on study day 10 vs. 47.25±.2.77 g on study day 5 (p<0.01). Animals treated with cells at a high concentration (Group 3M) had significant pain relief during all testing days (7, 10, 14, 17, 21, 26, 30) compared to the same group on study day 5 before treatment: 23.58±6.26 g on study day 7 vs. 46.58±2.30 g on study day 5 (p<0.01).

Treatment with the positive control item, gabapentin (Group 5M), had significant pain relief during all testing days compared to the same group on study day 5 before treatment: 14.92±6.46 g on study day 7 vs. 45.17±3.66 g on study day 5 (p<0.01).

In view of the findings obtained under the conditions of this study and confined to the in-life data, the hUTC at low, medium and high doses were effective as pain analgesic items as reflected in the parameters of the Von Frey test.

### EXAMPLE 5

### Isolation Of Cells

Umbilical cell isolation. Umbilical cords were obtained from National Disease Research Interchange (NDRI, Philadelphia, Pa.). The tissues were obtained following normal deliveries. The cell isolation protocols were performed aseptically in a laminar flow hood. To remove blood and debris, the cord was washed in phosphate buffered saline (PBS; Invitrogen, Carlsbad, Ca.) in the presence of penicillin at 100 Units/milliliter, streptomycin at 100 milligrams/milliliter and amphotericin B at .25 micrograms/milliliter (Invitrogen, Carlsbad, Ca.). The tissues were then mechanically dissociated in 150 cm2 tissue culture plates in the presence to 50 milliliters of medium (DMEM-low glucose and DMEM-high glucose; Invitrogen) until the tissue was minced into a fine pulp. The chopped tissues were transferred to 50 milliliter conical tubes (approximately 5 grams of tissue per tube).

The tissue was then digested in either DMEM-low glucose medium or DMEM-high glucose medium, each containing penicillin at 100 Units/milliliter, streptomycin at 100 milligrams/milliliter, amphotericin B at 0.25 micrograms/milliliter and the digestion enzymes. In some experiments an enzyme mixture of collagenase and dispase was used ("C:D") (collagenase (Sigma, St Louis, Mo.), 500 Units/milliliter; and dispase (Invitrogen), 50 Units/milliliter, in DMEM-Low glucose medium). In other experiments a mixture of collagenase, dispasc and hyaluronidase ("C:D:H") was used (C:D:H = collagenase, 500 Units/milliliter; dispase, 50 Units/milliliter; and hyaluronidase (Sigma), 5 Units/milliliter, in DMEM-Low glucose). The conical tubes containing the tissue, medium and digestion enzymes were incubated at 37°C in an orbital shaker (Environ, Brooklyn, N.Y.) at 225 rpm for 2 hrs.

After digestion, the tissues were centrifuged at 150 x g for 5 minutes, the supernatant was aspirated. The pellet was resuspended in 20 milliliters of growth medium (DMEM:Low glucose (Invitrogen), 15 percent (v/v) fetal bovine serum (FBS; defined fetal bovine serum; Lot #AND 18475; Hyclone, Logan, Ut.), 0.001 % (v/v) 2-mercaptoethanol (Sigma), penicillin at 100 units per milliliter, streptomycin at 100 micrograms per milliliter, and amphotericin B at 0.25 micrograms per milliliter; (each from Invitrogen, Carlsbad, Ca)). The cell suspension was filtered through a 70-micron nylon BD FALCON Cell Strainer (BD Biosciences, San Jose, Ca.). An additional 5 milliliters rinse comprising growth medium was passed through the strainer. The cell suspension was then passed through a 40-micrometer nylon cell strainer (BD Biosciences, San Jose, CA) and chased with a rinse of an additional 5 milliliters of growth medium.

The filtrate was resuspended in growth medium (total volume 50 milliliters) and centrifuged at 150 x g for 5 minutes. The supernatant was aspirated and the cells were resuspended in 50 milliliters of fresh growth medium. This process was repeated twice more.

After the final centrifugation, supernatant was aspirated and the cell pellet was resuspended in 5 milliliters of fresh growth medium. The number of viable cells was determined using trypan blue staining. Cells were then cultured under standard conditions.

The cells isolated from umbilical cord tissues were seeded at 5,000 cells/cm² onto gelatin-coated T-75 flasks (Corning Inc., Corning, N.Y.) in growth medium. After two days, spent medium and unadhered cells were aspirated from the flasks. Adherent cells were washed with PBS three times to remove debris and blood-derived cells. Cells were then replenished with growth medium and allowed to grow to confluence (about 10 days from passage 0 (to passage 1). On subsequent passages (from passage 1 to 2 etc), cells reached sub-confluence (75-85 percent confluence) in 4-5 days. For these subsequent passages, cells were seeded at 5,000 cells/cm². Cells were grown in a humidified incubator with 5 percent carbon dioxide at 37°C.

In some experiments, cells were isolated from umbilical cord tissues in DMEM-low glucose medium after digested with LIBERASE™ (2.5 milligrams per milliliter, Blendzyme 3; Roche Applied Sciences, Indianapolis, IN) and hyaluronidase (5 Units/milliliter, Sigma). Digestion of the tissue and isolation of the cells was as described for other protease digestions above, however, the LIBERASE™ /hyaluronidase mixture was used instead of the C:D or C:D:H enzyme mixture. Tissue digestion with LIBERASE™ resulted in the isolation of cell populations from postpartum tissues that expanded readily.

Procedures were compared for isolating cells from the umbilical cord using differing enzyme combinations. Enzymes compared for digestion included: i) collagenase; ii) dispase; iii) hyaluronidase; iv) collagenase:dispase mixture (C:D); v) collagenase:hyaluronidase mixture (C:H); vi) dispase:hyaluronidase mixture (D:H); and vii) collagenase:dispase:hyaluronidase mixture (C:D:H). Differences in cell isolation utilizing these different enzyme digestion conditions were observed (Table 4-1).

Other attempts were made to isolate pools of cells from umbilical cord by different approaches. In one instance, umbilical cord was sliced and washed with growth medium to dislodge the blood clots and gelatinous material. The mixture of blood, gelatinous material and growth medium was collected and centrifuged at 150 x g. The pellet was resuspended and seeded onto gelatin coated flasks in growth medium. From these experiments a cell population was isolated that readily expanded.

Cells have also been isolated from cord blood samples obtained from NDRI. The isolation protocol used was that of International Patent Application PCT/US2002/029971 by Ho et al. Samples (50 milliliter and 10.5 milliliters, respectively) of umbilical cord blood (NDRI, Philadelphia Pa.) were mixed with lysis buffer (filter-sterilized 155 millimolar ammonium chloride, 10 millimolar potassium bicarbonate, 0.1 millimolar EDTA buffered to pH 7.2 (all components from Sigma, St. Louis, Mo.). Cells were lysed at a ratio of 1:20 cord blood to lysis buffer. The resulting cell suspension was vortexed for 5 seconds, and incubated for 2 minutes at ambient temperature. The lysate was centrifuged (10 minutes at 200 x g). The cell pellet was resuspended in Complete Minimal Essential Medium (Gibco, Carlsbad Ca.) containing 10 percent fetal bovine serum (Hyclone, Logan Ut.), 4 millimolar glutamine (Mediatech Herndon, Va.), penicillin at 100 Units per milliliter and streptomycin at 100 micrograms per milliliter (Gibco, Carlsbad, Ca.). The resuspended cells were centrifuged (10 minutes at 200 x g), the supernatant was aspirated, and the cell pellet was washed in complete medium. Cells were seeded directly into either T75 flasks (Corning, N.Y.), T75 laminin-coated flasks, or T175 fibronectin-coated flasks (both Becton Dickinson, Bedford, Ma.).

To determine whether cell populations could be isolated under different conditions and expanded under a variety of conditions immediately after isolation, cells were digested in growth medium with or without 0.001 percent (v/v) 2-mercaptoethanol (Sigma, St. Louis, Mo.), using the enzyme combination of C:D:H, according to the procedures provided above. All cells were grown in the presence of penicillin at 100 Units per milliliter and streptomycin at 100 micrograms per milliliter. Under all tested conditions cells attached and expanded well between passage 0 and 1 (Table 4-2). Cells in conditions 5-8 and 13-16 were demonstrated to proliferate well up to 4 passages after seeding, at which point they were cryopreserved.

The combination of C:D:H, provided the best cell yield following isolation, and generated cells that expanded for many more generations in culture than the other conditions (Table 4-1). An expandable cell population was not attained using collagenase or hyaluronidase alone. No attempt was made to determine if this result is specific to the collagenase that was tested.

**Table 4-1: Isolation of cells from umbilical cord tissue using varying enzyme combinations**

| **Enzyme Digest** | **Cells Isolated** | **Cell Expansion** |
|---|---|---|
| Collagenase | X | X |
| Dispase | + (>10 h) | + |
| Hyaluronidase | X | X |
| Collagenase:Dispase | ++ (<3 h) | ++ |
| Collagenase:Hyaluronidase | ++ (<3 h) | + |
| Dispase:Hyaluronidase | + (>10 h) | + |
| Collagenase:Dispase:Hyaluronidase | +++ (<3 h) | +++ |

| | | |
|---|---|---|
| Key: + = good, ++ = very good, +++ = excellent, X = no success | | |

Cells attached and expanded well between passage 0 and 1 under all conditions tested for enzyme digestion and growth (Table 4-2). Cells in experimental conditions 5-8 and 13-16 proliferated well up to 4 passages after seeding, at which point they were cryopreserved. All cells were cryopreserved for further analysis.

**Table 4-2: Isolation and culture expansion of postpartum cells under varying conditions:**

| **Condition** | **Medium** | **15% FBS** | **BME** | **Gelatin** | **20% O₂** | **Growth Factors** |
|---|---|---|---|---|---|---|
| 1 | DMEM-Lg | Y | Y | Y | Y | N |
| 2 | DMEM-Lg | Y | Y | Y | N (5%) | N |
| 3 | DMEM-Lg | Y | Y | N | Y | N |
| 4 | DMEM-Lg | Y | Y | N | N (5%) | N |
| 5 | DMEM-Lg | N (2%) | Y | N (Laminin) | Y | EGF/FGF (20 ng/ml) |
| 6 | DMEM-Lg | N (2%) | Y | N (Laminin) | N (5%) | EGF/FGF (20 ng/ml) |
| 7 | DMEM-Lg | N (2%) | Y | N (Fibronectin) | Y | PDGFNEGF |
| 8 | DMEM-Lg | N (2%) | Y | N (Fibronectin) | N (5%) | PDGFNEGF |
| 9 | DMEM-Lg | Y | N | Y | Y | N |
| 10 | DMEM-Lg | Y | N | Y | N (5%) | N |
| 11 | DMEM-Lg | Y | N | N | Y | N |
| 12 | DMEM-Lg | Y | N | N | N (5%) | N |
| 13 | DMEM-Lg | N (2%) | N | N (Laminin) | Y | EGF/FGF (20 ng/ml) |
| 14 | DMEM-Lg | N (2%) | N | N (Laminin) | N (5%) | EGF/FGF (20 ng/ml) |
| 15 | DMEM-Lg | N (2%) | N | N (Fibronectin) | Y | PDGFNEGF |
| 16 | DMEM-Lg | N (2%) | N | N (Fibronectin) | N (5%) | PDGFNEGF |

Nucleated cells attached and grew rapidly. These cells were analyzed by flow cytometry and were similar to cells obtained by enzyme digestion.

The preparations contained red blood cells and platelets. No nucleated cells attached and divided during the first 3 weeks. The medium was changed 3 weeks after seeding and no cells were observed to attach and grow.

Populations of cells could be isolated from umbilical tissue efficiently using the enzyme combination collagenase (a metalloprotease), dispase (neutral protease) and hyaluronidase (mucolytic enzyme which breaks down hyaluronic acid). LIBERASE, which is a blend of collagenase and a neutral protease, may also be used. Blendzyme 3, which is collagenase (4 Wunsch units/gram) and thermolysin (1714 casein units/gram), was also used together with hyaluronidase to isolate cells. These cells expanded readily over many passages when cultured in growth expansion medium on gelatin coated plastic.

Cells were also isolated from residual blood in the cords, but not cord blood. The presence of cells in blood clots washed from the tissue, which adhere and grow under the conditions used, may be due to cells being released during the dissection process.

### EXAMPLE 6

### Growth Characteristics of Cells

The cell expansion potential of umbilical cord tissue-derived cells was compared to other populations of isolated stem cells. The process of cell expansion to senescence is referred to as Hayflick's limit (Hayflick L., J. Am. Geriatr. Soc. 1974; 22(1):1-12; Hayflick L., Gerontologist, 1974; 14(1):37-45.

Tissue culture plastic flasks were coated by adding 20 milliliters 2% (w/v) gelatin (Type B: 225 Bloom; Sigma, St Louis, Mo.) to a T75 flask (Corning Inc., Corning, N.Y.) for 20 minutes at room temperature. After removing the gelatin solution, 10 milliliters of phosphate-buffered saline (PBS) (Invitrogen, Carlsbad, Ca.) was added and then aspirated.

For comparison of growth expansion potential the following cell populations were utilized; i) mesenchymal stem cells (MSC; Cambrex, Walkersville, Md.); ii) adipose-derived cells (US Patent No. 6,555,374 B1; US Patent Application US20040058412); iii) normal dermal skin fibroblasts (cc-2509 lot # 9F0844; Cambrex, Walkersville, Md.); and iv) umbilicus-derived cells. Cells were initially seeded at 5,000 cells/cm² on gelatin-coated T75 flasks in growth medium. For subsequent passages, cell cultures were treated as follows. After trypsinization, viable cells were counted after trypan blue staining. Cell suspension (50 microliters) was combined with trypan blue (50 microliters, Sigma, St. Louis Mo.). Viable cell numbers were estimated using a hemocytometer.

Following counting, cells were seeded at 5,000 cells/cm² onto gelatin-coated T 75 flasks in 25 milliliters of fresh growth medium. Cells were grown in a standard atmosphere (5 percent carbon dioxide (v/v)) at 37 °C. The growth medium was changed twice per week. When cells reached about 85 percent confluence they were passaged; this process was repeated until the cells reached senescence.

At each passage, cells were trypsinized and counted. The viable cell yield, population doublings [ln (cells final/cells initial)/ln2], and doubling time (time in culture/population doubling) were calculated. For the purposes of determining optimal cell expansion, the total cell yield per passage was determined by multiplying the total yield for the previous passage by the expansion factor for each passage (i.e. expansion factor = cells final/cells initial).

The expansion potential of cells banked at passage 10 was also tested. A different set of conditions was used. Normal dermal skin fibroblasts (cc-2509 lot # 9F0844; Cambrex, Walkersville, Md.), umbilicus-derived cells were tested. These cell populations had been banked at passage 10 previously, having been cultured at 5,000 cells/cm² at each passage to that point. The effect of cell density on the cell populations following cell thaw at passage 10 was determined. Cells were thawed under standard conditions and counted using trypan blue staining. Thawed cells were then seeded at 1,000 cells/cm² in growth medium. Cells were grown under standard atmospheric conditions at 37 °C. growth medium was changed twice a week. Cells were passaged as they reached about 85% confluence. Cells were subsequently passaged until senescence, *i.e.,* until they could not be expanded any further. Cells were trypsinized and counted at each passage. The cell yield, population doubling (In (cells final/cells initial)/ln2) and doubling time (time in culture)/population doubling) were calculated. The total cell yield per passage was determined by multiplying total yield for the previous passage by the expansion factor for each passage (i.e., expansion factor = cells final/cells initial).

The expansion potential of freshly isolated umbilical cord tissue-derived cell cultures under low cell seeding conditions was tested in another experiment. Umbilicus-derived cells were isolated as described in Example 4. Cells were seeded at 1,000 cells/cm² and passaged as described above until senescence. Cells were grown under standard atmospheric conditions at 37°C. Growth medium was changed twice per week. Cells were passaged as they reached about 85% confluence. At each passage, cells were trypsinized and counted by trypan blue staining. The cell yield, population doubling (In (cell final/cell initial)/ln 2) and doubling time (time in culture/population doubling) were calculated for each passage. The total cell yield per passage was determined by multiplying the total yield for the previous passage by the expansion factor for each passage *(i.e.,* expansion factor = cell final/cell initial). Cells were grown on gelatin and non-gelatin coated flasks.

It has been demonstrated that low O₂ cell culture conditions can improve cell expansion in certain circumstances (US Publication Number US20040005704). In order to determine if cell expansion of umbilicus-derived cells could be improved by altering cell culture conditions, cultures of umbilicus-derived cells were grown in low oxygen conditions. Cells were seeded at 5,000 cells/cm² in growth medium on gelatin coated flasks. Cells were initially cultured under standard atmospheric conditions through passage 5, at which point they were transferred to low oxygen (5% O₂) culture conditions.

In other experiments cells were expanded on non-coated, collagen-coated, fibronectin-coated, laminin-coated and matrigel-coated plates. Cultures have been demonstrated to expand well on these different matrices.

Umbilicus-derived cells expanded for more than 40 passages generating cell yields of > 1 E17 cells in 60 days. In contrast, MSCs and fibroblasts senesced after < 25 days and <60 days, respectively. Although both adipose-derived and omental cells expanded for almost 60 days, they generated total cell yields of 4.5x10¹² and 4.24x10¹³ respectively. Thus, when seeded at 5,000 cells/cm² under the experimental conditions utilized, umbilicus-derived cells expanded much better than the other cell types grown under the same conditions (Table 6-1).

**Table 6-1: Growth characteristics for different cell populations grown to senescence**

| **Cell Type** | **Senescence** | **Total Population Doublings** | **Yield (Total Cells)** |
|---|---|---|---|
| MSC | 24 d | 8 | 4.72 E7 |
| Adipose-derived cell | 57 d | 24 | 4.5 E12 |
| Fibroblasts | 53 d | 26 | 2.82 E13 |
| Umbilical | 65 d | 42 | 6.15 E17 |

Umbilicus-derived cells and fibroblast cells expanded for greater than 10 passages generating cell yields of > 1E11 cells in 60 days (6-2). After 60 days under these conditions, the fibroblasts became senesced; whereas the umbilicus-derived cells senesced after 80 days, completing >50 population doublings.

**Table 6-2: Growth characteristics for different cell populations using low density growth expansion from passage 10 through senescence**

| **Cell Type (Passage No.)** | **Senescence** | **Total Population Doublings** | **Yield (Total Cells)** |
|---|---|---|---|
| Fibroblast (P10) | 80 days | 43.68 | 2.59 E11 |
| Umbilical (P10) | 80 days | 53.6 | 1.25 E14 |

Cells expanded well under the reduced oxygen conditions, however, culturing under low oxygen conditions does not appear to have a significant effect on cell expansion for umbilical cord tissue-derived cells. Standard atmospheric conditions have already proven successful for growing sufficient numbers of cells, and low oxygen culture is not required for the growth of umbilical cord tissue-derived cells.

The current cell expansion conditions of growing isolated umbilical cord tissue-derived cells at densities of about 5,000 cells/cm², in growth medium on gelatin-coated or uncoated flasks, under standard atmospheric oxygen, are sufficient to generate large numbers of cells at passage 11. Furthermore, the data suggests that the cells can be readily expanded using lower density culture conditions (e.g., 1,000 cells/cm²). Umbilical cord tissue -derived cell expansion in low oxygen conditions also facilitates cell expansion, although no incremental improvement in cell expansion potential has yet been observed when utilizing these conditions for growth. Presently, culturing umbilical cord tissue-derived cells under standard atmospheric conditions is preferred for generating large pools of cells. When the culture conditions are altered, however, umbilical cord tissue-derived cell expansion can likewise be altered. This strategy may be used to enhance the proliferative and differentiative capacity of these cell populations.

Under the conditions utilized, while the expansion potential of MSC and adipose-derived cells is limited, umbilical cord tissue-derived cells expand readily to large numbers.

### EXAMPLE 7

### Growth of Cells in Medium Containing D-Valine

It has been reported that medium containing D-valine instead of the normal L-valine isoform can be used to selectively inhibit the growth of fibroblast-like cells in culture (Hongpaisan, Cell Biol Int., 2000; 24:1-7; Sordillo et al., Cell Biol Int Rep., 1988; 12:355-64.). Experiments were performed to determine whether umbilical cord tissue-derived cells could grow in medium containing D-valine.

Umbilicus-derived cells (P5) and fibroblasts (P9) were seeded at 5,000 cells/cm² in gelatin-coated T75 flasks (Corning, Corning, N.Y.). After 24 hours the medium was removed and the cells were washed with phosphate buffered saline (PBS) (Gibco, Carlsbad, Ca.) to remove residual medium. The medium was replaced with a modified growth medium (DMEM with D-valine (special order Gibco), 15% (v/v) dialyzed fetal bovine serum (Hyclone, Logan, Ut.), 0.001% (v/v) betamercaptoethanol (Sigma), penicillin at 50 Units/milliliter and streptomycin at 50 milligrams/milliliter (Gibco)).

Umbilicus-derived cells and fibroblast cells seeded in the D-valine-containing medium did not proliferate, unlike cells seeded in growth medium containing dialyzed serum. Fibroblasts cells changed morphologically, increasing in size and changing shape. All of the cells died and eventually detached from the flask surface after four weeks. Thus, it may be concluded that umbilical cord tissue-derived cells require L-valine for cell growth and to maintain long-term viability. L-valine is preferably not removed from the growth medium for umbilical cord tissue-derived cells.

### EXAMPLE 8

### Karyotype Analysis of Cells

Cell lines used in cell therapy are preferably homogeneous and free from any contaminating cell type. Human cells used in cell therapy should have a normal number (46) of chromosomes with normal structure. To identify umbilical cord tissue-derived cell lines that are homogeneous and free from cells of non-postpartum tissue origin, karyotypes of cell samples were analyzed.

Umbilical cord tissue-derived cells from postpartum tissue of a male neonate were cultured in growth media. Umbilical cord tissue from a male neonate (X,Y) was selected to allow distinction between neonatal-derived cells and maternal derived cells (X,X). Cells were seeded at 5,000 cells per square centimeter in growth medium in a T25 flask (Corning, Corning, N.Y.) and expanded to 80% confluence. A T25 flask containing cells was filled to the neck with growth media. Samples were delivered to a clinical cytogenetics lab by courier (estimated lab to lab transport time is one hour). Chromosome analysis was performed by the Center for Human & Molecular Genetics at the New Jersey Medical School, Newark, N.J. Cells were analyzed during metaphase when the chromosomes are best visualized. Of twenty cells in metaphase counted, five were analyzed for normal homogeneous karyotype number (two). A cell sample was characterized as homogeneous if two karyotypes were observed. A cell sample was characterized as heterogeneous if more than two karyotypes were observed. Additional metaphase cells were counted and analyzed when a heterogeneous karyotype number (four) was identified.

All cell samples sent for chromosome analysis were interpreted by the cytogenetics laboratory staff as exhibiting a normal appearance. Three of the sixteen cell lines analyzed exhibited a heterogeneous phenotype (XX and XY) indicating the presence of cells derived from both neonatal and maternal origins (Table 8-1). Each of the cell samples was characterized as homogeneous. (Table 8-1).

**Table 8-1. Karyotype results of umbilical cord tissue-derived cells.**

| Tissue | passage | Metaphase cells counted | Metaphase cells analyzed | Number of karyotypes | ISCN Karyotype |
|---|---|---|---|---|---|
| Umbilical | 23 | 20 | 5 | 2 | 46, XX |
| Umbilical | 6 | 20 | 5 | 2 | 46, XY |
| Umbilical | 3 | 20 | 5 | 2 | 46, XX |

| | | | | | |
|---|---|---|---|---|---|
| Key: N- Neonatal side; V- villous region; M- maternal side; C- clone | | | | | |

Chromosome analysis identified umbilicus-derived cells whose karyotypes appear normal as interpreted by a clinical cytogenetic laboratory. Karyotype analysis also identified cell lines free from maternal cells, as determined by homogeneous karyotype.

### EXAMPLE 9

### Flow Cytometric Evaluation of Cell Surface Markers

Characterization of cell surface proteins or "markers" by flow cytometry can be used to determine a cell line's identity. The consistency of expression can be determined from multiple donors, and in cells exposed to different processing and culturing conditions. Cell lines isolated from the umbilicus were characterized by flow cytometry, providing a profile for the identification of these cell lines.

Cells were cultured in growth medium, in plasma-treated T75, T150, and T225 tissue culture flasks (Corning, Corning, N.Y.) until confluent. The growth surfaces of the flasks were coated with gelatin by incubating 2% (w/v) gelatin (Sigma, St. Louis, Mo.) for 20 minutes at room temperature.

Adherent cells in flasks were washed in phosphate buffered saline (PBS); (Gibco, Carlsbad, Mo.) and detached with trypsin/EDTA (Gibco). Cells were harvested, centrifuged, and resuspended in 3% (v/v) FBS in PBS at a cell concentration of 1x10⁷ per milliliter. In accordance with the manufacture's specifications, antibody to the cell surface marker of interest (see below) was added to 100 microliters of cell suspension and the mixture was incubated in the dark for 30 minutes at 4 °C. After incubation, cells were washed with PBS and centrifuged to remove unbound antibody. Cells were resuspended in 500 microliters PBS and analyzed by flow cytometry. Flow cytometry analysis was performed with a FACS calibur instrument (Becton Dickinson, San Jose, Ca.).

The following antibodies to cell surface markers were used.

**Table 9-1: Antibodies used in characterizing cell surface markers of an UDC.**

| **Antibody** | **Manufacture** | **Catalog Number** |
|---|---|---|
| CD10 | BD Pharmingen (San Diego, CA) | 555375 |
| CD13 | BD Pharmingen (San Diego, CA) | 555394 |
| CD31 | BD Pharmingen (San Diego, CA) | 555446 |
| CD34 | BD Pharmingen (San Diego, CA) | 555821 |
| CD44 | BD Pharmingen (San Diego, CA) | 555478 |
| CD45RA | BD Pharmingen (San Diego, CA) | 555489 |
| CD73 | BD Pharmingen (San Diego, CA) | 550257 |
| CD90 | BD Pharmingen (San Diego, CA) | 555596 |
| CD117 | BD Biosciences (San Jose, CA) | 340529 |
| CD141 | BD Pharmingen (San Diego, CA) | 559781 |
| PDGFr-alpha | BD Pharmingen (San Diego, CA) | 556002 |
| HLA-A, B, C | BD Pharmingen (San Diego, CA) | 555553 |
| HLA-DR, DP, DQ | BD Pharmingen (San Diego, CA) | 555558 |
| IgG-FITC | Sigma (St. Louis, MO) | F-6522 |
| IgG- PE | Sigma (St. Louis, MO) | P-4685 |

Umbilical cord cells were analyzed at passages 8, 15, and 20.

To compare differences among donors, umbilical cord-derived cells from different donors were compared to each other.

Umbilical cord-derived cells cultured on gelatin-coated flasks were compared to umbilical cord-derived cells cultured on uncoated flasks.

Four treatments used for isolation and preparation of cells were compared. Cells derived from postpartum tissue by treatment with: (1) collagenase; (2) collagenase/dispase; (3) collagenase/hyaluronidase; and (4) collagenase/hyaluronidase/dispase were compared.

Umbilical cord-derived cells at passage 8, 15, and 20 analyzed by flow cytometry all expressed CD 10, CD13, CD44, CD73, CD 90, PDGFr-alpha and HLA-A, B, C, indicated by increased fluorescence relative to the IgG control. These cells were negative for CD31, CD34, CD45, CD117, CD141, and HLA-DR, DP, DQ, indicated by fluorescence values consistent with the IgG control.

Umbilical cord-derived cells isolated from separate donors analyzed by flow cytometry each showed positive for production of CD10, CD13, CD44, CD73, CD 90, PDGFr-alpha and HLA-A, B, C, reflected in the increased values of fluorescence relative to the IgG control. These cells were negative for production of CD31, CD34, CD45, CD117, CD141, and HLA-DR, DP, DQ with fluorescence values consistent with the IgG control.

Umbilical cord-derived cells expanded on gelatin coated and uncoated flasks analyzed by flow cytometry were all positive for production of CD10, CD13, CD44, CD73, CD 90, PDGFr-alpha and HLA-A, B, C, with increased values of fluorescence relative to the IgG control. These cells were negative for production of CD31, CD34, CD45, CD117, CD 141, and HLA-DR, DP, DQ, with fluorescence values consistent with the IgG control.

Analysis of umbilical cord-derived cells by flow cytometry has established an identity of these cell lines. These umbilical cord-derived cells are positive for CD10, CD13, CD44, CD73, CD90, PDGFr-alpha, and HLA-A,B,C; and negative for CD31, CD34, CD45, CD117, CD141 and HLA-DR, DP, DQ. This identity was consistent between variations in variables including the donor, passage, culture vessel surface coating, digestion enzymes, and placental layer. Some variation in individual fluorescence value histogram curve means and ranges were observed, but all positive curves under all conditions tested were normal and expressed fluorescence values greater than the IgG control, thus confirming that the cells comprise a homogeneous population which has positive expression of the markers.

### EXAMPLE 10

### Analysis of Cells by Oligonucleotide Array

Oligonucleotide arrays were used to compare gene expression profiles of umbilicus-derived and placenta-derived cells with fibroblasts, human mesenchymal stem cells, and another cell line derived from human bone marrow. This analysis provided a characterization of the postpartum-derived cells and identified unique molecular markers for these cells.

*Postpartum tissue-derived cells.* Human umbilical cords and placenta were obtained from National Disease Research Interchange (NDRI, Philadelphia, Pa.) from normal full term deliveries with patient consent. The tissues were received and cells were isolated as described in Example 4 after digestion with a C:D:H mixture. Cells were cultured in growth medium on gelatin-coated plastic tissue culture flasks. The cultures were incubated at 37° C with 5% CO₂.

*Fibroblasts*. Human dermal fibroblasts were purchased from Cambrex Incorporated (Walkersville, Md.; Lot number 9F0844) and ATCC CRL-1501 (CCD39SK). Both lines were cultured in DMEM/F12 medium (Invitrogen, Carlsbad, Ca.) with 10% (v/v) fetal bovine serum (Hyclone) and penicillin/streptomycin (Invitrogen)). The cells were grown on standard tissue-treated plastic.

*Human Mesenchymal Stem Cells (hMSC).* hMSCs were purchased from Cambrex Incorporated (Walkersville, Md.; Lot numbers 2F1655, 2F1656 and 2F1657) and cultured according to the manufacturer's specifications in MSCGM Media (Cambrex). The cells were grown on standard tissue cultured plastic at 37 °C with 5% CO₂.

*Human Iliac Crest Bone Marrow Cells (ICBM).* Human iliac crest bone marrow was received from NDRI with patient consent. The marrow was processed according to the method outlined by Ho, et al. (WO03/025149). The marrow was mixed with lysis buffer (155 mM NH₄Cl, 10 mM KHCO₃, and 0.1 mM EDTA, pH 7.2) at a ratio of 1 part bone marrow to 20 parts lysis buffer. The cell suspension was vortexed, incubated for 2 minutes at ambient temperature, and centrifuged for 10 minutes at 500 x g. The supernatant was discarded and the cell pellet was resuspended in Minimal Essential Medium-alpha (Invitrogen) supplemented with 10% (v/v) fetal bovine serum and 4 mM glutamine. The cells were centrifuged again and the cell pellet was resuspended in fresh medium. The viable mononuclear cells were counted using trypan blue exclusion (Sigma, St. Louis, Mo.). The mononuclear cells were seeded in plastic tissue culture flasks at 5 x 10⁴ cells/cm². The cells were incubated at 37 °C with 5% CO₂ at either standard atmospheric O₂ or at 5% O₂. Cells were cultured for 5 days without a media change. Media and non-adherent cells were removed after 5 days of culturing. The adherent cells were maintained in culture.

Actively growing cultures of cells were removed from the flasks with a cell scraper in cold phosphate buffered saline (PBS). The cells were centrifuged for 5 minutes at 300 x g. The supernatant was removed and the cells were resuspended in fresh PBS and centrifuged again. The supernatant was removed and the cell pellet was immediately frozen and stored at-80° C. Cellular mRNA was extracted and transcribed into cDNA. cDNA was then transcribed into cRNA and biotin-labeled. The biotin-labeled cRNA was hybridized with Affymetrix GENECHIP HG-U133A oligonucleotide arrays (Affymetrix, Santa Clara, Ca.). The hybridizations and data collection were performed according to the manufacturer's specifications. The hybridization and data collection was performed according to the manufacturer's specifications. Data analyses were performed using "Significance Analysis of Microarrays" (SAM) version 1.21 computer software (Tusher, V.G. et al., Proc. Natl. Acad. Sci. USA, 2001; 98:5116-5121).

Different populations of cells were analyzed in this study. The cells along with passage information, culture substrate, and culture media are listed in Table 9-1.

**Table 10-1. Cells analyzed by the microarray study. The cells lines are listed by their identification code along with passage at the time of analysis, cell growth substrate, and growth media.**

| | | | |
|---|---|---|---|
| Umbilical (022803) | 2 | Gelatin | DMEM, 15% FBS, 2BME |
| Umbilical (042103) | 3 | Gelatin | DMEM, 15% FBS, 2BME |
| Umbilical (071003) | 4 | Gelatin | DMEM, 15% FBS, 2BME |
| Placenta (042203) | 12 | Gelatin | DMEM, 15% FBS, 2BME |
| Placenta (042903) | 4 | Gelatin | DMEM, 15% FBS, 2BME |
| Placenta (071003) | 3 | Gelatin | DMEM, 15% FBS, 2BME |
| ICBM (070203) (5% O₂) | 3 | Plastic | MEM 10% FBS |
| ICBM (062703) (std O₂) | 5 | Plastic | MEM 10% FBS |
| ICBM (062703)(5% O₂) | 5 | Plastic | MEM 10% FBS |
| hMSC (Lot 2F1655) | 3 | Plastic | MSCGM |
| hMSC (Lot 2F1656) | 3 | Plastic | MSCGM |
| hMSC (Lot 2F1657) | 3 | Plastic | MSCGM |
| hFibroblast (9F0844) | 9 | Plastic | DMEM-F12, 10% FBS |
| hFibroblast (CCD39SK) | 4 | Plastic | DMEM-F12, 10% FBS |

The data were evaluated by principle component analysis with SAM software as described above. Analysis revealed 290 genes that were expressed in different relative amounts in the cells tested. This analysis provided relative comparisons between the populations.

Table 10-2 shows the Euclidean distances that were calculated for the comparison of the cell pairs. The Euclidean distances were based on the comparison of the cells based on the 290 genes that were differentially expressed among the cell types. The Euclidean distance is inversely proportional to similarity between the expression of the 290 genes.

**Table 10-2. The Euclidean Distances for the Cell Pairs. The Euclidean distance was calculated for the cell types using the 290 genes that were-expressed differentially between the cell types. Similarity between the cells is inversely proportional to the Euclidean distance.**

| Cell Pair | **Euclidean Distance** |
|---|---|
| ICBM-hMSC | 24.71 |
| Placenta-umbilical | 25.52 |
| ICBM-Fibroblast | 36.44 |
| ICBM-placenta | 37.09 |
| Fibroblast-MSC | 39.63 |
| ICBM-Umbilical | 40.15 |
| Fibroblast-Umbilical | 41.59 |
| MSC-Placenta | 42.84 |
| MSC-Umbilical | 46.86 |
| ICBM-placenta | 48.41 |

Tables 10-3, 10-4, and 10-5 show the expression of genes increased in umbilical tissue-derived cells (Table 10-3) and placenta-derived cells (Table 10-4), increased in, and reduced in umbilical cord and placenta-derived cells (Table 10-5).

**Table 10-3. Genes that are specifically increased in expression in umbilical cord-derived cells as compared to the other cell lines assayed.**

| **Genes Increased in Umbilicus-Derived Cells** | | |
|---|---|---|
| **Probe Set ID** | **Gene Name** | **NCBI Accession Number** |
| 202859_x_at | Interleukin 8 | NM_000584 |
| 211506_s_at | Interleukin 8 | AF043337 |
| 210222_s_at | reticulon 1 | BC000314 |
| 204470_at | chemokine (C-X-C motif) ligand 1 (melanoma growth stimulating activity | NM_001511 |
| 206336_at | chemokine (C-X-C motif) ligand 6 (granulocyte chemotactic protein 2) | NM_002993 |
| 207850_at | Chemokine (C-X-C motif) ligand 3 | NM_002090 |
| 203485_at | reticulon 1 | NM_021136 |
| 202644_s_at | tumor necrosis factor, alpha-induced protein 3 | NM_006290 |

**Table 10-4. Genes that are specifically increased in expression in the placenta-derived cells as compared to the other cell lines assayed.**

| **Genes Increased in Placenta-Derived Cells** | | |
|---|---|---|
| **Probe Set ID** | **Gene Name** | **NCBI Accession Number** |
| 209732_at | C-type (calcium dependent, carbohydrate-recognition domain) lectin, superfamily member 2 (activation-induced) | AF070642 |
| 206067_s_at | Wilms tumor 1 | NM_024426 |
| 207016_s_at | aldehyde dehydrogenase 1 family, member A2 | AB015228 |
| 206367_at | Renin | NM_000537 |
| 210004_at | oxidized low density lipoprotein (lectin-like) receptor 1 | AF035776 |
| 214993_at | *Homo sapiens,* clone IMAGE:4179671, mRNA, partial cds | AF070642 |
| 202178_at | protein kinase C, zeta | NM_002744 |
| 209780_at | hypothetical protein DKFZp564F013 | AL136883 |
| 204135_at | downregulated in ovarian cancer 1 | NM_014890 |
| 213542_at | *Homo sapiens* mRNA; cDNA DKFZp547K1113 (from clone DKFZp547K1113) | AI246730 |

**Table 10-5. Genes that were decreased in expression in the umbilical cord-derived and the placenta-derived cells as compared to the other cell lines assayed.**

| **Genes Decreased in Umbilicus- and Placenta-Derived Cells** | | |
|---|---|---|
| **Probe Set ID** | **Gene name** | **NCBI Accession Number** |
| 210135_s_at | short stature homeobox 2 | AF022654.1 |
| 205824_at | heat shock 27kDa protein 2 | NM_001541.1 |
| 209687_at | chemokine (C-X-C motif) ligand 12 (stromal cell-derived factor 1) | U19495.1 |
| 203666_at | chemokine (C-X-C motif) ligand 12 (stromal cell-derived factor 1) | NM_000609.1 |
| 212670_at | elastin (supravalvular aortic stenosis, Williams-Beuren syndrome) | AA479278 |
| 213381_at | *Homo sapiens* mRNA; cDNA DKFZp586M2022 (from clone DKFZp586M2022) | N91149 |
| 206201_s_at | mesenchyme homeobox 2 (growth arrest-specific homeobox) | NM_005924.1 |
| 205817_at | Sine oculis homeobox homolog 1 (*Drosophila*) | NM_005982.1 |
| 209283_at | crystallin, alpha B | AF007162.1 |
| 212793_at | dishevelled associated activator of morphogenesis 2 | BF513244 |
| 213488_at | DKFZP586B2420 protein | AL050143.1 |
| 209763_at | similar to neuralin 1 | AL049176 |
| 205200_at | Tetranectin (plasminogen binding protein) | NM_003278.1 |
| 205743_at | src homology three (SH3) and cysteine rich domain | NM_003149.1 |
| 200921_s_at | B-cell translocation gene 1, anti-proliferative | NM_001731.1 |
| 206932_at | cholesterol 25-hydroxylase | NM_003956.1 |
| 204198_s_at | runt-related transcription factor 3 | AA541630 |
| 219747_at | hypothetical protein FLJ23191 | NM_024574.1 |
| 204773_at | Interleukin 11 receptor, alpha | NM_004512.1 |
| 202465_at | Procollagen C-endopeptidase enhancer | NM_002593.2 |
| 203706_s_at | Frizzled homolog 7 (*Drosophila*) | NM_003507.1 |
| 212736_at | hypothetical gene BC008967 | BE299456 |
| 214587_at | Collagen, type VIII, alpha 1 | BE877796 |
| 201645_at | Tenascin C (hexabrachion) | NM_002160.1 |
| 210239_at | iroquois homeobox protein 5 | U90304.1 |
| 203903_s_at | Hephaestin | NM_014799.1 |
| 205816_at | integrin, beta 8 | NM_002214.1 |
| 203069_at | synaptic vesicle glycoprotein 2 | N_014849.1 |
| 213909_at | *Homo sapiens* cDNA FLJ12280 fis, clone MAMMA1001744 | AU147799 |
| 206315_at | cytokine receptor-like factor 1 | NM_004750.1 |
| 204401_at | potassium intermediate/small conductance calcium-activated channel, subfamily N, member 4 | NM_002250.1 |
| 216331_at | integrin, alpha 7 | AK022548.1 |
| 209663_s_at | integrin, alpha 7 | AF072132.1 |
| 213125_at | DKFZP586L151 protein | AW007573 |
| 202133_at | transcriptional co-activator with PDZ-binding motif (TAZ) | AA081084 |
| 206511_s_at | Sine oculis homeobox homolog 2 (*Drosophila*) | NM_016932.1 |
| 213435_at | KIAA1034 protein | AB028957.1 |
| 206115_at | early growth response 3 | NM_004430.1 |
| 213707_s_at | distal-less homeobox 5 | N_005221.3 |
| 218181_s_at | hypothetical protein FLJ20373 | NM_017792.1 |
| 209160_at | aldo-keto reductase family 1, member C3 (3-alpha hydroxysteroid dehydrogenase, type II) | AB018580.1 |
| 213905_x_at | Biglycan | AA845258 |
| 201261_x_at | Biglycan | BC002416.1 |
| 202132_at | transcriptional co-activator with PDZ-binding motif (TAZ) | AA081084 |
| 214701_s_at | fibronectin 1 | AJ276395.1 |
| 213791_at | Proenkephalin | NM_006211.1 |
| 205422_s_at | Integrin, beta-like 1 (with EGF-like repeat domains) | NM_004791.1 |
| 214927_at | *Homo sapiens* mRNA full length insert cDNA clone EUROIMAGE 1968422 | AL359052.1 |
| 206070_s_at | EphA3 | AF213459.1 |
| 212805_at | KIAA0367 protein | AB002365.1 |
| 219789_at | natriuretic peptide receptor C/guanylate cyclase C (atrionatriuretic peptide receptor C) | AI628360 |
| 219054_at | hypothetical protein FLJ14054 | NM_024563.1 |
| 213429_at | *Homo sapiens* mRNA; cDNA DKFZp564B222 (from clone DKFZp564B222) | AW025579 |
| 204929_s_at | vesicle-associated membrane protein 5 (myobrevin) | NM_006634.1 |
| 201843_s_at | EGF-containing fibulin-like extracellular matrix protein 1 | NM_004105.2 |
| 221478_at | BCL2/adenovirus E1B 19kDa interacting protein 3-like | AL132665.1 |
| 201792_at | AE binding protein 1 | NM_001129.2 |
| 204570_at | cytochrome c oxidase subunit VIIa polypeptide 1 (muscle) | NM_001864.1 |
| 201621_at | neuroblastoma, suppression of tumorigenicity 1 | NM_005380.1 |
| 202718_at | Insulin-like growth factor binding protein 2, 36kDa | NM_000597.1 |

Tables 10-6, 10-7, and 10-8 show the expression of genes increased in human fibroblasts (Table 10-6), ICBM cells (Table 10-7), and MSCs (Table 10-8).

**Table 10-6. Genes that were increased in expression in fibroblasts as compared to the other cell lines assayed.**

| **Genes increased in fibroblasts** |
|---|
| |
| dual specificity phosphatase 2 |
| KIAA0527 protein |
| Homo sapiens cDNA: FLJ23224 fis, clone ADSU02206 |
| dynein, cytoplasmic, intermediate polypeptide 1 |
| ankyrin 3, node of Ranvier (ankyrin G) |
| inhibin, beta A (activin A, activin AB alpha polypeptide) |
| ectonucleotide pyrophosphatase/phosphodiesterase 4 (putative function) |
| KIAA1053 protein |
| microtubule-associated protein 1A |
| zinc finger protein 41 |
| HSPC019 protein |
| Homo sapiens cDNA: FLJ23564 fis, clone LNG 10773 |
| Homo sapiens mRNA; cDNA DKFZp564A072 (from clone DKFZp564A072) |
| LIM protein (similar to rat protein kinase C-binding enigma) |
| inhibitor of kappa light polypeptide gene enhancer in B-cells, kinase complex-associated protein |
| hypothetical protein FLJ22004 |
| Human (clone CTG-A4) mRNA sequence |
| ESTs, Moderately similar to cytokine receptor-like factor 2; cytokine receptor CRL2 precursor [Homo sapiens] |
| transforming growth factor, beta 2 |
| hypothetical protein MGC29643 |
| antigen identified by monoclonal antibody MRC OX-2 |

**Table 10-7. Genes that were increased in expression in the ICBM-derived cells as compared to the other cell lines assayed.**

| **Genes Increased In ICBM Cells** |
|---|
| •cardiac ankyrin repeat protein |
| •MHC class 1 region ORF |
| •integrin, alpha 10 |
| •hypothetical protein FLJ22362 |
| •UDP-N-acetyl-alpha-D-galactosamine:polypeptide N-acetylgalactosaminyltransferase 3 (GalNAc-T3) |
| •interferon-induced protein 44 |
| •SRY (sex determining region Y)-box 9 (campomelic dysplasia, autosomal sex-reversal) |
| •keratin associated protein 1-1 |
| •hippocalcin-like 1 |
| •jagged 1 (Alagille syndrome) |
| •proteoglycan 1, secretory granule |

**Table 10-8. Genes that were increased in expression in the MSC cells as compared to the other cell lines assayed.**

| **Genes Increased In MSC Cells** |
|---|
| •interleukin 26 |
| •maltase-glucoamylase (alpha-glucosidase) |
| •nuclear receptor subfamily 4, group A, member 2 |
| •v-fos FBJ murine osteosarcoma viral oncogene homolog |
| •hypothetical protein DC42 |
| •nuclear receptor subfamily 4, group A, member 2 |
| •FBJ murine osteosarcoma viral oncogene homolog B |
| •WNT1 inducible signaling pathway protein 1 |
| •MCF.2 cell line derived transforming sequence |
| •potassium channel, subfamily K, member 15 |
| •cartilage paired-class homeoprotein 1 |
| •*Homo sapiens* cDNA FLJ12232 fis, clone MAMMA1001206 |
| •*Homo sapiens* cDNA FLJ34668 fis, clone LIVER2000775 |
| •jun B proto-oncogene |
| •B-cell CLL/lymphoma 6 (zinc finger protein 51) |
| •zinc finger protein 36, C3H type, homolog (mouse) |

The present example was performed to provide a molecular characterization of the cells derived from umbilical cord and placenta. This analysis included cells derived from three different umbilical cords and three different placentas. The study also included two different lines of dermal fibroblasts, three lines of mesenchymal stem cells, and three lines of iliac crest bone marrow cells. The mRNA that was expressed by these cells was analyzed on a GENECHIP oligonucleotide array that contained oligonucleotide probes for 22,000 genes.

The analysis revealed that transcripts for 290 genes were present in different amounts in these five different cell types. These genes include seven genes specifically increased in the umbilical tissue-derived cells and ten genes that are specifically increased in the placenta-derived cells. Fifty-four genes were found to have specifically lower expression levels in placenta-derived and umbilical cord-derived cells.

The expression of selected genes has been confirmed by PCR, as shown in Example 10. Postpartum-derived cells generally, and umbilical derived cells, in particular, have distinct gene expression profiles, for example, as compared to other human cells, such as the bone marrow-derived cells and fibroblasts tested here.

### EXAMPLE 11

### Cell Markers

Gene expression profiles of cells derived from umbilical cord were compared with those of cells derived from other sources using an Affymetrix GENECHIP. Six "signature" genes were identified: oxidized LDL receptor 1, interleukin-8 (IL-8), renin, reticulon, chemokine receptor ligand 3 (CXC ligand 3), and granulocyte chemotactic protein 2 (GCP-2). These "signature" genes were expressed at relatively high levels in umbilicus-derived cells.

The procedures described in this example were conducted to verify the microarray data and compare data for gene and protein expression, as well as to establish a series of reliable assays for detection of unique identifiers for umbilical cord tissue-derived cells.

Umbilicus-derived cells (four isolates), and normal human dermal fibroblasts (NHDF; neonatal and adult) were grown in growth medium in gelatin-coated T75 flasks. mesenchymal stem cells (MSCs) were grown in mesenchymal stem cell growth medium bullet kit (MSCGM; Cambrex, Walkerville, Md.).

For IL-8 experiments, cells were thawed from liquid nitrogen and plated in gelatin-coated flasks at 5,000 cells/cm², grown for 48 hours in growth medium and then grown further for 8 hours in 10 milliliters of serum starvation medium [DMEM -low glucose (Gibco, Carlsbad, Ca.), penicillin (50 Units/milliliter), streptomycin (50 micrograms/milliliter)(Gibco) and 0.1% (w/v) Bovine Serum Albumin (BSA; Sigma, St. Louis, Mo.)]. RNA was then extracted and the supernatants were centrifuged at 150 x g for 5 minutes to remove cellular debris. Supernatants were frozen at -80°C until ELISA analysis.

The umbilical cord-derived cells, as well as human fibroblasts derived from human neonatal foreskin, were cultured in growth medium in gelatin-coated T75 flasks. Cells were frozen at passage 11 in liquid nitrogen. Cells were thawed and transferred to 15 milliliter centrifuge tubes. After centrifugation at 150 x g for 5 minutes, the supernatant was discarded. Cells were resuspended in 4 milliliters culture medium and counted. Cells were grown in a 75 cm² flask containing 15 milliliters of growth medium at 375,000 cell/flask for 24 hours. The medium was changed to a serum starvation medium for 8 hours. Serum starvation medium was collected at the end of incubation, centrifuged at 14,000 x g for 5 minutes (and stored at -20°C).

To estimate the number of cells in each flask, 2 milliliters of trypsin/EDTA (Gibco, Carlsbad, Ca.) were added to each flask. After cells detached from the flask, trypsin activity was neutralized with 8 milliliters of growth medium. Cells were transferred to a 15 milliliter centrifuge tube and centrifuged at 150 x g for 5 minutes. Supernatant was removed and 1 milliliter growth medium was added to each tube to resuspend the cells. Cell number was determined with a hemocytometer.

The amount of IL-8 secreted by the cells into serum starvation medium was analyzed using ELISA assays (R&D Systems, Minneapolis, Mn.). All assays were conducted according to the instructions provided by the manufacturer.

RNA was extracted from confluent umbilical cord-derived cells and fibroblasts, or for IL-8 expression, from cells treated as described above. Cells were lysed with 350 microliters buffer RLT containing beta-mercaptoethanol (Sigma, St. Louis, Mo.) according to the manufacturer's instructions (RNeasy Mini Kit; Qiagen, Valencia, Ca.). RNA was extracted according to the manufacturer's instructions (RNeasy Mini Kit; Qiagen, Valencia, Ca.) and subjected to DNase treatment (2.7 Units/sample) (Sigma St. Louis, Mo.). RNA was eluted with 50 microliters DEPC-treated water and stored at -80°C. RNA was also extracted from human umbilical cord. Tissue (30 milligrams) was suspended in 700 microliters of buffer RLT containing beta-mercaptoethanol. Samples were mechanically homogenized and the RNA extraction proceeded according to manufacturer's specification. RNA was extracted with 50 microliters of DEPC-treated water and stored at -80°C.

RNA was reverse-transcribed using random hexamers with the TaqMan reverse transcription reagents (Applied Biosystems, Foster City, Ca.) at 25°C for 10 minutes, 37°C for 60 minutes, and 95°C for 10 minutes. Samples were stored at -20°C.

Genes identified by cDNA microarray as uniquely regulated in umbilical cord cells (signature genes - including oxidized LDL receptor, interleukin-8, renin, and reticulon), were further investigated using real-time and conventional PCR.

PCR was performed on cDNA samples using gene expression products sold under the tradename Assays-On-Demand (Applied Biosystems) gene expression products. Oxidized LDL receptor (Hs00234028); renin (Hs00166915); reticulon (Hs00382515); CXC ligand 3 (Hs00171061); GCP-2 (Hs00605742); IL-8 (Hs00174103); and GAPDH were mixed with cDNA and TaqMan Universal PCR master mix according to the manufacturer's instructions (Applied Biosystems) using a 7000 sequence detection system with ABI Prism 7000 SDS software (Applied Biosystems). Thermal cycle conditions were initially 50°C for 2 minutes and 95°C for 10 minutes, followed by 40 cycles of 95°C for 15 seconds and 60°C for 1 minute. PCR data were analyzed according to manufacturer's specifications (User Bulletin #2 from Applied Biosystems for ABI Prism 7700 Sequence Detection System).

Conventional PCR was performed using an ABI PRISM 7700 (Perkin Elmer Applied Biosystems, Boston, Ma.) to confirm the results from real-time PCR. PCR was performed using 2 microliters of cDNA solution (1 × Taq polymerase (tradename AMPLITAQ GOLD) universal mix PCR reaction buffer (Applied Biosystems) and initial denaturation at 94°C for 5 minutes. Amplification was optimized for each primer set. For IL-8, CXC ligand 3, and reticulon (94°C for 15 seconds, 55°C for 15 seconds and 72°C for 30 seconds for 30 cycles); for renin (94°C for 15 seconds, 53°C for 15 seconds and 72°C for 30 seconds for 38 cycles); for oxidized LDL receptor and GAPDH (94°C for 15 seconds, 55°C for 15 seconds and 72°C for 30 seconds for 33 cycles). Primers used for amplification are listed in Table 11-1. Primer concentration in the final PCR reaction was 1 micromolar except for GAPDH which was 0.5 micromolar. GAPDH primers were the same as for real-time PCR, except that the manufacturer's TaqMan probe was not added to the final PCR reaction. Samples were separated on 2% (w/v) agarose gel and stained with ethidium bromide (Sigma, St. Louis, Mo.). Images were captured on 667 film (Universal Twinpack, VWR International, South Plainfield, N.J.) using a fixed focal-length POLAROID camera (VWR International, South Plainfield, N.J.).

**Table 11-1: Primers used**

| a. | Primer name | Primers |
|---|---|---|
| **Oxidized LDL receptor** | S: 5'- GAGAAATCCAAAGAGCAAATGG-3 (SEQ ID NO:1) | |
| | A: 5'-AGAATGGAAAACTGGAATAGG -3' (SEQ ID NO:2) | |
| **Renin** | S: 5'-TCTTCGATGCTTCGGATTCC -3' (SEQ ID NO:3) | |
| | A: 5'-GAATTCTCGGAATCTCTGTTG -3' (SEQ ID NO:4) | |
| **Reticulon** | S: 5'- TTACAAGCAGTGCAGAAAACC-3' (SEQ ID NO:5) | |
| | A: 5'- AGTAAACATTGAAACCACAGCC-3' (SEQ ID NO:6) | |
| **Interleukin-8** | S: 5'- TCTGCAGCTCTGTGTGAAGG-3' (SEQ ID NO:7) | |
| | A: 5'-CTTCAAAAACTTCTCCACAACC- 3' (SEQ ID NO:8) | |
| **Chemokine (CXC) ligand 3** | S: 5'- CCCACGCCACGCTCTCC-3' (SEQ ID NO:9) | |
| | A: 5'-TCCTGTCAGTTGGTGCTCC -3' (SEQ ID NO:10) | |

Umbilical cord-derived cells were fixed with cold 4% (w/v) paraformaldehyde (Sigma-Aldrich, St. Louis, Mo.) for 10 minutes at room temperature. One isolate each of umbilical cord-derived cells at passage 0 (P0) (directly after isolation) and passage 11 (P11) (two isolates of Umbilical cord-derived cells) and fibroblasts (P11) were used. Immunocytochemistry was performed using antibodies directed against the following epitopes: vimentin (1:500, Sigma, St. Louis, Mo.), desmin (1:150; Sigma - raised against rabbit; or 1:300; Chemicon, Temecula, Ca. - raised against mouse,), alpha-smooth muscle actin (SMA; 1:400; Sigma), cytokeratin 18 (CK18; 1:400; Sigma), von Willebrand Factor (vWF; 1:200; Sigma), and CD34 (human CD34 Class III; 1:100; DAKOCytomation, Carpinteria, Ca.). In addition, the following markers were tested on passage 11 umbilical cord-derived cells: anti-human GROalpha - PE (1:100; Becton Dickinson, Franklin Lakes, N.J.), anti-human GCP-2 (1:100; Santa Cruz Biotech, Santa Cruz, Ca.), anti-human oxidized LDL receptor 1 (ox-LDL R1; 1:100; Santa Cruz Biotech), and anti-human NOGA-A (1:100; Santa Cruz, Biotech).

Cultures were washed with phosphate-buffered saline (PBS) and exposed to a protein blocking solution containing PBS, 4% (v/v) goat serum (Chemicon, Temecula, Ca.), and 0.3% (v/v) Triton (Triton X-100; Sigma, St. Louis, Mo.) for 30 minutes to access intracellular antigens. Where the epitope of interest was located on the cell surface (CD34, ox-LDL R1), Triton X-100 was omitted in all steps of the procedure in order to prevent epitope loss. Furthermore, in instances where the primary antibody was raised against goat (GCP-2, ox-LDL R1, NOGO-A), 3% (v/v) donkey serum was used in place of goat serum throughout the process. Primary antibodies, diluted in blocking solution, were then applied to the cultures for a period of 1 hour at room temperature. The primary antibody solutions were removed and the cultures were washed with PBS prior to application of secondary antibody solutions (1 hour at room temperature) containing block along with goat anti-mouse IgG - Texas Red (1:250; Molecular Probes, Eugene, Or.) and/or goat anti-rabbit IgG - Alexa 488 (1:250; Molecular Probes) or donkey anti-goat IgG - FITC (1:150, Santa Cruz Biotech). Cultures were then washed and 10 micromolar DAPI (Molecular Probes) applied for 10 minutes to visualize cell nuclei.

Following immunostaining, fluorescence was visualized using an appropriate fluorescence filter on an Olympus inverted epi-fluorescent microscope (Olympus, Melville, N.Y.). In all cases, positive staining represented fluorescence signal above control staining where the entire procedure outlined above was followed with the exception of application of a primary antibody solution (no 1° control). Representative images were captured using a digital color videocamera and ImagePro software (Media Cybernetics, Carlsbad, Ca.). For triple-stained samples, each image was taken using only one emission filter at a time. Layered montages were then prepared using Adobe Photoshop software (Adobe, San Jose, Ca.).

Adherent cells in flasks were washed in phosphate buffered saline (PBS) (Gibco, Carlsbad, Ca.) and detached with Trypsin/EDTA (Gibco, Carlsbad, Ca.). Cells were harvested, centrifuged, and re-suspended 3% (v/v) FBS in PBS at a cell concentration of 1x10⁷/milliliter. One hundred microliter aliquots were delivered to conical tubes. Cells stained for intracellular antigens were permeabilized with Perm/Wash buffer (BD Pharmingen, San Diego, Ca.). Antibody was added to aliquots as per manufacturer's specifications, and the cells were incubated for in the dark for 30 minutes at 4°C. After incubation, cells were washed with PBS and centrifuged to remove excess antibody. Cells requiring a secondary antibody were resuspended in 100 microliter of 3% FBS. Secondary antibody was added as per manufacturer's specification, and the cells were incubated in the dark for 30 minutes at 4°C. After incubation, cells were washed with PBS and centrifuged to remove excess secondary antibody. Washed cells were resuspended in 0.5 milliliter PBS and analyzed by flow cytometry. The following antibodies were used: oxidized LDL receptor 1 (sc-5813; Santa Cruz, Biotech), GROa (555042; BD Pharmingen, Bedford, Ma.), Mouse IgG1 kappa, (P-4685 and M-5284; Sigma), and Donkey against Goat IgG (sc-3743; Santa Cruz, Biotech.). Flow cytometry analysis was performed with FACScalibur (Becton Dickinson San Jose, Ca.).

Results of real-time PCR for selected "signature" genes performed on cDNA from cells derived from human umbilical cord, adult and neonatal fibroblasts, and Mesenchymal Stem Cells (MSCs) indicate that both reticulon and oxidized LDL receptor expression were higher in umbilicus-derived cells as compared to other cells. The data obtained from real-time PCR were analyzed by the ΔΔCT method and expressed on a logarithmic scale. No significant differences in the expression levels of CXC ligand 3 and GCP-2 were found between postpartum cells and controls. The results of real-time PCR were confirmed by conventional PCR. Sequencing of PCR products further validated these observations. No significant difference in the expression level of CXC ligand 3 was found between postpartum cells and controls using conventional PCR CXC ligand 3 primers listed in Table 11-1.

The expression of the cytokine, IL-8 in umbilical cord-derived cells was elevated in both growth medium-cultured and serum-starved umbilical cord-derived cells. All real-time PCR data were validated with conventional PCR and by sequencing PCR products.

After growth in scrum-free media, the conditioned media were examined for the presence of IL-8. The greatest amounts of IL-8 were detected in media in which umbilical cells had been grown (Table 11-2). No IL-8 was detected in medium in which human dermal fibroblasts had been grown.

**Table 11-2: IL-8 protein expression measured by ELISA**

| **Cell type** | **IL-8** |
|---|---|
| Human fibroblasts | ND |
| Placenta Isolate 1 | ND |
| UMBC Isolate 1 | 2058.42±144.67 |
| Placenta Isolate 2 | ND |
| UMBC Isolate 2 | 2368.86±22.73 |
| Placenta Isolate3 (normal O₂) | 17.27±8.63 |
| Placenta Isolate 3 (lowO₂, W/O BME) | 264.92±9.88 |
| **Results of the ELISA assay for interleukin-8 (IL-8) performed on placenta-and umbilical cord-derived cells as well as human skin fibroblasts. Values are presented here are picogram/million cells, n=2, sem.** ND: Not Detected | |

Cells derived from the human umbilical cord at passage 0 were probed for the production of selected proteins by immunocytochemical analysis. Immediately after isolation (passage 0), cells were fixed with 4% paraformaldehyde and exposed to antibodies for six proteins: von Willebrand Factor, CD34, cytokeratin 18, desmin, alpha-smooth muscle actin, and vimentin. Umbilical cord-derived cells were positive for alpha-smooth muscle actin and vimentin, with the staining pattern consistent through passage 11.

The production of GROalpha, GCP-2, oxidized LDL receptor 1 and reticulon (NOGO-A) in umbilical cord-derived cells at passage 11 was investigated by immunocytochemistry. Umbilical cord-derived cells were GCP-2 positive, but GRO alpha production was not detected by this method. Furthermore, cells were NOGO-A positive.

Accordance between gene expression levels measured by microarray and PCR (both real-time and conventional) has been established for four genes: oxidized LDL receptor 1, renin, reticulon, and IL-8. The expression of these genes was differentially regulated at the mRNA level in umbilical cord-derived cells, with IL-8 also differentially regulated at the protein level. Differential expression of GCP-2 and CXC ligand 3 was not confirmed at the mRNA level. Although this result does not support data originally obtained from the microarray experiment, this may be due to a difference in the sensitivity of the methodologies.

Cells derived from the human umbilical cord at passage 0 were probed for the expression of alpha-smooth muscle actin and vimentin, and were positive for both. The staining pattern was preserved through passage 11.

In conclusion, the complete mRNA data at least partially verifies the data obtained from the microarray experiments.

### EXAMPLE 12

### Immunohistochemical Characterization of Cellular Phenotypes

The phenotypes of cells found within human umbilical cord were analyzed by immunohistochemistry.

Human umbilical cord tissue was harvested and immersion fixed in 4% (w/v) paraformaldehyde overnight at 4°C. Immunohistochemistry was performed using antibodies directed against the following epitopes (see Table 12-1): vimentin (1:500; Sigma, St. Louis, Mo.), desmin (1:150, raised against rabbit; Sigma; or 1:300, raised against mouse; Chemicon, Temecula, Ca.), alpha-smooth muscle actin (SMA; 1:400; Sigma), cytokeratin 18 (CK18; 1:400; Sigma), von Willebrand Factor (vWF; 1:200; Sigma), and CD34 (human CD34 Class III; 1:100; DAKOCytomation, Carpinteria, Ca.). In addition, the following markers were tested: anti-human GROalpha-PE (1:100; Becton Dickinson, Franklin Lakes, N.J.), anti-human GCP-2 (1:100; Santa Cruz Biotech, Santa Cruz, Ca.), anti-human oxidized LDL receptor 1 (ox-LDL R1; 1:100; Santa Cruz Biotech), and anti-human NOGO-A (1:100; Santa Cruz Biotech). Fixed specimens were trimmed with a scalpel and placed within OCT embedding compound (Tissue-Tek OCT; Sakura, Torrance, Ca.) on a dry ice bath containing ethanol. Frozen blocks were then sectioned (10 microns thick) using a standard cryostat (Leica Microsystems) and mounted onto glass slides for staining.

Immunohistochemistry was performed similar to previous studies *(e.g.,* Messina, et al. Exper. Neurol., 2003; 184: 816-829). Tissue sections were washed with phosphate-buffered saline (PBS) and exposed to a protein blocking solution containing PBS, 4% (v/v) goat serum (Chemicon, Temecula, Ca.), and 0.3% (v/v) Triton (Triton X-100; Sigma) for 1 hour to access intracellular antigens. In instances where the epitope of interest would be located on the cell surface (CD34, ox-LDL R1), triton was omitted in all steps of the procedure in order to prevent epitope loss. Furthermore, in instances where the primary antibody was raised against goat (GCP-2, ox-LDL R1, NOGO-A), 3% (v/v) donkey serum was used in place of goat serum throughout the procedure. Primary antibodies, diluted in blocking solution, were then applied to the sections for a period of 4 hours at room temperature. Primary antibody solutions were removed, and cultures washed with PBS prior to application of secondary antibody solutions (1 hour at room temperature) containing block along with goat anti-mouse IgG-Texas Red (1:250; Molecular Probes, Eugene, Or.) and/or goat anti-rabbit IgG-Alexa 488 (1:250; Molecular Probes) or donkey anti-goat IgG-FITC (1:150; Santa Cruz Biotech). Cultures were washed, and 10 micromolar DAPI (Molecular Probes) was applied for 10 minutes to visualize cell nuclei.

Following immunostaining, fluorescence was visualized using the appropriate fluorescence filter on an Olympus inverted epifluorescent microscope (Olympus, Melville, N.Y.). Positive staining was represented by fluorescence signal above control staining. Representative images were captured using a digital color videocamera and ImagePro software (Media Cybernetics, Carlsbad, Ca.). For triple-stained samples, each image was taken using only one emission filter at a time. Layered montages were then prepared using Adobe Photoshop software (Adobe, San Jose, Ca.).

**Table 12-1: Summary of Primary Antibodies Used**

| **Antibody** | **Concentration** | **Vendor** |
|---|---|---|
| Vimentin | 1:500 | Sigma, St. Louis, MO |
| Desmin (rb) | 1:150 | Sigma |
| Desmin (m) | 1:300 | Chemicon, Temecula, CA |
| alpha-smooth muscle actin (SMA) | 1:400 | Sigma |
| Cytokeratin 18 (CK18) | 1:400 | Sigma |
| von Willebrand factor (vWF) | 1:200 | Sigma |
| CD34 III | 1:100 | DakoCytomation, Carpinteria, CA |
| GROalpha-PE | 1:100 | BD, Franklin Lakes, NJ |
| GCP-2 | 1:100 | Santa Cruz Biotech |
| Ox-LDL R1 | 1:100 | Santa Cruz Biotech |
| NOGO-A | 1:100 | Santa Cruz Biotech |

Vimentin, desmin, SMA, CK18, vWF, and CD34 markers were expressed in a subset of the cells found within umbilical cord (data not shown). In particular, vWF and CD34 expression were restricted to blood vessels contained within the cord. CD34+ cells were on the innermost layer (lumen side). Vimentin expression was found throughout the matrix and blood vessels of the cord. SMA was limited to the matrix and outer walls of the artery and vein, but not contained within the vessels themselves. CK18 and desmin were observed within the vessels only, desmin being restricted to the middle and outer layers.

None of these markers were observed within umbilical cord (data not shown).

Vimentin, desmin, alpha-smooth muscle actin, cytokeratin 18, von Willebrand Factor, and CD 34 are expressed in cells within human umbilical cord. Based on *in vitro* characterization studies showing that only vimentin and alpha-smooth muscle actin are expressed, the data suggests that the current process of umbilical cord-derived cell isolation harvests a subpopulation of cells or that the cells isolated change expression of markers to express vimentin and alpha-smooth muscle actin.

### EXAMPLE 13

### Secretion of Trophic Factors bv Cells

The secretion of selected trophic factors from umbilicus-derived cells was measured. Factors were selected that have angiogenic activity such as hepatocyte growth factor (HGF) (Rosen et al,. Ciba Found. Symp., 1997; 212:215-26), monocyte chemotactic protein 1 (MCP-1) (Salcedo et al., Blood, 2000; 96;34-40), interleukin-8 (IL-8) (Li et al., J. Immunol., 2003; 170:3369-76), keratinocyte growth factor (KGF), basic fibroblast growth factor (bFGF), vascular endothelial growth factor (VEGF) (Hughes et al., Ann. Thonac. Surg., 2004; 77:812-8), tissue inhibitor of matrix metalloproteinase 1 (TIMP1), angiopoietin 2 (ANG2), platelet derived growth factor (PDGFbb), thrombopoietin (TPO), heparin-binding epidermal growth factor (HB-EGF), stromal-derived factor 1alpha (SDF-1alpha)), neurotrophic/neuroprotective activity (brain-derived neurotrophic factor (BDNF) (Cheng et al., Dev. Biol., 2003; 258;319-33), interleukin-6 (IL-6), granulocyte chemotactic protein-2 (GCP-2), transforming growth factor beta2 (TGFbeta2)), or chemokine activity (macrophage inflammatory protein 1 alpha (MIP1alpha), macrophage inflammatory protein 1 beta (MIP1beta), monocyte chemoattractant-1 (MCP-1), Rantes (regulated on activation, normal T cell expressed and secreted), 1309, thymus and activation-regulated chemokine (TARC), Eotaxin, macrophage-derived chemokine (MDC), and IL-8.

Cells derived from umbilical cord, as well as human fibroblasts derived from human neonatal foreskin, were cultured in growth medium on gelatin-coated T75 flasks. Cells were cryopreserved at passage 11 and stored in liquid nitrogen. After thawing, growth medium was added to the cells, followed by transfer to a 15 milliliter centrifuge tube and centrifugation of the cells at 150 x g for 5 minutes. The cell pellet was resuspended in 4 milliliters growth medium, and cells were counted. Cells were seeded at 5,000 cells/cm² in T75 flasks each containing 15 milliliters of growth medium, and cultured for 24 hours. The medium was changed to a serum-free medium (Low glucose (Gibco), 0.1% (w/v) bovine serum albumin

(Sigma), penicillin (50 Units/milliliter) and streptomycin (50 micrograms/milliliter, Gibco)) for 8 hours. Conditioned serum-free medium was collected at the end of incubation by centrifugation at 14,000 x g for 5 minutes and stored at -20°C.

To estimate the number of cells in each flask, cells were washed with phosphate-buffered saline (PBS) and detached using 2 milliliters trypsin/EDTA (Gibco). Trypsin activity was inhibited by addition of 8 milliliters growth medium. Cells were centrifuged at 150 x g for 5 minutes. The supernatant was removed, and cells were resuspended in 1 milliliter growth medium. Cell number was estimated with a hemocytometer.

Cells were grown at 37°C in 5% carbon dioxide and atmospheric oxygen. The amount of MCP-1, IL-6, VEGF, SDF-1alpha, GCP-2 , IL-8, and TGF-beta2 produced by each cell sample was determined by ELISA (R&D Systems, Minneapolis, Mn.. All assays were performed according to the manufacturer's instructions. Values presented are picograms per milliliter per million cells (n=2, sem).

Chemokines (MIP1alpha, MIP1beta, MCP-1, Rantes, 1309, TARC, Eotaxin, MDC, IL8), BDNF, and angiogenic factors (HGF, KGF, bFGF, VEGF, TIMP1, ANG2, PDGFbb, TPO, HB-EGF were measured using SearchLight Proteome Arrays (Pierce Biotechnology Inc.). The Proteome Arrays are multiplexed sandwich ELISAs for the quantitative measurement of two to sixteen proteins per well. The arrays are produced by spotting a 2 x 2, 3 x 3, or 4 x 4 pattern of four to sixteen different capture antibodies into each well of a 96-well plate. Following a sandwich ELISA procedure, the entire plate is imaged to capture the chemiluminescent signal generated at each spot within each well of the plate. The signal generated at each spot is proportional to the amount of target protein in the original standard or sample.

MCP-1 and IL-6 were secreted by umbilicus-derived cells and dermal fibroblasts (Table 13-1). SDF-1alpha and GCP-2 were secreted by fibroblasts. GCP-2 and IL-8 were secreted by umbilicus-derived cells. TGF-beta2 was not detected from either cell type by ELISA.

**Table 13-1. ELISA Results: Detection of Trophic Factors**

| | **MCP-1** | **IL-6** | **VEGF** | **SDF-1α** | **GCP-2** | **IL-8** | **TGF**-**beta2** |
|---|---|---|---|---|---|---|---|
| **Fibroblast** | 17±1 | 61±3 | 29±2 | 19±1 | 21±1 | ND | ND |
| **Umbilical (022803)** | 1150±74 | 4234±289 | ND | ND | 160±11 | 2058±145 | ND |
| **Umbilical (071003)** | 2794±84 | 1356±43 | ND | ND | 2184±98 | 2369±23 | ND |

| | | | | | | | |
|---|---|---|---|---|---|---|---|
| Key: ND: Not Detected., =/- sem | | | | | | | |

**SearchLight Multiplexed ELISA assay.** TIMP1, TPO, KGF, HGF, FGF, HBEGF, BDNF, MIP1beta, MCP1, RANTES, 1309, TARC, MDC, and IL-8 were secreted from umbilicus-derived PPDCs (Tables 13-2 and 13-3). No Ang2, VEGF, or PDGFbb were detected.

**Table 13-2. SearchLight Multiplexed ELISA assay results**

| | **TIMP1** | **ANG2** | **PDGFbb** | **TPO** | **KGF** | **HGF** | **FGF** | **VEGF** | **HBEGF** | **BDNF** |
|---|---|---|---|---|---|---|---|---|---|---|
| **hFB** | 19306.3 | ND | ND | 230.5 | 5.0 | ND | ND | 27.9 | 1.3 | ND |
| **U1** | 57718.4 | ND | ND | 1240.0 | 5.8 | 559.3 | 148.7 | ND | 9.3 | 165.7 |
| **U3** | 21850.0 | ND | ND | 1134.5 | 9.0 | 195.6 | 30.8 | ND | 5.4 | 388.6 |

| | | | | | | | | | | |
|---|---|---|---|---|---|---|---|---|---|---|
| Key: hFB (human fibroblasts), U1 (umbilicus-derived PPDC (022803)), U3 (umbilicus-derived PPDC (071003)). ND: Not Detected. | | | | | | | | | | |

**Table 13-3. SearchLight Multiplexed ELISA assay results**

| | **MIP1a** | **MIP1b** | **MCP1** | **RANTES** | **1309** | **TARC** | **Eotaxin** | **MDC** | **IL8** |
|---|---|---|---|---|---|---|---|---|---|
| **hFB** | ND | ND | 39.6 | ND | ND | 0.1 | ND | ND | 204.9 |
| **U1** | ND | 8.0 | 1694.2 | ND | 22.4 | 37.6 | ND | 18.9 | 51930.1 |
| **U3** | ND | 5.2 | 2018.7 | 41.5 | 11.6 | 21.4 | ND | 4.8 | 10515.9 |

| | | | | | | | | | |
|---|---|---|---|---|---|---|---|---|---|
| Key: hFB (human fibroblast), U1 (umbilicus-derived PPDC (022803)), U3 (umbilicus-derived PPDC (071003)). ND: Not Detected. | | | | | | | | | |

Umbilicus-derived cells secreted a number of trophic factors. Some of these trophic factors, such as HGF, bFGF, MCP-1 and IL-8, play important roles in angiogenesis. Other trophic factors, such as BDNF and IL-6, have important roles in neural regeneration or protection.

### EXAMPLE 14

### In Vitro Immunology

Umbilical cord cell lines were evaluated *in vitro* for their immunological characteristics in an effort to predict the immunological response, if any, these cells would elicit upon *in vivo* transplantation. Umbilical cord cell lines were assayed by flow cytometry for the expression of HLA-DR, HLA-DP, HLA-DQ, CD80, CD86, and B7-H2. These proteins are expressed by antigen-presenting cells (APC) and are required for the direct stimulation of naïve CD4⁺ T cells (Abbas & Lichtman, Cellular and Molecular Immunology, 5th Ed. (2003) Saunders, Philadelphia, p. 171). The cell lines were also analyzed by flow cytometry for the expression of HLA-G (Abbas & Lichtman, Cellular and Molecular Immunology, 5th Ed. (2003) Saunders, Philadelphia, p. 171), CD178 (Coumans, et.al., Journal of Immunological Methods, 1999; 224, 185-196), and PD-L2 (Abbas & Lichtman, Cellular and Molecular Immunology, 5th Ed. (2003) Saunders, Philadelphia, p. 171; Brown, et. al. The Journal of Immunology, 2003; 170, 1257-1266). The expression of these proteins by cells residing in placental tissues is thought to mediate the immuno-privileged status of placental tissues *in utero.* To predict the extent to which postpartum umbilicus-derived cell lines elicit an immune response *in vivo,* the cell lines were tested in a one-way mixed lymphocyte reaction (MLR).

Cells were cultured in growth medium (DMEM-low glucose (Gibco, Carlsbad, Ca.), 15% (v/v) fetal bovine serum (FBS); (Hyclone, Logan, Ut.), 0.001% (v/v) betamercaptoethanol (Sigma, St. Louis, Mo.), 50 Units/milliliter penicillin, 50 micrograms/milliliter streptomycin (Gibco, Carlsbad, Ca.) until confluent in T75 flasks (Corning, Corning, N.Y.) coated with 2% gelatin (Sigma, St. Louis, Mo.).

Cells were washed in phosphate buffered saline (PBS) (Gibco, Carlsbad, Ca.) and detached with Trypsin/EDTA (Gibco, Carlsbad, Ca.). Cells were harvested, centrifuged, and re-suspended in 3% (v/v) FBS in PBS at a cell concentration of 1x10⁷ per milliliter. Antibody (Table 14-1) was added to one hundred microliters of cell suspension as per manufacture's specifications and incubated in the dark for 30 minutes at 4°C. After incubation, cells were washed with PBS and centrifuged to remove unbound antibody. Cells were re-suspended in five hundred microliters of PBS and analyzed by flow cytometry using a FACS calibur instrument (Becton Dickinson, San Jose, Ca.).

**Table 14-1. Antibodies**

| **Antibody** | **Manufacturer** | **Catalog Number** |
|---|---|---|
| HLA-DRDPDQ | BD Pharmingen (San Diego, Ca.) | 555558 |
| CD80 | BD Pharmingen (San Diego, Ca.) | 557227 |
| CD86 | BD Pharmingen (San Diego, Ca.) | 555665 |
| B7-H2 | BD Pharmingen (San Diego, Ca.) | 552502 |
| HLA-G | Abcam (Cambridgeshire, UK) | ab 7904-100 |
| CD 178 | Santa Cruz (San Cruz, Ca.) | sc-19681 |
| PD-L2 | BD Pharmingen (San Diego, Ca.) | 557846 |
| Mouse IgG2a | Sigma (St. Louis, Mo.) | F-6522 |
| Mouse IgG 1 kappa | Sigma (St. Louis, Mo.) | P-4685 |

Cryopreserved vials of passage 10 umbilical cord-derived cells labeled as cell line A were paclaged on dry ice and sent to CTBR (Senneville, Quebec) to conduct a mixed lymphocyte reaction using CTBR SOP no. CAC-031. Peripheral blood mononuclear cells (PBMCs) were collected from multiple male and female volunteer donors. Stimulator (donor) allogeneic PBMC, autologous PBMC, and umbilical cord tissue-derived cell lines were treated with mitomycin C. Autologous and mitomycin C-treated stimulator cells were added to responder (recipient) PBMCs and cultured for 4 days. After incubation, [³H]thymidine was added to each sample and cultured for 18 hours. Following harvest of the cells, radiolabeled DNA was extracted, and [³H]-thymidine incorporation was measured using a scintillation counter.

The stimulation index for the allogeneic donor (SIAD) was calculated as the mean proliferation of the receiver plus mitomycin C-treated allogeneic donor divided by the baseline proliferation of the receiver. The stimulation index of the umbilical cord-derived cells was calculated as the mean proliferation of the receiver plus mitomycin C-treated umbilical cord tissue-derived cell line divided by the baseline proliferation of the receiver.

Six human volunteer blood donors were screened to identify a single allogeneic donor that will exhibit a robust proliferation response in a mixed lymphocyte reaction with the other five blood donors. This donor was selected as the allogeneic positive control donor. The remaining five blood donors were selected as recipients. The allogeneic positive control donor and umbilical cord-derived cell lines were mitomycin C-treated and cultured in a mixed lymphocyte reaction with the five individual allogeneic receivers. Reactions were performed in triplicate using two cell culture plates with three receivers per plate (Table 14-2). The average stimulation index ranged from 6.5 (plate 1) to 9 (plate 2) and the allogeneic donor positive controls ranged from 42.75 (plate 1) to 70 (plate 2) (Table 14-3).

**Table 14-2. Mixed Lymphocyte Reaction Data- Cell Line A (Umbilical Cord)**

| DPM for Proliferation Assay | | | | | | | | | |
|---|---|---|---|---|---|---|---|---|---|
| Plate ID: Plate1 | | | | | | | | | |
| Analytical number | Culture | | Replicates | | | | | | |
| | System | | 1 | 2 | 3 | | Mean | SD | CV |
| IM04-2478 | Proliferation baseline of receiver | | 1074 | 406 | 391 | | 623.7 | 390.07 | 62.5 |
| | Control of autostimulation (Mitomycin C treated autologous cells) | | 672 | 510 | 1402 | | 861.3 | 475.19 | 55.2 |
| | MLR allogenic donor IM04-2477 (Mitomycin C treated) | | 43777 | 48391 | 38231 | | 43466.3 | 5087.12 | 11.7 |
| | MLR with cell line (Mitomycin C treated cell type A) | | 2914 | 5622 | 6109 | | 4881.7 | 1721.36 | 35.3 |
| SI (donor) | | | | | | | 70 | | |
| SI (cell line) | | | | | | | 8 | | |
| IM04-2479 | Proliferation baseline of receiver | | 530 | 508 | 527 | | 521.7 | 11.93 | 2.3 |
| | Control of autostimulation (Mitomycin C treated autologous cells) | | 701 | 567 | 1111 | | 793.0 | 283.43 | 35.7 |
| | MLR allogenic donor IM04-2477 (Mitomydn C treated) | | 25593 | 24732 | 22707 | | 24344.0 | 1481.61 | 6.1 |
| | MLR with cell line (Mitomycin C treated cell type A) | | 5086 | 3932 | 1497 | | 3505.0 | 1832.21 | 52.3 |
| SI (donor) | | | | | | | 47 | | |
| SI (cell line) | | | | | | | 7 | | |
| IM04-2480 | Proliferation baseline of receiver | | 1192 | 854 | 1330 | | 1125.3 | 244.90 | 21.8 |
| | Control of autostimulation (Mitomycin C treated autologous cells) | | 2963 | 993 | 2197 | | 2051.0 | 993.08 | 48.4 |
| | MLR allogenic donor IM04-2477 (Mitomycin C treated) | | 25416 | 29721 | 23757 | | 26298.0 | 3078.27 | 11.7 |
| | MLR with cell line (Mitomycin C treated cell type A) | | 2596 | 5076 | 3426 | | 3699.3 | 1262.39 | 34.1 |
| SI (donor) | | | | | | | 23 | | |
| SI (cell line) | | | | | | | 3 | | |
| IM04-2481 | Proliferation baseline of receiver | | 695 | 451 | 555 | | 567.0 | 122.44 | 21.6 |
| | Control of autostimulation (Mitomycin C treated autologous cells) | | 738 | 1252 | 464 | | 818.0 | 400.04 | 48.9 |
| | MLR allogenic donor IM04-2477 (Mitomycin C treated) | | 13177 | 24885 | 15444 | | 17835.3 | 6209.52 | 34.8 |
| | MLR with cell line (Mitomycin C treated cell type A) MLR cell (Mitomycin C treated A) | | 4495 | 3671 | 4674 | | 4280.0 | 534.95 | 12.5 |
| SI (donor) | | | | | | | 31 | | |
| SI (cell line) | | | | | | | 8 | | |
| | | | | | | | | | |
| | | | | | | | | | |

| Plate ID: Plate 2 | | | | | | | | | |
|---|---|---|---|---|---|---|---|---|---|
| Analytical number | Culture | | Replicates | | | | | | |
| | System | | 1 | 2 | 3 | | Mean | SD | CV |
| IM04-2482 | Proliferation baseline of receiver | | 432 | 533 | 274 | | 413.0 | 130.54 | 31.6 |
| | Control of autostimulation (Mitomycin C treated autologous cells) | | 1459 | 633 | 598 | | 896.7 | 487.31 | 54.3 |
| | MLR allogenic donor IM04-2477 (Mitomycin C treated) | | 24286 | 30823 | 31346 | | 28818.3 | 3933.82 | 13.7 |
| | MLR with cell line (Mitomycin C treated cell type A) | | 2762 | 1502 | 6723 | | 3662.3 | 2724.46 | 74.4 |
| SI (donor) | | | | | | | 70 | | |
| SI (cell line) | | | | | | | 9 | | |
| IM04-2477 (allogenic donor) | Proliferation baseline of receiver | | 312 | 419 | 349 | | 360.0 | 54.34 | 15.1 |
| | Control of autostimulation (Mitomycin treated autologous cells) | | 567 | 604 | 374 | | 515.0 | 123.50 | 24.0 |
| | | | | | | | | | |
| Cell line type A | Proliferation baseline of receiver | | 5101 | 3735 | 2973 | | 3936.3 | 1078.19 | 27.4 |
| | Control of autostimulation (Mitomycin treated autologous cells) | | 1924 | 4570 | 2153 | | 2882.3 | 1466.04 | 50.9 |

**Table 14-3. Average stimulation index of umbilical cord-derived cells and an allogeneic donor in a mixed lymphocyte reaction with five individual allogeneic receivers.**

| | Recipient | Umbilical Cord |
|---|---|---|
| Plate 1 (receivers 1-4) | 42.75 | 6.5 |
| Plate 2 (receiver 5) | 70 | 9 |

Histograms of umbilical cord-derived cells analyzed by flow cytometry show negative expression of HLA-DR, DP, DQ, CD80, CD86, and B7-H2, as noted by fluorescence value consistent with the IgG control, indicating that umbilical cord-derived cell lines lack the cell surface molecules required to directly stimulate allogeneic PBMCs (e.g., CD4⁺ T cells).

Histograms of umbilical cord-derived cells analyzed by flow cytometry show positive expression of PD-L2, as noted by the increased value of fluorescence relative to the IgG control, and negative expression of CD 178 and HLA-G, as noted by fluorescence value consistent with the IgG control.

In the mixed lymphocyte reactions conducted with umbilical cord-derived cell lines, the average stimulation index ranged from 6.5 to 9, and that of the allogeneic positive controls ranged from 42.75 to 70. Umbilical cord-derived cell lines were negative for the expression of the stimulating proteins HLA-DR, HLA-DP, HLA-DQ, CD80, CD86, and B7-H2, as measured by flow cytometry. Umbilical cord-derived cell lines were negative for the expression of immuno-modulating proteins HLA-G and CD 178 and positive for the expression of PD-L2, as measured by flow cytometry. Allogeneic donor PBMCs contained antigen-presenting cells expressing HLA-DP, DR, DQ, CD80, CD86, and B7-H2, thereby allowing for the stimulation of allogeneic PBMCs (e.g., naïve CD4⁺ T cells). The absence of antigen-presenting cell surface molecules on umbilical cord-derived cells required for the direct stimulation of allogeneic PBMCs (e.g., naïve CD4⁺ T cells) and the presence of PD-L2, an immuno-modulating protein, may account for the low stimulation index exhibited by these cells in a MLR as compared to allogeneic controls.

### EXAMPLE 15

Telomerase functions to synthesize telomere repeats that serve to protect the integrity of chromosomes and to prolong the replicative life span of cells (Liu, K, et al., PNAS, 1999: 96:5147-5152). Telomerase consists of two components, telomerase RNA template

(hTER) and telomerase reverse transcriptase (hTERT). Regulation of telomerase is determined by transcription of hTERT but not hTER. Real-time polymerase chain reaction (PCR) for hTERT mRNA thus is an accepted method for determining telomerase activity of cells.

### Cell Isolation

Real-time PCR experiments were performed to determine telomerase production of human umbilical cord tissue-derived cells. Human umbilical cord tissue-derived cells were prepared in accordance with Examples 4 - 14 and the examples set forth in U.S. Application Serial No. 10/877,012 (the '012 application), which issued as US Patent No. 7, 510, 873. Generally, umbilical cords obtained from National Disease Research Interchange (Philadelphia, Pa.) following a normal delivery were washed to remove blood and debris and mechanically dissociated. The tissue was then incubated with digestion enzymes including collagenase, dispase and hyaluronidase in culture medium at 37°C. Human umbilical cord tissue-derived cells were cultured according to the methods set forth in the examples of the '012 application. Mesenchymal stem cells and normal dermal skin fibroblasts (cc-2509 lot # 9F0844) were obtained from Cambrex, Walkersville, Md. A pluripotent human testicular embryonal carcinoma (teratoma) cell line nTera-2 cells (NTERA-2 cl.D1), (*see,* Plaia et al., Stem Cells, 2006; 24(3):531-546) was purchased from ATCC (Manassas, Va.) and was cultured according to the methods set forth in the '012 application.

### Total RNA Isolation

RNA was extracted from the cells using (RNeasy® kit (Qiagen, Valencia, Ca.). RNA was eluted with 50 microliters DEPC-treated water and stored at -80°C. RNA was reverse transcribed using random hexamers with the TaqMan® reverse transcription reagents (Applied Biosystems, Foster City, Ca.) at 25°C for 10 minutes, 37°C for 60 minutes and 95°C for 10 minutes. Samples were stored at -20°C.

### Real-time PCR

PCR was performed on cDNA samples using the Applied Biosystems Assays-On-Demand™ (also known as Tasman® Gene Expression Assays) according to the manufacturer's specifications (Applied Biosystems). This commercial kit is widely used to assay for telomerase in human cells. Briefly, hTERT (human telomerase gene) (Hs00162669) and human GAPDH (an internal control) were mixed with cDNA and TaqMan® Universal PCR master mix using a 7000 sequence detection system with ABI prism 7000 SDS software (Applied Biosystems). Thermal cycle conditions were initially 50°C for 2 min and 95°C for 10 min followed by 40 cycles of 95°C for 15 sec and 60°C for 1 min. PCR data was analyzed according to the manufacturer's specifications.

Human umbilical cord tissue-derived cells (ATCC Accession No. PTA-6067), fibroblasts, and mesenchymal stem cells were assayed for hTERT and 18S RNA. As shown in Table 15-1, hTERT, and hence telomerase, was not detected in human umbilical cord tissue-derived cells.

**Table 15-1**

| | hTERT | 18S RNA |
|---|---|---|
| Umbilical cells (022803) | ND | + |
| Fibroblasts | ND | + |

| | | |
|---|---|---|
| ND- not detected; + signal detected | | |

Human umbilical cord tissue-derived cells (isolate 022803, ATCC Accession No. PTA-6067) and nTera-2 cells were assayed and the results showed no expression of the telomerase in two lots of human umbilical cord tissue-derived cells while the teratoma cell line revealed high level of expression (Table 15-2).

**Table 15-2**

| Cell type | hTERT | | GAPDH | | |
|---|---|---|---|---|---|
| | Exp.1 | Exp.2 | Exp.1 | Exp.2 | hTERT norm |
| nTera2 | 22.85 | 27.31 | 16.41 | 16.31 | .61 |
| 022803 | - | - | 22.97 | 22.79 | - |

Therefore, it can be concluded that human umbilical tissue-derived cells do not express telomerase.

## Claims

1. A composition comprising a population of isolated umbilical cord tissue-derived cells for use in treating a subject having neuropathic pain, neural spasticity or chronic pain, wherein said cells are derived from human umbilical cord tissue substantially free of blood, are capable of self-renewal and expansion in culture, are negative for CD117 and do not express hTERT or telomerase, wherein the treating comprises administering the population of isolated umbilical cord tissue-derived cells systemically.

2. The composition for use according to claim 1, wherein the treating comprises administering the population of isolated umbilical cord tissue-derived cells intravenously, interperitoneally, intraarterial, or via syringes with needles or catheters with or without pump devices.

3. The composition for use according to claim 1 or claim 2, wherein the composition comprises pharmaceutically acceptable carriers and/or diluents selected from the group consisting of: saline, aqueous buffer solutions, solvents, dispersion media composition and a mix thereof.

4. The composition for use according to any one of claim 1 to 3, wherein the composition further comprises one or more hydrogels which are selected from the group consisting of: collagen, atelocollagen, fibrin, thrombin-fibrin, and a mix thereof.

5. The composition for use according to any one of claims 1 to 4, wherein the population of isolated umbilical cord tissue-derived cells comprises cells that are genetically modified to produce therapeutically useful gene products or to produce agents to facilitate or support neural tissue formation or growth.

6. The composition for use according to any one of claims 1 to 5, said composition further comprising a population of cells derived from one or more of the following tissues: dermal tissue, vascular tissue, connective tissue, cartilage, adipose tissue, muscle tissue, tendons or ligaments.

7. The composition for use according to any one of claims 1 to 6, wherein the isolated umbilical cord tissue-derived cells further comprise the following characteristics:
the potential for at least about 40 doublings in culture;
production of CD 10, CD 13, CD44, CD73, and CD90;
expression of a gene, which relative to a human cell that is a fibroblast, a mesenchymal stem cell, or an iliac crest bone marrow cell, is increased for a gene encoding interleukin 8 and reticulon 1; and
lack of production of CD45.

## Patentansprüche

1. Zusammensetzung, umfassend eine Population isolierter, von Nabelschnurgewebe stammenden Zellen zur Verwendung bei der Behandlung eines Patienten mit neuropathischen Schmerzen, neuraler Spastizität oder chronischen Schmerzen, wobei die Zellen von im Wesentlichen blutfreien Nabelschnurgewebe stammen, zur Selbsterneuerung und Expansion in Kultur fähig sind, CD117-negativ sind und hTERT oder Telomerase nicht exprimieren, wobei die Behandlung die systematische Verabreichung der Population isolierter, von Nabelschnurgewebe stammenden Zellen umfasst.

2. Zusammensetzung zur Verwendung nach Anspruch 1, wobei die Behandlung die intravenöse, interperitoneale oder intraarterielle Verabreichung der Population isolierter, von Nabelschnurgewebe stammenden Zellen oder deren Verabreichung mittels Spritzen mit Nadeln oder Kathetern mit oder ohne Pumpvorrichtung umfasst.

3. Zusammensetzung zur Verwendung nach Anspruch 1 oder Anspruch 2, wobei die Zusammensetzung pharmazeutisch unbedenkliche Träger und/oder Verdünnungsmittel ausgewählt aus der Gruppe bestehend aus Kochsalzlösung, wässrigen Pufferlösungen, Lösungsmitteln, Dispersionsmedienzusammensetzung und einer Mischung davon umfasst.

4. Zusammensetzung zur Verwendung nach einem der Ansprüche 1 bis 3, wobei die Zusammensetzung weiterhin ein oder mehrere Hydrogele ausgewählt aus der Gruppe bestehend aus Collagen, Atelocollagen, Fibrin, Thrombin-Fibrin und einer Mischung davon umfasst.

5. Zusammensetzung zur Verwendung nach einem der Ansprüche 1 bis 4, wobei die Population isolierter, von Nabelschnurgewebe stammenden Zellen Zellen umfasst, die genetisch so modifiziert sind, dass sie therapeutisch brauchbare Genprodukte produzieren oder Mittel zur Erleichterung oder Unterstützung der Bildung oder des Wachstums von Nervengewebe produzieren.

6. Zusammensetzung zur Verwendung nach einem der Ansprüche 1 bis 5, wobei die Zusammensetzung weiterhin eine Population von Zellen umfasst, die aus einem oder mehreren der folgenden Gewebe stammen: Hautgewebe, Gefäßgewebe, Bindegewebe, Knorpel, Fettgewebe, Muskelgewebe, Sehnen oder Bänder.

7. Zusammensetzung zur Verwendung nach einem der Ansprüche 1 bis 6, wobei die isolierten, von Nabelschnurgewebe stammenden Zellen weiterhin die folgenden Eigenschaften umfassen:
das Potential für mindestens etwa 40 Verdopplungen in Kultur;
die Produktion von CD10, CD13, CD44, CD73 und CD90;
die Expression eines Gens, welches im Vergleich zu einer menschlichen Zelle, bei der es sich um einen Fibroblasten, eine mesenchymale Stammzelle oder eine Knochenmarkzelle aus dem Beckenkamm handelt, erhöht für ein für Interleukin 8 und Reticulon 1 codierendes Gen ist; und
die fehlende Produktion von CD45.

## Revendications

1. Composition comprenant une population de cellules dérivées de tissu de cordon ombilical isolées pour utilisation dans le traitement d'un sujet ayant une douleur neuropathique, une spasticité neurale ou une douleur chronique, où lesdites cellules sont dérivées de tissu de cordon ombilical humain sensiblement exempt de sang, sont capables d'autorenouvellement et d'expansion en culture, sont négatives pour CD117 et n'expriment pas hTERT ou la télomérase, où le traitement comprend l'administration de la population de cellules dérivées de tissu de cordon ombilical isolées de façon systémique.

2. Composition pour utilisation selon la revendication 1, le traitement comprenant l'administration de la population de cellules dérivées de tissu de cordon ombilical isolées par voie intraveineuse, interpéritonéale, intra-artérielle, ou par l'intermédiaire de seringues avec des aiguilles ou des cathéters avec ou sans dispositifs à pompe.

3. Composition pour utilisation selon la revendication 1 ou la revendication 2, la composition comprenant des véhicules et/ou diluants pharmaceutiquement acceptables choisis dans le groupe constitué de : soluté, solutions tampons aqueuses, solvants, composition de milieux de dispersion et un mélange de ceux-ci.

4. Composition pour utilisation selon l'une quelconque des revendications 1 à 3, la composition comprenant en outre un ou plusieurs hydrogels qui sont choisis dans le groupe constitué de : collagène, atélocollagène, fibrine, thrombine-fibrine, et un mélange de ceux-ci.

5. Composition pour utilisation selon l'une quelconque des revendications 1 à 4, la population de cellules dérivées de tissu de cordon ombilical isolées comprenant des cellules qui sont génétiquement modifiées pour produire des produits géniques thérapeutiquement utiles ou pour produire des agents pour faciliter la formation ou la croissance de tissu de support neural.

6. Composition pour utilisation selon l'une quelconque des revendications 1 à 5, ladite composition comprenant en outre une population de cellules dérivées d'un ou plusieurs des tissus suivants : tissu dermique, tissu vasculaire, tissu conjonctif, cartilage, tissu adipeux, tissu musculaire, tendons ou ligaments.

7. Composition pour utilisation selon l'une quelconque des revendications 1 à 6, les cellules dérivées de tissu de cordon ombilical isolées comprenant en outre les caractéristiques suivantes :
le potentiel pour au moins 40 doublements en culture ;
la production de CD10, CD13, CD44, CD73, et CD90 ;
l'expression d'un gène, qui par rapport à une cellule humaine qui est un fibroblaste, une cellule souche mésenchymateuse, ou une cellule de moelle osseuse de crête iliaque, est augmentée pour un gène codant pour l'interleukine 8 et réticulon 1 ; et
l'absence de production de CD45.


### SEQUENCE LISTING

<110> KRAMER, BRIAN C. HERZBERG, URI
<120> SYSTEMICALLY AND LOCALLY ADMINISTERED CELLS FOR NEUROPATHIC PAIN
<130> 026038.0227PTWO
<140> PCTUS0968879
   <141> 2009-12-19
<150> 61/139,169
   <151> 2008-12-19
<160> 10
<170> PatentIn version 3.5
<210> 1
   <211> 22
   <212> DNA
   <213> Artificial Sequence
<220>
   <223> Description of Artificial Sequence: Synthetic primer
<400> 1
   gagaaatcca aagagcaaat gg 22
<210> 2
   <211> 21
   <212> DNA
   <213> Artificial Sequence
<220>
   <223> Description of Artificial Sequence: Synthetic primer
<400> 2
   agaatggaaa actggaatag g 21
<210> 3
   <211> 20
   <212> DNA
   <213> Artificial Sequence
<220>
   <223> Description of Artificial Sequence: Synthetic primer
<400> 3
   tcttcgatgc ttcggattcc 20
<210> 4
   <211> 21
   <212> DNA
   <213> Artificial Sequence
<220>
   <223> Description of Artificial Sequence: Synthetic primer
<400> 4
   gaattctcgg aatctctgtt g 21
<210> 5
   <211> 21
   <212> DNA
   <213> Artificial Sequence
<220>
   <223> Description of Artificial Sequence: Synthetic primer
<400> 5
   ttacaagcag tgcagaaaac c 21
<210> 6
   <211> 22
   <212> DNA
   <213> Artificial Sequence
<220>
   <223> Description of Artificial Sequence: Synthetic primer
<400> 6
   agtaaacatt gaaaccacag cc 22
<210> 7
   <211> 20
   <212> DNA
   <213> Artificial Sequence
<220>
   <223> Description of Artificial Sequence: Synthetic primer
<400> 7
   tctgcagctc tgtgtgaagg 20
<210> 8
   <211> 22
   <212> DNA
   <213> Artificial Sequence
<220>
   <223> Description of Artificial Sequence: Synthetic primer
<400> 8
   cttcaaaaac ttctccacaa cc 22
<210> 9
   <211> 17
   <212> DNA
   <213> Artificial Sequence
<220>
   <223> Description of Artificial Sequence: Synthetic primer
<400> 9
   cccacgccac gctctcc 17
<210> 10
   <211> 19
   <212> DNA
   <213> Artificial Sequence
<220>
   <223> Description of Artificial Sequence: Synthetic primer
<400> 10
   tcctgtcagt tggtgctcc 19